(19) Europäisches Patentamt
European Patent Office
Office européen des brevets

(11) EP 1 800 130 B1

(12) **EUROPEAN PATENT SPECIFICATION**

(45) Date of publication and mention of the grant of the patent:
**17.08.2011 Bulletin 2011/33**

(21) Application number: **05810348.2**

(22) Date of filing: **22.09.2005**

(51) Int Cl.:
***G01N 33/574*** *(2006.01)*

(86) International application number:
**PCT/US2005/034152**

(87) International publication number:
**WO 2006/036788 (06.04.2006 Gazette 2006/14)**

(54) **METHODS AND COMPOSITIONS FOR EVALUATING BREAST CANCER PROGNOSIS**

VERFAHREN UND ZUSAMMENSETZUNGEN ZUR BEWERTUNG EINER BRUSTKREBSPROGNOSE

MÉTHODES ET COMPOSITIONS PERMETTANT D'ÉVALUER UN PRONOSTIC DE CANCER DU SEIN

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **22.09.2004 US 611965 P**
**22.09.2004 US 612073 P**

(43) Date of publication of application:
**27.06.2007 Bulletin 2007/26**

(73) Proprietor: **Tripath Imaging, Inc.**
**Burlington, NC 27215 (US)**

(72) Inventors:
- **FISCHER, Timothy, J.**
**Raleigh, NC 27613 (US)**
- **WHITEHEAD, Clark, M.**
**Apex, NC 27502 (US)**
- **MALINOWSKI, Douglas, P.**
**Hillsborough, NC 27278 (US)**
- **MARCELPOIL, Raphael**
**F-3800 Grenoble (FR)**
- **MOREL, Didier**
**F-38000 Grenoble (FR)**

(74) Representative: **Bentham, Andrew et al**
**J.A. Kemp & Co.**
**14 South Square**
**Gray's Inn**
**London WC1R 5JJ (GB)**

(56) References cited:

| | |
|---|---|
| **WO-A-03/069307** | **WO-A-2005/071419** |
| **WO-A-2005/095964** | **US-A1- 2002 098 535** |
| **US-A1- 2002 132 246** | **US-A1- 2003 077 832** |
| **US-A1- 2003 087 265** | **US-A1- 2004 002 067** |
| **US-A1- 2004 018 525** | **US-A1- 2004 229 299** |
| **US-A1- 2005 221 398** | |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

The present invention relates to methods and compositions for evaluating the prognosis of a patient afflicted with breast cancer, particularly early-stage breast cancer.

BACKGROUND OF THE INVENTION

Breast cancer is the second most common cancer among American women, less frequent only than skin cancer. An American woman has a one in eight chance of developing breast cancer during her lifetime, and the American Cancer Society estimates that more than 250,000 new cases of breast cancer will be reported in the U.S. this year. Breast cancer is the second leading cause of cancer deaths in women, with more than 40,000 Americans expected to die from the disease in 2004.

Improved detection methods, mass screening, and advances in treatment over the last decade have significantly improved the outlook for woman diagnosed with breast cancer. Today, approximately 80% of breast cancer cases are diagnosed in the early stages of the disease when survival rates are at their highest. As a result, about 85% percent of breast cancer patients are alive at least 5 years after diagnosis.

Despite these advances, approximately 20% of women diagnosed with early-stage breast cancer have a poor ten-year outcome and will suffer disease recurrence, metastasis, or death within this time period. The remaining 80% of breast cancer patients diagnosed at an early stage, however, have a good 10-year prognosis and are unlikely to need, or benefit from, additional aggressive adjuvant therapy (e.g., chemotherapy). The current clinical consensus is that at least some early-stage, node-negative breast cancer patients should receive adjuvant chemotherapy, but presently there are no widely used assays to risk stratify patients for more aggressive treatment. Since the majority of these early-stage cancer patients enjoy long-term survival following surgery and/or radiation therapy without further treatment, it is likely inappropriate to recommend aggressive adjuvant therapy for all of these patients, particularly in light of the significant side effects associated with cancer chemotherapeutics. Compositions and methods that permit the differentiation of these populations of early-stage breast cancer patients at the time of initial diagnosis into good and bad prognosis groups would assist clinicians in selecting appropriate courses of treatment. Thus, methods for evaluating the prognosis of breast cancer patients, particularly early-stage breast cancer patients, are needed.

Significant research has focused on identifying methods and factors for assessing breast cancer prognosis and predicting therapeutic response. (See generally, Ross and Hortobagyi, eds. (in press) Molecular Oncology of Breast Cancer (Jones and Bartlett Publishers, Boston, MA) and the references cited therein ). Prognostic indicators include more conventional factors, such as tumor size, nodal status, and histological grade, as well as molecular markers that provide some information regarding prognosis and likely response to particular treatments. For example, determination of estrogen (ER) and progesterone (PR) steroid hormone receptor status has become a routine procedure in assessment of breast cancer patients. See, for example, Fitzgibbons et al. (2000) Arch. Pathol..Lab. Med. 124:966-978. Tumors that are hormone receptor positive are more likely to respond to hormone therapy and also typically grow less aggressively, thereby resulting in a better prognosis for patients with ER+/PR+ tumors.

Overexpression of human epidermal growth factor receptor 2 (HER-2/neu), a transmembrane tyrosine kinase receptor protein, has been correlated with poor breast cancer prognosis. Ross et al. (2003) The Oncologist :307-325. Her2/neu expression levels in breast tumors are currently used to predict response to the anti-Her-2/neu antibody therapeutic trastuzumab (Herceptin®; Genentech). See, for example, *Id.* and Ross *et al.*, *supra*. Furthermore, approximately one-third of breast cancers have mutations in the tumor suppressor gene p53, and these mutations have been associated with increased disease aggressiveness and poor prognostic outcome. Fitzgibbons *et al.*, *supra*. Ki-67 is a non-histone nuclear protein that is expressed during the G1 through M phases of the cell cycle. Studies have shown that overexpression of the cellular proliferation marker Ki-67 also correlates with poor breast cancer prognosis. *Id.*

Although current prognostic criteria and molecular markers provide some guidance in predicting patient outcome and selecting appropriate course of treatment, a significant need exists for a specific and sensitive method for evaluating breast cancer prognosis, particularly in early-stage, lymph-node negative patients. Such a method should specifically distinguish breast cancer patients with a poor prognosis from those with a good prognosis and permit the identification of high-risk, early- stage breast cancer patients who are likely to need aggressive adjuvant therapy.

SUMMARY OF THE INVENTION

Methods and compositions for evaluating the prognosis of a cancer patient, particularly a breast cancer patient, are provided. The methods comprise:

a) contacting a sample from a patient with at least five antibodies that each specifically bind to a distinct biomarker protein, wherein a first antibody specifically binds to SLPI, wherein a second antibody specifically binds to src, wherein a third antibody specifically binds to PSMB9, wherein a fourth antibody specifically binds to p21ras, and wherein a fifth antibody specifically binds to E2F1;
b) detecting binding of said antibodies to said biomarker proteins;
c) determining if said biomarker proteins are overexpressed in said sample, wherein overexpression of at least two biomarker proteins is indicative of a poor prognosis; and,
d) thereby evaluating the prognosis of said breast cancer patient.

Overexpression of the biomarker or combination of biomarkers of the invention is indicative of either a good prognosis (i.e., disease- free survival) or a bad prognosis (i.e., cancer recurrence, metastasis, or death from the underlying cancer). Thus, the present method permits the differentiation of breast cancer patients with a good prognosis from those patients with a bad prognosis. The methods disclosed herein can be used in combination with assessment of conventional clinical factors (e.g., tumor size, tumor grade, lymph node status, and family history) and/or analysis of the expression level of molecular markers, such as Her2/neu, Ki67, p53, and estrogen and progesterone hormone receptors. In this manner, the methods of the invention permit a more accurate evaluation of breast cancer prognosis.

The biomarkers of the invention are proteins whose overexpression is indicative of cancer prognosis, including those biomarkers involved in cell cycle regulation, DNA replication, transcription, signal transduction, cell proliferation, invasion, or metastasis. The detection of overexpression of the biomarker proteins of the invention permits the evaluation of cancer prognosis and facilitates the separation of breast cancer patients into good and bad prognosis risk groups for the purposes of, for example, treatment selection.

Biomarker expression can be assessed at the protein or nucleic acid level. In some embodiments, immunohistochemistry techniques are provided that utilize antibodies to detect the expression of biomarker proteins in breast tumor samples. In this aspect of the invention, at least one antibody directed to a specific biomarker of interest is used. Expression can also be detected by nucleic acid-based techniques, including, for example, hybridization and RT-PCR.

Compositions include monoclonal antibodies capable of binding to biomarker proteins of the invention. Antigen-binding fragments and variants of these monoclonal antibodies, hybridoma cell lines producing these antibodies, and isolated nucleic acid molecules encoding the amino acid sequences of these monoclonal antibodies are also encompassed herein. Kits comprising reagents for practicing the methods of the invention are further provided.

## BRIEF DESCRIPTION OF THE DRAWINGS

Figure 1 shows the distribution of percentage of cells staining with an intensity of 2 as a function of actual breast cancer outcome. Experimental details are provided in Example 4.
Figure 2 provides the ROC curve obtained using the sequence-based interpretation approach for the SLPI/p21ras/E2F1/PSMB9/src/ phospho-p27 combination. Experimental details are provided in Example 5.
Figure 3 provides the Kaplan-Meier plot for the prognostic performance of the SLPI, src, PSMB9, p21ras, and E2F1 biomarker panel. Details are provided in Example 8.
Figure 4 provides a graphical representation of the long-term survival data for the general breast cancer patient population, independent of analysis of biomarker overexpression. Details are provided in Example 8.
Figure 5 provides the Kaplan-Meier plot for the prognostic performance of the SLPI, src, PSMB9, p21ras, E2F1, and MUC-1 biomarker panel. Details are provided in Example 9.

## DETAILED DESCRIPTION OF THE INVENTION

The present invention provides methods and compositions for evaluating the prognosis of a cancer patient, particularly a breast cancer patient, more particularly an early-stage breast cancer patient. The methods comprise detecting the expression of biomarkers in a patient tissue or body fluid sample and determining if said biomarkers are overexpressed. Overexpression of a biomarker or combination of biomarkers used in the practice of the invention is indicative of breast cancer prognosis (i.e., bad or good prognosis). Thus, overexpression of a particular biomarker or combination of biomarkers of interest permits the differentiation of breast cancer patients that are likely to experience disease recurrence (i.e., poor prognosis) from those who are more likely to remain cancer-free (i.e., good prognosis). In some aspects of the invention, the methods involve detecting the overexpression of biomarkers in a breast tumor sample that are indicative of a poor breast cancer prognosis and thereby identifying patients who are more likely to suffer a recurrence of the underlying cancer. The methods of the invention can also be used to assist in selecting appropriate courses of treatment and to identify patients that would benefit from more aggressive therapy. In particular embodiments, antibodies and immunohistochemistry techniques are used to detect expression of a biomarker of interest and to evaluate the prognosis of a breast cancer patient. Monoclonal antibodies specific for biomarkers of interest and kits for practicing the methods of the invention are further provided.

By "breast cancer" is intended, for example, those conditions classified by biopsy as malignant pathology. The clinical delineation of breast cancer diagnoses is well-known in the medical arts. One of skill in the art will appreciate that breast cancer refers to any malignancy of the breast tissue, including, for example, carcinomas and sarcomas. In particular embodiments, the breast cancer is ductal carcinoma *in situ* (DCIS), lobular carcinoma *in situ* (LCIS), or mucinous carcinoma. Breast cancer also refers to infiltrating ductal (IDC) or infiltrating lobular carcinoma (ILC). In most embodiments of the invention, the subject of interest is a human patient suspected of or actually diagnosed with breast cancer.

The American Joint Committee on Cancer (AJCC) has developed a standardized system for breast cancer staging using a "TNM" classification scheme. Patients are assessed for primary tumor size (T), regional lymph node status (N), and the presence/absence of distant metastasis (M) and then classified into stages O-IV based on this combination of factors. In this system, primary tumor size is categorized on a scale of 0-4 (T0 = no evidence of primary tumor; T1 = ≤2 cm; T2 = >2 cm - ≤5 cm; T3 = >5 cm; T4 = tumor of any size with direct spread to chest wall or skin). Lymph node status is classified as N0-N3 (NO = regional lymph nodes are free of metastasis; N1 = metastasis to movable, same-side axillary lymph node(s); N2 = metastasis to same-side lymph node(s) fixed to one another or to other structures; N3 = metastasis to same-side lymph nodes beneath the breastbone). Metastasis is categorized by the absence (M0) or presence of distant metastases (M1). While breast cancer patients at any clinical stage are encompassed by the present invention, breast cancer patients in early-stage breast cancer are of particular interest. By "early-stage breast cancer" is intended stages 0 (*in situ* breast cancer), I (T1, N0, M0), IIA (TO-1, N1, M0 or T2, N0, M0), and IIB (T2, N1, M0 or T3, N0, M0). Early-stage breast cancer patients exhibit little or no lymph node involvement. As used herein, "lymph node involvement" or "lymph node status" refers to whether the cancer has metastasized to the lymph nodes. Breast cancer patients are classified as "lymph node-positive" or "lymph node-negative" on this basis. Methods of identifying breast cancer patients and staging the disease are well known and may include manual examination, biopsy, review of patient's and/or family history, and imaging techniques, such as mammography, magnetic resonance imaging (MRI), and positron emission tomography (PET).

The term "prognosis" is recognized in the art and encompasses predictions about the likely course of disease or disease progression, particularly with respect to likelihood of disease remission, disease relapse, tumor recurrence, metastasis, and death. "Good prognosis" refers to the likelihood that a patient afflicted with cancer, particularly breast cancer, will remain disease-free (i.e., cancer-free). "Poor prognosis" is intended to mean the likelihood of a relapse or recurrence of the underlying cancer or tumor, metastasis, or death. Cancer patients classified as having a "good outcome" remain free of the underlying cancer or tumor. In contrast, "bad outcome" cancer patients experience disease relapse, tumor recurrence, metastasis, or death. In particular embodiments, the time frame for assessing prognosis and outcome is, for example, less than one year, one, two, three, four, five, six, seven, eight, nine, ten, fifteen, twenty or more years. As used herein, the relevant time for assessing prognosis or disease-free survival time begins with the surgical removal of the tumor or suppression, mitigation, or inhibition of tumor growth. Thus, for example, in particular embodiments, a "good prognosis" refers to the likelihood that a breast cancer patient will remain free of the underlying cancer or tumor for a period of at least five, more particularly, a period of at least ten years. In further aspects of the invention, a "bad prognosis" refers to the likelihood that a breast cancer patient will experience disease relapse, tumor recurrence, metastasis, or death within less than five years, more particularly less than ten years. Time frames for assessing prognosis and outcome provided above are illustrative and are not intended to be limiting.

In some embodiments described herein, prognostic performance of the biomarkers and/or other clinical parameters was assessed utilizing a Cox Proportional Hazards Model Analysis, which is a regression method for survival data that provides an estimate of the hazard ratio and its confidence interval. The Cox model is a well-recognized statistical technique for exploring the relationship between the survival of a patient and particular variables. This statistical method permits estimation of the hazard (i.e., risk) of individuals given their prognostic variables (e.g., overexpression of particular biomarkers, as described herein). Cox model data are commonly presented as Kaplan-Meier curves. The "hazard ratio" is the risk of death at any given time point for patients displaying particular prognostic variables. See generally Spruance et al. (2004) Antimicrob. Agents & Chemo. 48:2787-2792. In particular embodiments, the biomarkers of interest are statistically significant for assessment of the likelihood of breast cancer recurrence or death due to the underlying breast cancer. Methods for assessing statistical significance are well known in the art and include, for example, using a log-rank test Cox analysis and Kaplan-Meier curves. In some aspects of the invention, a p-value of less than 0.05 constitutes statistical significance.

As described herein above, a number of clinical and prognostic breast cancer factors are known in the art and are used to predict treatment outcome and the likelihood of disease recurrence. Such factors include lymph node involvement, tumor size, histologic grade, family history, estrogen and progesterone hormone receptor status, Her 2/neu levels, and tumor ploidy. As used herein, estrogen and progesterone hormone receptor status refers to whether these receptors are expressed in the breast tumor of a particular breast cancer patient. Thus, an "estrogen receptor-positive patient" displays estrogen receptor expression in a breast tumor, whereas an "estrogen receptor-negative patient" does not. Using the methods of the present invention, the prognosis of a breast cancer patient can be determined independent of or in combination with assessment of these or other clinical and prognostic factors. In some embodiments, combining the methods disclosed herein with evaluation of other prognostic factors may permit a more accurate determination of breast cancer prognosis. The methods of the invention may be coupled with analysis of, for example, Her2/neu, Ki67, and/or p53 expression levels. Other factors, such as patient clinical history, family history, and menopausal status, may also be considered when evaluating breast cancer prognosis via the methods of the invention. In some embodiments, patient data obtained via the methods disclosed herein may be coupled with analysis of clinical information and existing tests for breast cancer prognosis to develop a reference laboratory prognostic algorithm. Such algorithms find used in stratifying breast cancer patients, particularly early-stage breast cancer patients, into good and bad prognosis populations. Patients assessed as having a poor prognosis may be upstaged for more aggressive breast cancer treatment.

The methods of the invention permit the superior assessment of breast cancer prognosis in comparison to analysis of other known prognostic indicators (e.g., lymph node involvement, tumor size, histologic grade, estrogen and progesterone receptor levels, Her 2/neu status, tumor ploidy, and family history). In particular aspects of the invention, the sensitivity and specificity is equal to or greater than that of known cancer prognostic evaluation methods. The endpoint for assessing specificity and sensitivity is comparison of the prognosis or outcome predicted using the methods of the invention (i.e., at or near the time of diagnosis) with the actual clinical outcome (i.e., whether the patient remained cancer-free or suffered a recurrence within a specified time period). As used herein, "specificity" refers to the level at which a method of the invention can accurately identify true negatives. In a clinical study, specificity is calculated by dividing the number of true negatives by the sum of true negatives and false positives. By "sensitivity" is intended the level at which a method of the invention can accurately identify samples that are true positives. Sensitivity is calculated in a clinical study by dividing the number of true positives by the sum of true positives and false negatives. In some embodiments, the sensitivity of the disclosed methods for the evaluation of breast cancer is at least about 40%, 45%, 50%, 55%, 60%, 65%, 70%, 75%, 80%, 85%, 90%, 91%, 92%, 93%, 94%, 95%, 96%, 97%, 98%, 99% or more. Furthermore, the specificity of the present methods is preferably at least about 40%, 45%, 50%, 55%, 60%, 65%, 70%, 75%, 80%, 85%, 90%, 91%, 92%, 93%, 94%, 95%, 96%, 97%, 98%, 99% or more. In further embodiments, the combined sensitivity and specificity value for the prognostic methods of the invention is assessed. By "combined sensitivity and specificity value" is intended the sum of the individual specificity and sensitivity values, as defined herein above. The combined sensitivity and specificity value of the present methods is preferably at least about 105%, 110%, 115%, 120%, 130%, 140%, 150%, 160% or more.

As used herein, the definitions of "true" and "false" positives and negatives will be dependent upon whether the biomarker or combination of biomarkers under consideration are good outcome or bad outcome biomarkers. That is, in the case of good outcome biomarkers (i.e., those indicative of a good prognosis), "true positive" refers to those samples exhibiting overexpression of the biomarker of interest, as determined by the methods of the invention (e.g., positive staining by immunohistochemistry), that have a confirmed good actual clinical outcome. In contrast, "false positives" display overexpression of the good outcome biomarker(s) but have a confirmed bad actual clinical outcome. "True negatives" and "false negatives" with respect to good outcome biomarkers do not display biomarker overexpression (e.g., do not stain positive in immunohistochemistry methods) and have confirmed bad and good actual clinical outcomes, respectively.

Similarly, in the case of bad outcome biomarkers, "true positives" refers to those samples exhibiting overexpression of the biomarker or combination of biomarkers of interest that have a confirmed bad actual clinical outcome. That is, "true positive" with respect to both good and bad outcome biomarkers refers to samples in which the actual clinical outcome (i.e., good or bad) is accurately predicted. "False positives" display overexpression of the bad outcome biomarker but have a confirmed good actual clinical outcome. "True negatives" and "false negatives" with respect to bad outcome biomarkers do not display biomarker overexpression and have conformed good and bad actual clinical outcomes, respectively.

Breast cancer is managed by several alternative strategies that may include, for example, surgery, radiation therapy, hormone therapy, chemotherapy, or some combination thereof As is known in the art, treatment decisions for individual breast cancer patients can be based on the number of lymph nodes involved, estrogen and progesterone receptor status, size of the primary tumor, and stage of the disease at diagnosis. Analysis of a variety of clinical factors and clinical trials has led to the development of recommendations and treatment guidelines for early-stage breast cancer by the International Consensus Panel of the St. Gallen Conference (2001). See Goldhirsch et al. (2001) J. Clin. Oncol. 19:3817-3827 . The guidelines indicate that treatment for patients with node-negative breast cancer varies substantially according to the baseline prognosis. More aggressive treatment is recommended for patients with a relative high risk of recurrence when compared to patients with a relatively low risk of recurrence. It has been demonstrated that chemotherapy for the high risk population has resulted in a reduction in the risk of relapse. Women with a low risk category are usually treated with radiation and hormonal therapy. Stratification of patients into poor prognosis or good prognosis risk groups at the time of diagnosis using the methods disclosed herein may provide an additional or alternative treatment decision-making factor. The methods of the invention permit the differentiation of breast cancer patients with a good prognosis from those more likely to suffer a recurrence (i.e., patients who might need or benefit from additional aggressive treatment at the time of diagnosis). The methods of the invention find particular use in choosing appropriate treatment for early stage breast cancer patients. As discussed above, the majority of breast cancer patients diagnosed at an early-stage of the disease enjoy long-term survival following surgery and/or radiation therapy without further adjuvant therapy. A significant percentage (approximately 20%) of these patients, however, will suffer disease recurrence or death, leading to clinical recommendations that some or all early-stage breast cancer patients should receive adjuvant therapy (e.g., chemotherapy). The methods of the present invention find use in identifying this high-risk, poor prognosis population of early-stage breast cancer patients and thereby determining which patients would benefit from continued and/or more aggressive therapy and close monitoring following treatment. For example, early-stage breast cancer patients assessed as having a poor prognosis by the methods disclosed herein may be selected for more aggressive adjuvant therapy, such as chemotherapy, following surgery and/or radiation treatment. In particular embodiments, the methods of the present invention may be used in conjunction with the treatment guidelines established by the St. Gallens Conference to permit physicians to make more informed breast cancer treatment decisions. The present methods for evaluating breast cancer prognosis can also be combined with other prognostic methods and molecular marker analyses known in the art (e.g., Her2/neu, Ki67, and p53 expression levels) for purposes of selecting an appropriate breast cancer treatment. Furthermore, the methods of the invention can be combined with later-developed prognostic methods and molecular marker analyses not currently known in the art.

The methods disclosed herein also find use in predicting the response of a breast cancer patient to a selected treatment. By "predicting the response of a breast cancer patient to a selected treatment" is intended assessing the likelihood that a patient will experience a positive or negative outcome with a particular treatment. As used herein, "indicative of a positive treatment outcome" refers to an increased likelihood that the patient will experience beneficial results from the selected treatment (e.g., complete or partial remission, reduced tumor size, etc.). By "indicative of a negative treatment outcome" is intended an increased likelihood that the patient will not benefit from the selected treatment with respect to the progression of the underlying breast cancer. In some aspects of the invention, the selected treatment is chemotherapy.

In certain embodiments, methods for predicting the likelihood of survival of a breast cancer patient are provided. In particular, the methods may be used predict the likelihood of long-term, disease-free survival. By "predicting the likelihood of survival of a breast cancer patient" is intended assessing the risk that a patient will die as a result of the underlying breast cancer. "Long-term, disease-free survival" is intended to mean that the patient does not die from or suffer a recurrence of the underlying breast cancer within a period of at least five years, more particularly at least ten or more years, following initial diagnosis or treatment. Such methods for predicting the likelihood of survival of a breast cancer patient comprise detecting expression of multiple biomarkers in a patient sample, wherein the likelihood of survival, particularly long-term, disease-free survival, decreases as the number of biomarkers determined to be overexpressed in the patient sample increases. For example, in one aspect of the invention, the expression of at least five biomarkers is determined, wherein overexpression of none of the biomarkers is indicative of an increased likelihood of survival, and wherein overexpression of two or more biomarkers is indicative of a decreased likelihood of survival. Likelihood of survival may be assessed in comparison to, for example, breast cancer survival statistics available in the art. In other embodiments, methods for predicting the likelihood of survival of breast cancer patient comprise determining the expression of at least six biomarkers and assessing the number of these biomarkers that are overexpressed. Biomarkers useful for these methods may be selected from, for example, E2F1, SLPI, MUC-1, src, p21ras, and PSMB9. See generally examples 8 and 9.

The biomakers of the invention are proteins. A biomarker protein comprises the entire or partial amino acid sequence of any of the biomarker proteins or polypeptides. Fragments of biomarker proteins are also encompassed by the present invention. By "fragment" is intended a portion of the amino acid sequence and hence protein encoded thereby.

A "biomarker" is any protein whose level of expression in a tissue or cell is altered compared to that of a normal or healthy cell or tissue. The biomarkers of the present invention are proteins whose overexpression correlates with cancer, particularly breast cancer, prognosis. Selective overexpression of a biomarkers or combination of biomarkers of interest in a patient sample is indicative of a poor cancer prognosis. By "indicative of a poor prognosis" is intended that overexpression of the particular biomarker or combination of biomarkers is associated with an increased likelihood of relapse or recurrence of the underlying cancer or tumor, metastasis, or death, as defined herein above. For example, "indicative of a poor prognosis" may refer to an increased likelihood of relapse or recurrence of the underlying cancer or tumor, metastasis, or death within five years, more particularly ten years. Biomarkers that are indicative of a poor prognosis may be referred to herein as "bad outcome biomarkers." In other aspects of the invention, the absence of overexpression of a biomarkers or combination of biomarkers of interest is indicative of a good prognosis. As used herein, "indicative of a good prognosis" refers to an increased likelihood that the patient will remain cancer-free, as defined herein above. In some embodiments, "indicative of a good prognosis" refers to an increased likelihood that the patient will remain cancer-free for at least five, more particularly at least ten years. Such biomarkers may be referred to as "good outcome biomarkers."

The biomarkers of the present invention include any protein whose overexpression correlates with breast cancer prognosis, as described herein above. Biomarkers include proteins that are indicative of a poor breast cancer prognosis (i.e., bad outcome biomarkers) as well as those that are indicative of a good prognosis (i.e., good outcome biomarkers). Biomarkers of particular interest include proteins that are involved in regulation of cell growth and proliferation, cell cycle control, DNA replication and transcription, apoptosis, signal transduction, angiogenesis/lymphogenesis, or metastasis. In some embodiments, the biomarkers regulate protease systems involved in tissue remodelling, extracellular matrix degradation, and adjacent tissue invasion.

The invention requires that at least five antibodies that each specifically bind to a distinct biomarker protein are used, wherein a first antibody specifically binds to SLPI, wherein a second antibody specifically binds to src, wherein a third antibody specifically binds to PSMB9, wherein a fourth antibody specifically binds to p21ras, and wherein a fifth antibody specifically binds to E2F1.

In an other embodiment, the method of the invention may further comprise determining if one or more additional biomarker proteins is overexpressed in the sample, wherein the one or more additional biomarker proteins is selected from the group consisting of MUC-1, DARPP-32, phospho-p27, MGC 14832, myc, TGFβ-3, SERHL, PDGFRα, NDRG-1, MCM2, and MCM6.

Secretory Leukocyte Protease Inhibitor (SLPI) is a non-specific inhibitor that can inactivate a number of proteases including leukocyte elastase, trypsin, chymotrypsin and the cathepsins (e.g., cathepsin G). SLPI is known to be involved in inflammation and the inflammatory response in relation to tissue repair. Protease inhibitors have generally been considered to counteract tumor progression and metastasis. However, expression of serine protease inhibitors (SPI's) in tumors is often associated with poor prognosis of cancer patients. Cathepsin G is over expressed in breast cancer and is an indicator of poor prognosis. Its inhibitory effect contributes to the immune response by protecting epithelial surfaces from attack by endogenous proteolytic enzymes. The gene location for SLPI is 20q12, which is a chromosomal region implicated in breast cancer chromosomal alterations and aneuploidy.

PSMB9 is a member of the proteasome B-type family, also known as the T1B family, that is a 20S core beta subunit. This gene is located in the class n region of the MHC (major histocompatibility complex). Expression of this gene is induced by gamma interferon, and this gene product replaces catalytic subunit 1 (proteasome beta 6 subunit) in the immunoproteasome. Proteolytic processing is required to generate a mature subunit

NDRG-1 (N-Myc downstream regulated) is upregulated during cell differentiation, repressed by N-myc and c-myc in embryonic cells, and suppressed in several tumor cells. Overexpression may be related to hypoxia and the subsequent signaling to induce angiogenesis. Hypoxia causes the accumulation of the transcription factor hypoxia-inducible factor 1 (HIF-1), culminating in the expression of hypoxia-inducible genes such as those for vascular endothelial growth factor (VEGF) and NDRG-1. NDRG-1 is found in some breast cancers as an overexpressed mRNA. NDRG-1 is located on chromosome 8q24 adjacent to the c-myc gene.

MUC1 is a heavily O-glycosylated transmembrane protein expressed on most secretory epithelium, including mammary glands and some hematopoietic cells. It is expressed abundantly in lactating mammary glands and overexpressed in more than 90% of breast carcinomas and metastases. In normal mammary glands, it is expressed on the apical surface of glandular epithelium.

p27 is a key regulator of the cell cycle and participates in the G1-to-S phase progression. It interacts specifically with the cyclin E/cdk2 complex during G1 phase and also with D-type cyclin-cdks. p27 can be phosphorylated on threonine 187 by Cdks. Phosphorylation of p27 at threonine 187 is also cell-cycle dependent, present in proliferating cells but undetectable in G1 cells. Activation of p27 degradation is seen in proliferating cells and in many types of aggressive human carcinomas. Overexpression of p27 may lead to an inhibition of apoptosis and resistance to some chemotherapy.

The Src family of protein tyrosine kinases (including Src, Lyn, Fyn, Yes, Lck, B1k, Hck, etc.) is important in the regulation of growth and differentiation of eukaryotic cells. Src activity is regulated by tyrosine phosphorylation at two sites with opposing effects. Phosphorylation of Tyr416 in the activation loop of the kinase domain upregulates the enzyme. Phosphorylation of Tyr527 in the C-terminal tail by Csk renders the enzyme less active.

E2F1 is a member of a family of transcription factors involved in the regulation of both G1 and S phase cyclins, in particular cyclin D1. These proteins participate in the Rb pathway of cell-cycle regulation and control of DNA synthesis. During the G1 phase of the cell-cycle, the E2F transcription factors are bound in an inactive complex with the Rb tumor suppressor protein. During the G1/S boundary of the cell cycle, the Rb protein is hyperphosphorylated and releases the E2F transcription factor from its inhibitory complex. The E2F transcription factor then activates transcription for those genes responsible for the S-phase of the cell-cycle, predominantly resulting in initiation of DNA synthesis and preparation for mitosis and subsequent cell division. Overexpression of E2F1 has been shown to lead to the induction of apoptosis possibly through the inhibition of cyclinD1-dependent kinase activity coupled with the induction of a p16 related transcript. In addition, regulation of E2F1 at the level of transcription, E2F1 protein levels are also controlled by the ubiquitin-proteosome dependent degradation pathway. Ubiquitination is blocked by the Rb and E2F1 complex, which directly controls aspects of cell cycle progression. p21ras is a member of a large group of cytoplasmic proteins involved in signal transduction. Guanine nucleotide binding proteins (G proteins) comprise a large group of cytoplasmic proteins present in eukaryotic cells that are involved in signal transduction. There are two forms, the large heterotrimeric G proteins and the smaller monomers. The 3 ras oncogenes, H-ras, K-ras, and N-ras are members of the smaller monomeric G proteins and are located on chromosomes 11,12 and 1 respectively. They encode 21-kD proteins called p21s and contain 188 amino acids. p21 ras proteins are involved in normal cell growth, protease activities, and cell adhesion.

Collectively, the three forms of p21ras function by linking ligand-mediated extracellular receptor activation with intracellular tyrosine kinase activation and subsequent initiation of a number of cellular processes relevant to breast cancer progression, including DNA replication, proliferation, and anchorage independent growth. The K- and H-ras genes are most often implicated in breast cancer. In both of these ras genes, mutations at codons 12 and 13 are common. These gain-of-function mutations result in constitutive activation that uncouples the normal ligand-induced signal transduction within the ras signaling pathways. Less common in breast cancer is the involvement of N-ras. Two mechanisms have been reported for N-ras associated changes in breast cancer: mutation at codon 61 resulting in constitutive activation of the oncogene, similar to the mutations mentioned above for K- and H-ras, and chromosomal amplification. Moreover, in addition to activation of intracellular signaling pathways, the ras oncogenes have been reported to induce overexpression of proteases important for tissue remodeling and invasion. H-ras has been implicated in matrix metalloprotease-2 (MMP-2) overexpression, and N-ras has been associated with overexpression of MMP-9. See generally Correll and Zoll (1988) Human Genetics 79:225-259; Tong et al. (1989) Nature 337:90-93; Watson et al. (1991) Breast Cancer Res. Treat. 17:161-169; Dati et al. (1991) Int. J. Cancer 47:833-838; Archer et al. (1995) Br. J. Cancer 72:1259-1266; Bland et al. (1995) Ann. Surg. 221:706-718; Shackney et al. (1998) Clin. Cancer Res. 4:913-928; and Gohring et al. (1999) Tumor Biol. 20:173-183 . Detection of any form (i.e., H-, K-, N-ras) of the p21ras proteins is encompassed by the present invention.

Minichromosome maintenance (MCM) proteins play an essential part in eukaryotic DNA replication. Each of the MCM proteins has DNA-dependent ATPase motifs in their highly conserved central domain. Levels of MCM proteins generally increase in a variable manner as normal cells progress from G0 into the G1/S phase of the cell cycle. In the G0 phase, MCM2 and MCM5 proteins are much less abundant than are the MCM7 and MCM3 proteins. MCM6 forms a complex with MCM2, MCM4, and MCM7, which binds histone H3. In addition, the subcomplex of MCM4, MCM6, and MCM7 has helicase activity, which is mediated by the ATP-binding activity of MCM6 and the DNA-binding activity of MCM4. See, for example, Freeman et al. (1999) Clin. Cancer Res. 5:2121-2132; Lei et al. (2001) J. Cell Sci. 114: 1447-1454; Ishimi et al. (2003) Eur. J. Biochem. 270:1089-1101.

DARPP32 is an inhibitor of protein phosphatase 1 whose biological function and inhibitory activity are modulated through specific amino acid residue phosphorylation within the DARPP32 protein. Threonine 34 (T34) phosphorylation renders the DARPP32 protein a specific protein phosphatase 1 inhibitor. However, threonine 75 (T75) phosphorylation renders the DARPP32 an inhibitor of protein kinase A (PKA). The gene location for DARPP32 is 17q21.2, which is known to be adjacent to the her2/neu (c-erb-B2 receptor tyrosine kinase) gene at 17q12. This region has been implicated in breast cancer chromosomal amplifications and resultant poor outcome within 25-35% of breast cancers. Several publications have demonstrated specific transcriptional activation of this 17q12-21 amplicon in breast cancer, with a number of genes located within this amplicon being overexpressed.

p53 plays multiple roles in cells. Expression of high levels of wild-type, but not mutant, p53 has two outcomes: cell cycle arrest or apoptosis. The observation that DNA-damaging agents induce levels of p53 in cells led to the definition of p53 as a checkpoint factor, akin perhaps to the product of the fad9 gene in yeast. While dispensable for viability, in response to genotoxic stress p53 acts as an "emergency brake" inducing either arrest or apoptosis, protecting the genome from accumulating excess mutations. Consistent with this notion, cells lacking p53 have been shown to be genetically unstable and, thus, more prone to tumors. The p53 protein is located in the nucleus of cells and is very labile. p53 is mutated in roughly 50% of all human tumors, predominantly in the DNA-binding domain codons.

In particular embodiments, the biomarkers are kinases that are involved in signal transduction pathways, such as PI3K regulatory a, LTk, Ser/thr kinase 15, MAPKBIPI, MAPKAPK2, and PK428, PRKR. Growth factors, extracellular signal transduction proteins, and extracellular matrix proteins are also biomarkers of interest. Such proteins include EGFR, TNF receptor associated factor 4, GFR bound protein 7, ErbB2 (her 2), VEGF, GDF1, IGFBP5, EGF8 ras homolog, MMP 9, MMP 7, SLPI, keratin 5, keratin 17, laminim gamma 2 (laminin V), troponin, and tubulin.

Although the methods of the invention require the detection of at least five, biomarker(s) in a patient sample for evaluating breast cancer prognosis, 6, 7, 8, 9, 10 or more biomarkers may be used to practice the present invention. It is recognized that detection of more than five biomarkers in a body sample may be used to evaluate cancer, particularly breast cancer, prognosis. Therefore, in some embodiments, five or more biomarkers are used, more preferably, five or more complementary biomarkers. By "complementary" is intended that detection of the combination of biomarkers in a body sample results in the accurate determination of cancer prognosis in a greater percentage of cases than would be identified if only one of the biomarkers was used. Accordingly, where at least five biomarker proteins are used, at least five antibodies directed to distinct biomarker proteins will be used to practice the immunohistochemistry methods disclosed herein. The antibodies may be contacted with the body sample simultaneously or successively.

When a combination of five or more biomarkers is used, the biomarkers will typically be substantially statistically independent of one another. By "statistically independent" biomarkers is intended that the prognoses generated therefrom are independent such that one biomarker does not provide substantially repetitive information with regard to the complementary biomarker. This may ensure, for instance, that a biomarker is not used in conjunction with a first biomarker when the two are not substantially statistically independent. The dependence of the two biomarkers may indicate that they are duplicative and that the addition of a second biomarker adds no additional value to the prognostic power of a given pair of biomarkers. In order to optimize the prognostic power of a given panel of biomarkers it is also desirable to reduce the amount of signal "noise" by minimizing the use of biomarkers that provide duplicative prognostic information when compared to another biomarker in the panel. Methods for determining statistical independence are known in the art. Statistical independence of biomarkers of interest can be assessed using any method . Where independent, prognostic biomarkers are used to practice the present methods, the prognostic value is increased by detecting the expression of 2, 3, 4, 5, 6, 7 or more biomarkers. In such cases, any combination of independent biomarkers can be used.

One of skill in the art will also recognize that a panel of biomarkers can be used to evaluate the prognosis of a breast cancer patient in accordance with the methods of the invention. A panel comprises at least five statistically independent, prognostic biomarkers.

In particular embodiments, the methods for evaluating breast cancer prognosis comprise collecting a patient body sample, preferably a breast tissue sample, more preferably a primary breast tumor tissue sample, contacting the sample with at least five antibodies specific for a biomarker of interest, detecting antibody binding, and determining if the biomarker is overexpressed. That is, samples are incubated with the biomarker antibody for a time sufficient to permit the formation of antibody-antigen complexes, and antibody binding is detected, for example, by a labeled secondary antibody. Samples that exhibit overexpression of at least one bad outcome biomarker, as determined by antibody binding, are classified as having a poor prognosis. Similarly, patient samples that display overexpression of at least one good outcome biomarker are categorized as having a good prognosis. Furthermore, the overexpression of certain combinations of biomarkers of interest is specifically used to distinguish breast cancer patients with a poor prognosis from those with a good prognosis. In some aspects of the invention, the methods comprise detecting the expression of five or more biomarkers in a patient sample and determining if said biomarkers are overexpressed, wherein overexpression of all or some subset of these biomarkers is indicative of breast cancer prognosis. The methods of the invention comprise detecting the expression of SLPI, p21ras, E2F1, PSMB9, and src, wherein overexpression of all five of these biomarkers is indicative of a poor prognosis.

By "body sample" is intended any sampling of cells, tissues, or bodily fluids in which expression of a biomarker can be detected. Examples of such body samples include but are not limited to blood, lymph, urine, gynecological fluids, biopsies, and smears. Bodily fluids useful in the present invention include blood, urine, saliva, nipple aspirates, or any other bodily secretion or derivative thereof. Blood can include whole blood, plasma, serum, or any derivative of blood. In preferred embodiments, the body sample comprises breast cells, particularly breast tissue from a biopsy, more particularly a breast tumor tissue sample. Body samples may be obtained from a patient by a variety of techniques including, for example, by scraping or swabbing an area, by using a needle to aspirate bodily fluids, or by removing a tissue sample (i.e., biopsy). Methods for collecting various body samples are well known in the art. In some embodiments, a breast tissue sample is obtained by, for example, fine needle aspiration biopsy, core needle biopsy, or excisional biopsy. Fixative and staining solutions may be applied to the cells or tissues for preserving the specimen and for facilitating examination. Body samples, particularly breast tissue samples, may be transferred to a glass slide for viewing under magnification. In preferred embodiments, the body sample is a formalin-fixed, paraffin embedded breast tissue sample, particularly a primary breast tumor sample.

Any methods available in the art for detecting expression of biomarkers are encompassed herein. The expression of a biomarker of the invention can be detected on a protein level. By "detecting expression" is intended determining the quantity or presence of a biomarker protein. Thus, "detecting expression" encompasses instances where a biomarker is determined not to be expressed, not to be detectably expressed, expressed at a low level, expressed at a normal level, or overexpressed. In order to determine overexpression, the body sample to be examined may be compared with a corresponding body sample that originates from a healthy person. That is, the "normal" level of expression is the level of expression of the biomarker in, for example, a breast tissue sample from a human subject or patient not afflicted with breast cancer. Such a sample can be present in standardized form. In some embodiments, determination of biomarker overexpression requires no comparison between the body sample and a corresponding body sample that originates from a healthy person. For example, detection of overexpression of a biomarker indicative of a poor prognosis in a breast tumor sample may preclude the need for comparison to a corresponding breast tissue sample that originates from a healthy person. Moreover, in some aspects of the invention, no expression, underexpression, or normal expression (i.e., the absence of overexpression) of a biomarker or combination of biomarkers of interest provides useful information regarding the prognosis of a breast cancer patient.

Methods for detecting expression of the biomarkers of the invention comprise any methods that determine the quantity or the presence of the biomarkers either at the protein level. Such methods are well known in the art and include but are not limited to western blots, northern blots, southern blots, ELISA, immunoprecipitation, immunofluorescence, flow cytometry and immunohistochemistry. In particular embodiments, expression of a biomarker is detected on a protein level using, for example, antibodies that are directed against specific biomarker proteins. These antibodies can be used in various methods such as Western blot, ELISA, immunoprecipitation, or immunohistochemistry techniques. Likewise, immunostaining of breast tissue, particularly breast tumor tissue, can be combined with assessment of clinical information, conventional prognostic methods, and expression of molecular markers (e.g., Her2/neu, Ki67, p53, and hormone receptor status) known in the art. In this manner, the disclosed methods may permit the more accurate determination of breast cancer prognosis.

One aspect of the present invention provides an immunohistochemistry technique for evaluating the prognosis of a breast cancer patient Specifically, this method comprises antibody staining of biomarkers within a breast tissue sample, more particularly a breast tumor sample, that are indicative of prognosis. One of skill in the art will recognize that the immunohistochemistry methods described herein below may be performed manually or in an automated fashion using, for example, the Autostainer Universal Staining System (Dako). One protocol for antibody staining (i.e., immunohistochemistry) of breast tissue samples is provided in Example 1.

In one immunohistochemistry method, a patient breast tissue sample is collected by, for example, biopsy techniques known in the art. Samples may be frozen for later preparation or immediately placed in a fixative solution. Tissue samples may be fixed by treatment with a reagent such as formalin, gluteraldehyde, methanol, or the like and embedded in paraffin. Methods for preparing slides for immunohistochemical analysis from formalin-fixed, paraffin-embedded tissue samples are well known in the art.

In some embodiments, particularly the immunohistochemistry methods of the invention, samples may need to be modified in order to make the biomarker antigens accessible to antibody binding. For example, formalin fixation of tissue samples results in extensive cross-linking of proteins that can lead to the masking or destruction of antigen sites and, subsequently, poor antibody staining. As used herein, "antigen retrieval" or "antigen unmaksing" refers to methods for increasing antigen accessibility or recovering antigenicity in, for example, formalin-fixed, paraffin-embedded tissue samples. Any method for making antigens more accessible for antibody binding may be used in the practice of the invention, including those antigen retrieval methods known in the art. See, for example, Hanausek and Walaszek, eds. (1998) Tumor Marker Protocols (Humana Press, Inc., Totowa, New Jersey); and Shi et al., eds. (2000) Antigen Retrieval Techniques: Immunohistochemistry and Molecular Morphology (Eaton Publishing, Natick, MA)

Antigen retrieval methods include but are not limited to treatment with proteolytic enzymes (e.g., trypsin, chymoptrypsin, pepsin, pronase, etc.) or antigen retrieval solutions. Antigen retrieval solutions of interest include, for example, citrate buffer, pH 6.0 (Dako), tris buffer, pH 9.5 (Biocare), BDTA, pH 8.0 (Biocare), L.A.B. ("Liberate Antibody Binding Solution;" Polysciences), antigen retrieval Glyca solution (Biogenex), citrate buffer solution, pH 4.0 (Zymed), Dawn® detergent (Proctor & Gamble), deionized water, and 2% glacial acetic acid. In some embodiments, antigen retrieval comprises applying the antigen retrieval solution to a formalin-fixed tissue sample and then heating the sample in an oven (e.g., 60°C), steamer (e.g., 95°C), or pressure cooker (e.g., 120°C) at specified temperatures for defined time periods. In other aspects of the invention, antigen retrieval may be performed at room temperature. Incubation times will vary with the particular antigen retrieval solution selected and with the incubation temperature. For example, an antigen retrieval solution may be applied to a sample for as little as 5, 10, 20, or 30 minutes or up to overnight. The design of assays to determine the appropriate antigen retrieval solution and optimal incubation times and temperatures is standard and well within the routine capabilities of those of ordinary skill in the art.

Following antigen retrieval, samples are blocked using an appropriate blocking agent, e.g., hydrogen peroxide. An antibody directed to a biomarker of interest is then incubated with the sample for a time sufficient to permit antigen-antibody binding. The antibodies of the invention may be added to a single sample sequentially as individual antibody reagents or simultaneously as an antibody cocktail. Alternatively, each individual antibody may be added to a separate tissue section from a single patient sample, and the resulting data pooled.

Techniques for detecting antibody binding are well known in the art. Antibody binding to a biomarker of interest may be detected through the use of chemical reagents that generate a detectable signal that correspond to the level of antibody binding and, accordingly, to the level of biomarker protein expression. For example, antibody binding can be detected through the use of a secondary antibody that is conjugated to a labeled polymer. Examples of labeled polymers include but are not limited to polymer-enzyme conjugates. The enzymes in these complexes are typically used to catalyze the deposition of a chromogen at the antigen-antibody binding site, thereby resulting in cell staining that corresponds to expression level of the biomarker of interest. Enzymes of particular interest include horseradish peroxidase (HRP) and alkaline phosphatase (AP). Commercial antibody detection systems, such as, for example the Dako Envision+ system and Biocare Medical's Mach 3 system, may be used to practice the present invention.

In one immunohistochemistry method of the invention, antibody binding to a biomarker is detected through the use of an HRP-labeled polymer that is conjugated to a secondary antibody. Slides are stained for antibody binding using the chromogen 3,3-diaminobenzidine (DAB) and then counterstained with hematoxylin and, optionally, a bluing agent such as ammonium hydroxide. In some aspects of the invention, slides are reviewed microscopically by a pathologist to assess cell staining (i.e., biomarker overexpression) and to evaluate breast cancer prognosis. Alternatively, samples may be reviewed via automated microscopy or by personnel with the assistance of computer software that facilitates the identification of positive staining cells.

The terms "antibody" and "antibodies" broadly encompass naturally occurring forms of antibodies and recombinant antibodies such as single-chain antibodies, chimeric and humanized antibodies and multi-specific antibodies as well as fragments and derivatives of all of the foregoing, which fragments and derivatives have at least an antigenic binding site. Antibody derivatives may comprise a protein or chemical moiety conjugated to the antibody.

"Antibodies" and "immunoglobulins" (Igs) are glycoproteins having the same structural characteristics. While antibodies exhibit binding specificity to an antigen, immunoglobulins include both antibodies and other antibody-like molecules that lack antigen specificity. Polypeptides of the latter kind are, for example, produced at low levels by the lymph system and at increased levels by myelomas.

The term "antibody" is used in the broadest sense and covers fully assembled antibodies, antibody fragments that can bind antigen (e.g., Fab', $F'(ab)_2$, Fv, single chain antibodies, diabodies), and recombinant peptides comprising the foregoing.

The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Antibody fragments" comprise a portion of an intact antibody, preferably the antigen-binding or variable region of the intact antibody. Examples of antibody fragments include Fab, Fab', F(ab')2, and Fv fragments; diabodies; linear antibodies (Zapata et al. (1995) Protein Eng. 8(10):1057-1062); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize 35 readily. Pepsin treatment yields an F(ab')2 fragment that has two antigen-combining sites and is still capable of cross-linking antigen.

"Fv" is the minimum antibody fragment that contains a complete antigen recognition and binding site. In a two-chain Fv species, this region consists of a dimer of one heavy- and one light-chain variable domain in tight, non-covalent association. In a single-chain Fv species, one heavy- and one light-chain variable domain can be covalently linked by flexible peptide linker such that the light and heavy chains can associate in a "dimeric" structure analogous to that in a two-chain Fv species. It is in this configuration that the three CDRs of each variable domain interact to define an antigen-binding site on the surface of the $V_H$-$V_L$ dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain ($C_H1$) of the heavy chain. Fab fragments differ from Fab' fragments by the addition of a few residues at the carboxy terminus of the heavy-chain $C_H1$ domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments that have hinge cysteines between them.

Monoclonal antibodies can be prepared using the method of Kohler et al. (1975) Nature 256:495-496, or a modification thereof. Typically, a mouse is immunized with a solution containing an antigen. Immunization can be performed by mixing or emulsifying the antigen-containing solution in saline, preferably in an adjuvant such as Freund's complete adjuvant, and injecting the mixture or emulsion parenterally. Any method of immunization known in the art may be used to obtain the monoclonal antibodies of the invention. After immunization of the animal, the spleen (and optionally, several large lymph nodes) are removed and dissociated into single cells. The spleen cells may be screened by applying a cell suspension to a plate or well coated with the antigen of interest. The B cells expressing membrane bound immunoglobulin specific for the antigen bind to the plate and are not rinsed away. Resulting B cells, or all dissociated spleen cells, are then induced to fuse with myeloma cells to form hybridomas, and are cultured in a selective medium. The resulting cells are plated by serial dilution and are assayed for the production of antibodies that specifically bind the antigen of interest (and that do not bind to unrelated antigens). The selected monoclonal antibody (mAb)-secreting hybridomas are then cultured either *in vitro* (e.g., in tissue culture bottles or hollow fiber reactors), or *in vivo* (as ascites in mice).

As an alternative to the use of hybridomas, antibody can be produced in a cell line such as a CHO cell line, as disclosed in U.S. Patent Nos. 5,545,403; 5,545,405; and 5,998,144 . Briefly the cell line is transfected with vectors capable of expressing a light chain and a heavy chain, respectively. By transfecting the two proteins on separate vectors, chimeric antibodies can be produced. Another advantage is the correct glycosylation of the antibody. A monoclonal antibody can also be identified and isolated by screening a recombinant combinatorial immunoglobulin library (e.g., an antibody phage display library) with a biomarker protein to thereby isolate immunoglobulin library members that bind the biomarker protein. Kits for generating and screening phage display libraries are commercially available (e.g., the Pharmacia *Recombinant Phage Antibody System*, Catalog No. 27-9400-01; and the Stratagene *SurfZAP9 Phage Display*

*Kit*, Catalog No. 240612). Additionally, examples of methods and reagents particularly amenable for use in generating and screening antibody display library can be found in, for example, U.S. Patent No. 5,223,409; PCT Publication Nos. WO 92/18619; WO 91/17271; WO 92/20791; WO 92/15679; 93/01288; WO 92/01047; 92/09690; and 90/02809; Fuchs et al. (1991) Bio/Technology 9:1370-1372; Hay et al. (1992) Hum. Antibod. Hybridomas 3:81-85; Huse et al. (1989) Science 246:1275-1281; Griffiths et al. (1993) EMBO J. 12:725-734.

Polyclonal antibodies can be prepared by immunizing a suitable subject (e.g., rabbit, goat; mouse, or other mammal) with a biomarker protein immunogen. The antibody titer in the immunized subject can be monitored over time by standard techniques, such as with an enzyme linked immunosorbent assay (ELISA) using immobilized biomarker protein. At an appropriate time after immunization, e.g., when the antibody titers are highest, antibody-producing cells can be obtained from the subject and used to prepare monoclonal antibodies by standard techniques, such as the hybridoma technique originally described by Kohler and Milstein (1975) Nature 256:495-497, the human B cell hybridoma technique (Kozbor et al. (1983) Immunol. Today 4:72), the EBV-hybridoma technique (Cole et al. (1985) in Monoclonal Antibodies and Cancer Therapy, ed. Reisfeld and Sell (Alan R. Liss, Inc., New York, NY), pp. 77-96) or trioma techniques. The technology for producing hybridomas is well known (*see* generally Coligan et al., eds. (1994) Current Protocols in Immunology (John Wiley & Sons, Inc., New York, NY); Galfre et al. (1977) Nature 266:55052; Kenneth (1980) in Monoclonal Antibodies: A New Dimension In Biological Analyses (Plenum Publishing Corp., NY; and Lerner (1981) Yale J. Biol. Med., 54:387-402).

The compositions of the invention further comprise monoclonal antibodies and fragments thereof that specifically bind to biomarker proteins of interest. For example, monoclonal antibodies specific for SLPI (designated clone 506.24), DARPP-32 (8G11.20), MGC 14832 (1F3.9 and 2D1.14), NDRG-1 (10A9.34), PSMB9 (3A2.4), and MUC-1 (16E3.3) are provided. The monoclonal antibodies may be labeled with a detectable substance as described below to facilitate biomarker protein detection in the sample. Such antibodies find use in practicing the methods of the invention. Monoclonal antibodies having the binding characteristics of the antibodies disclosed herein are also encompassed by the present invention. Compositions further comprise antigen-binding fragments of the monoclonal antibodies, hybridoma cell lines producing these antibodies, and isolated nucleic acid molecules encoding the amino acid sequences of these monoclonal antibodies.

Antibodies having the binding characteristics of a monoclonal antibody of the invention are also provided. "Binding characteristics" or "binding specificity" when used in reference to an antibody means that the antibody recognizes the same or similar antigenic epitope as a comparison antibody. Examples of such antibodies include, for example, an antibody that competes with a monoclonal antibody of the invention in a competitive binding assay. One of skill in the art could determine whether an antibody competitively interferes with another antibody using standard methods.

By "epitope" is intended the part of an antigenic molecule to which an antibody is produced and to which the antibody will bind. Epitopes can comprise linear amino acid residues (i.e., residues within the epitope are arranged sequentially one after another in a linear fashion), nonlinear amino acid residues (referred to herein as "nonlinear epitopes"; these epitopes are not arranged sequentially), or both linear and nonlinear amino acid residues. Typically epitopes are short amino acid sequences, e.g. about five amino acids in length. Systematic techniques for identifying epitopes are known in the art and are described, for example, in U.S. Pat. No. 4,708,871. Briefly, a set of overlapping oligopeptides derived from the antigen may be synthesized and bound to a solid phase array of pins, with a unique oligopeptide on each pin. The array of pins may comprise a 96-well microtiter plate, permitting one to assay all 96 oligopeptides simultaneously, e.g., for binding to a biomarker-specific monoclonal antibody. Alternatively, phage display peptide library kits (New England BioLabs) are currently commercially available for epitope mapping. Using these methods, the binding affinity for every possible subset of consecutive amino acids may be determined in order to identify the epitope that a given antibody binds. Epitopes may also be identified by inference when epitope length peptide sequences are used to immunize animals from which antibodies are obtained.

Antigen-binding fragments and variants of the monoclonal antibodies disclosed herein are further provided. Such variants will retain the desired binding properties of the parent antibody. Methods for making antibody fragments and variants are generally available in the art For example, amino acid sequence variants of a monoclonal antibody described herein, can be prepared by mutations in the cloned DNA sequence encoding the antibody of interest. Methods for mutagenesis and nucleotide sequence alterations are well known in the art. See, for example, Walker and Gaastra, eds. (1983) Techniques in Molecular Biology (MacMillan Publishing Company, New York); Kunkel (1985) Proc. Natl. Acad. Sci. USA 82:488-492; Kunkel et al. (1987) Methods Enzymol. 154:367-382; Sambrook et al. (1989) Molecular Cloning: A Laboratory Manual (Cold Spring Harbor, New York); U.S. Patent No. 4,873,192; and the references cited therein . Guidance as to appropriate amino acid substitutions that do not affect biological activity of the polypeptide of interest may be found in the model of Dayhoff et al. (1978) in Atlas of Protein Sequence and Structure (Natl. Biomed. Res. Found., Washington, D.C.). Conservative substitutions, such as exchanging one amino acid with another having similar properties, may be preferred. Examples of conservative substitutions include, but are not limited to, Gly⇔Ala, Val⇔Ile⇔Leu, Asp⇔Glu, Lys⇔Arg, Asn⇔Gln, and Phe⇔Trp⇔Tyr.

In constructing variants of the antibody polypeptide of interest, modifications are made such that variants continue to possess the desired activity, i.e., similar binding affinity to the biomarker. Obviously, any mutations made in the DNA encoding the variant polypeptide must not place the sequence out of reading frame and preferably will not create complementary regions that could produce secondary mRNA structure. See EP Patent Application Publication No. 75,444.

Preferably, variants of a reference biomarker antibody have amino acid sequences that have at least 70% or 75% sequence identity, preferably at least 80% or 85% sequence identity, more preferably at least 90%, 91%, 92%, 93%, 94% or 95% sequence identity to the amino acid sequence for the reference antibody molecule, or to a shorter portion of the reference antibody molecule. More preferably, the molecules share at least 96%, 97%, 98% or 99% sequence identity. For purposes of the present invention, percent sequence identity is determined using the Smith-Waterman homology search algorithm using an affine gap search with a gap open penalty of 12 and a gap extension penalty of 2, BLOSUM matrix of 62. The Smith-Waterman homology search algorithm is taught in Smith and Waterman (1981) Adv. Appl. Math. 2:482-489. A variant may, for example, differ from the reference antibody by as few as 1 to 15 amino acid residues, as few as 1 to 10 amino acid residues, such as 6-10, as few as 5, as few as 4, 3, 2, or even 1 amino acid residue.

With respect to optimal alignment of two amino acid sequences, the contiguous segment of the variant amino acid sequence may have additional amino acid residues or deleted amino acid residues with respect to the reference amino acid sequence. The contiguous segment used for comparison to the reference amino acid sequence will include at least 20 contiguous amino acid residues, and may be 30, 40, 50, or more amino acid residues. Corrections for sequence identity associated with conservative residue substitutions or gaps can be made (see Smith-Waterman homology search algorithm).

The antibodies used to practice the invention are selected to have specificity for the biomarker proteins of interest. Methods for making antibodies and for selecting appropriate antibodies are known in the art. See, for example, Celis, ed. (in press) Cell Biology & Laboratory Handbook, 3rd edition (Academic Press, New York). In some embodiments, commercial antibodies directed to specific biomarker proteins may be used to practice the invention. The antibodies of the invention may be selected on the basis of desirable staining of histological samples. That is, in preferred embodiments the antibodies are selected with the end sample type (e.g., formalin-fixed, paraffin-embedded breast tumor tissue samples) in mind and for binding specificity.

In some aspects of the invention, antibodies directed to specific biomarkers of interest are selected and purified via a multi-step screening process. In particular embodiments, polydomas are screened to identify biomarker-specific antibodies that possess the desired traits of specificity and sensitivity. As used herein, "polydoma" refers to multiple hybridomas. The polydomas of the invention are typically provided in multi-well tissue culture plates. In the initial antibody screening step, a set of individual slides or tumor tissue microarrays comprising normal (i.e., non-cancerous) breast tissue and stage I, II, III, and IV breast tumor samples is used. Methods and equipment, such as the Chemicon® Advanced Tissue Arrayer, for generating arrays of multiple tissues on a single slide are known in the art. See, for example, U.S. Pat. No. 4,820,504. Undiluted supernatants from each well containing a polydoma are assayed for positive staining using standard immunohistochemistry techniques. At this initial screening step, background, non-specific binding is essentially ignored. Polydomas producing positive staining are selected and used in the second phase of antibody screening.

In the second screening step, the positive polydomas are subjected to a limiting dilution process. The resulting unscreened antibodies are assayed via standard immunohistochemistry techniques for positive staining of breast tumor tissue samples with known 5-year outcomes. To do this, tissue microarrays comprising normal breast tissue, early-stage breast tumor samples with known good 5-year outcomes, early-stage breast tumor samples with known bad 5-year outcomes, normal non-breast tissue, and cancerous non-breast tissue are generated. At this stage, background staining is relevant, and the candidate polydomas that stain positive for abnormal cells (i.e., cancer cells) only are selected for further analysis to identify antibodies that differentiate good and bad outcome patient samples.

Positive-staining cultures are prepared as individual clones in order to select individual candidate monoclonal antibodies. Methods for isolating individual clones and for purifying antibodies through affinity adsorption chromatography are well known in the art. Individual clones are further analyzed to determine the optimized antigen retrieval conditions and working dilution.

One of skill in the art will recognize that optimization of staining reagents and conditions, for example, antibody titer and detection chemistry parameters, is needed to maximize the signal to noise ratio for a particular antibody. Antibody concentrations that maximize specific binding to the biomarkers of the invention and minimize non-specific binding (or "background") will be determined. In particular embodiments, appropriate antibody titers are determined by initially testing various antibody dilutions on formalin-fixed, paraffin-embedded normal and cancerous breast tissue samples. The design of assays to optimize antibody titer and detection conditions is standard and well within the routine capabilities of those of ordinary skill in the art. Some antibodies require additional optimization to reduce background staining and/or to increase specificity and sensitivity of staining.

Furthermore, one of skill in the art will recognize that the concentration of a particular antibody used to practice the methods of the invention will vary depending on such factors as time for binding, level of specificity of the antibody for the biomarker protein, and method of body sample preparation. Moreover, when multiple antibodies are used in a single sample, the required concentration may be affected by the order in which the antibodies are applied to the sample, i.e., simultaneously as a cocktail or sequentially as individual antibody reagents. Furthermore, the detection chemistry used to visualize antibody binding to a biomarker of interest must also be optimized to produce the desired signal to noise ratio. One example of optimization of staining reagents and conditions for immunohistochemistry is described in Example 6.

Detection of antibody binding can be facilitated by coupling the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include $^{125}I$, $^{131}I$, $^{35}S$, or $^{3}H$.

In regard to detection of antibody staining in the immunohistochemistry methods of the invention, there also exist in the art, video-microscopy and software methods for the quantitative determination of an amount of multiple molecular species (e.g., biomarker proteins) in a biological sample wherein each molecular species present is indicated by a representative dye marker having a specific color. Such methods are also known in the art as a colorimetric analysis methods. In these methods, video-microscopy is used to provide an image of the biological sample after it has been stained to visually indicate the presence of a particular biomarker of interest. Some of these methods use an imaging system and associated software to determine the relative amounts of each molecular species present based on the presence of representative color dye markers as indicated by those color dye markers' optical density or transmittance value, respectively, as determined by an imaging system and associated software. These techniques provide quantitative determinations of the relative amounts of each molecular species in a stained biological sample using a single video image that is "deconstructed" into its component color parts.

The methods of the invention can be used in conjunction with imaging systems and associated imaging software for the detection of biomarker expression. Biomarkers for use in the methods of the invention can be selected based on methods and computer programs.

Kits for practicing the methods of the invention are further provided. By "kit" is intended any manufacture (e.g., a package or a container) comprising at least one reagent, e.g. an antibody, a nucleic acid probe, etc. for specifically detecting the expression of a biomarker of the invention. The kit may be promoted, distributed, or sold as a unit for performing the methods of the present invention. Additionally, the kits may contain a package insert describing the kit and methods for its use.

In particular embodiments, kits for practicing the immunohistochemistry methods of the invention are provided. Such kits are compatible with both manual and automated immunohistochemistry techniques (e.g., cell staining) as described herein below in Example 1. These kits comprise at least fine antibodies directed to a biomarker protein of interest. Chemicals for the detection of antibody binding to the biomarker, a counterstain, and a bluing agent to facilitate identification of positive staining cells are optionally provided. Alternatively, the immunochemistry kits of the present invention are used in conjunction with commercial antibody binding detection systems, such as, for example the Dako Envision+ system and Biocare Medical's Mach 3 system. Any chemicals that detect antigen-antibody binding may be used in the practice of the invention. In some embodiments, the detection chemicals comprise a labeled polymer conjugated to a secondary antibody. For example, a secondary antibody that is conjugated to an enzyme that catalyzes the deposition of a chromogen at the antigen-antibody binding site may be provided. Such enzymes and techniques for using them in the detection of antibody binding are well known in the art. In one embodiment, the kit comprises a secondary antibody that is conjugated to an HRP-labeled polymer. Chromogens compatible with the conjugated enzyme (e.g., DAB in the case of an HRP-labeled secondary antibody) and solutions, such as hydrogen peroxide, for blocking non-specific staining may be further provided. The kits of the present invention may also comprise a counterstain, such as, for example, hematoxylin. A bluing agent (e.g., ammonium hydroxide) may be further provided in the kit to facilitate detection of positive staining cells.

The immunohistochemistry kits of the invention comprise reagents, e.g., antibodies, for specifically detecting the expression of at least two distinct biomarkers. Each antibody may be provided in the kit as an individual reagent or, alternatively, as an antibody cocktail comprising all of the antibodies directed to the different biomarkers of interest. Furthermore, any or all of the kit reagents may be provided within containers that protect them from the external environment, such as in sealed containers. Positive and/or negative controls may be included in the kits to validate the activity and correct usage of reagents employed in accordance with the invention. Controls may include samples, such as tissue sections, cells fixed on glass slides, etc., known to be either positive or negative for the presence of the biomarker of interest. The design and use of controls is standard and well within the routine capabilities of those of ordinary skill in the art. Kits for evaluating the prognosis of a breast cancer patient as described comprising detecting biomarker overexpression at the nucleic acid level are further provided. Such kits comprise, for example, at least one nucleic acid probe that specifically binds to a biomarker nucleic acid or fragment thereof. The kits comprise at least two nucleic acid probes that hybridize with distinct biomarker nucleic acids.

One of skill in the art will appreciate that any or all steps in the methods of the invention could be implemented by personnel or, alternatively, performed in an automated fashion. Thus, the steps of body sample preparation, sample staining, and detection of biomarker expression may be automated. Moreover, in some embodiments, the immunohistochemical methods of the invention are used in conjunction with computerized imaging equipment and software to facilitate the identification of positive-staining cells by a pathologist. The methods disclosed herein can also be combined with other prognostic methods or analyses (e.g., tumor size, lymph node status, expression levels of Her2/neu, Ki67, and p53). In this manner detection of overexpression of the biomarkers of the invention can permit a more accurate determination of the prognosis of a breast cancer patient.

The article "a" and "an" are used herein to refer to one or more than one (i.e., to at least one) of the grammatical object of the article. By way of example, "an element" means one or more element.

Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps.

The following examples are offered by way of illustration and not by way of limitation:

EXPERIMENTAL

**Example 1: Detection of Biomarker Overexpression Using Immunohistochemistry**

Slide Preparation

4 μM sections of formalin-fixed, paraffin-embedded breast tumor tissue samples are cut using a microtome and placed on SuperFrost+ slides (VWR). The slides are baked in a forced air oven for 20 minutes and then contacted with a Histo-Orienter until the paraffin melts. Slides are washed three times with xylene for 5 minutes to remove paraffin and then rinsed three times in absolute alcohol at 2 minutes/rinse.

Pretreatment and Antigen Retrieval

To prevent non-specific background staining, the slides are incubated in a hydrogen peroxide/methanol block for five minutes at room temperature. Slides are then rinsed thoroughly with several changes of $dH_2O$.

In order to make the antigens accessible to antibody binding, slides are incubated in an antigen retrieval solution in a pressure cooker for 5 minutes. Slides are allowed to cool to room temperature for 20 minutes on the bench, and the citrate buffer is gradually replaced with $dH_2O$, tris buffered saline (TBS), or phosphate buffered saline (PBS) by successive dilutions. The slides are then rinsed three times in TBS at 2 minutes per rinse. To break the surface tension, 750 μ/50 ml of 1% BSA/TBS is added to each slide.

Manual Immunohistochemistry

To prevent non-specific background staining, slides are not permitted to dry out during the staining procedure. Slides that have been subjected to antigen retrieval are loaded into a humidity chamber filled with water moistened paper towels. A SLPI antibody (clone 5G6.24; 1:100 dilution) is applied to the slide in a volume sufficient to completely cover the tissue section for 1 hour at room temperature. Following incubation with the primary antibody, the slides are rinsed three times in TBS at 2 minutes per wash. 750 μl/50 ml of 1% BSA/TBS is added to the final wash.

The Dako Envision+ HRP-labeled polymer secondary antibody is applied to the slide for 30 minutes at room temperature, followed by a TBS rinse. The HRP substrate chromogen DAB is applied for 10 minutes, and then the slides are rinsed for 5 minutes with water. Each slide is counterstained with hematoxylin for 5 seconds and then rinsed with water until clear. Following counterstaining, the slides are "blued" by soaking in ammonia water for 10 seconds and then rinsed with water for 1 minute.

Samples are dehydrated by immersing the slides in 95% ethanol for 1 minute and then in absolute ethanol for an additional minute. Slides are cleared by rinsing 3 times in xylene for 1 minute per rinse. Slides are then coverslipped with permanent mounting media and incubated at 35°C to dry. Biomarker staining is visualized using a bright-field microscope. Scoring is performed by a board certified pathologist in a blind manner.

Automated Immunohistochemistry

The Dako Autostainer Universal Staining system is programmed according to the manufacturer's instructions, and the necessary staining and counterstaining reagents described above for manual immunohistochemistry are loaded onto the machine. The prepared slides are loaded onto the Autostainer, and the program is run. At the end of the run, the slides are removed and rinsed in water for 5 minutes. The slides are dehydrated, cleared, coverslipped, and analyzed as described above.

**Example 2: Detection of Overexpression of Individual Biomarkers in Clinical Samples**

Approximately 130 breast tumor tissue samples from patients at various disease stages were collected. The average patient age was 77. Actual clinical outcome data for each patient was known, and each patient was categorized as having a good or bad outcome. In this study, good outcome was defined as remaining cancer-free for at least 5 years; bad outcome was defined as suffering disease relapse, recurrence, or death within 5 years. The following table indicates the number of samples within each diagnosis group analyzed, as well as actual clinical outcome data.

Table 1: Clinical Samples Analyzed

| Stage | Good Outcome | Bad Outcome | Total |
|---|---|---|---|
| T1N0 | 50 | 13 | 63 |
| T1N1 | 6 | 4 | 10 |
| T2N0 | 26 | 19 | 45 |
| T2N1 | 9 | 7 | 16 |
| T3N0 | 0 | 3 | 3 |
| T3N1 | 0 | 1 | 1 |
| | | | |
| **Lymph Node Status** | **Good Outcome** | | **Bad Outcome** |
| N0 | 76 | | 35 |
| N1 | 15 | | 12 |

The samples were analyzed by the automated immunohistochemistry described in Example 1 to identify biomarkers whose overexpression is indicative of a bad cancer prognosis. That is, the goal of this clinical study was to identify biomarkers that can distinguish good and bad outcome patient samples. Antibodies were used to detect the overexpression of eight biomarkers of interest: SLPI, PSMB9, NDRG-1, E2F1, p21ras, MUC-1, phospho-p27, and src. For quality control purposes, samples were also analyzed for ER, PR, p53, Ki67, and Her2/neu expression

Commercial antibodies or monoclonal antibodies, identified by polydoma screening as described herein, directed to the biomarkers of interest were diluted as indicated in Table 2 and used to detect biomarker overexpression. The antigen retrieval conditions for each biomarker are also listed below.

Table 2: Antibody Dilutions and Antigen Retrieval Conditions

| Biomarker | Antibody (Dilution) | Antigen Retrieval Conditions |
|---|---|---|
| SLPI | Clone 5G6.24 (1:100) | Citrate buffer, pH 6.0/pressure cooker |
| PSMB9 | Clone 3A2.4 (1:500) | Citrate buffer, pH 4.0/steamer |
| NDRG-1 | Zymed (1:200) | Citrate buffer, pH 4.0/steamer |
| E2F1 | Calbiochem (1:50) | Tris, pH 9.5/pressure cooker |
| p21ras | Dako (1:50) | Citrate buffer, pH 4.0/steamer |
| MUC-1 | Clone 16E3.3 (1:400) | Citrate buffer, pH 4.0/steamer |
| phospho-p27 | Zymed (1:100) | EDTA, pH 8.0/steamer |
| src | Upstate (1:50) | Citrate buffer, pH 4.0/steamer |

Interpretation of Slides

Each slide was reviewed and scored by a board certified pathologist that was unaware of the actual clinical patient outcomes. Samples were scored for biomarker staining intensity on a scale of 0-3. See, for example, Hanausek and Walaszek, eds. (1998) Tumor Marker Protocols (Humana Press, Inc., Totowa, New Jersey); and Shi et al., eds. (2000) Antigen Retrieval Techniques: Immunohistochemistry and Molecular Morphology (Eaton Publishing, Natick, MA) . For each biomarker, a threshold staining intensity was established. Samples exhibiting a staining intensity of less than this threshold value for a particular biomarker were deemed negative for that biomarker. The staining intensity threshold values for the biomarkers of interest were as follows:

Src: ≥1
MUC-1: ≥3
Phospho-p27: ≥0.5
PSMB9: ≥0.5
NDRG-1: ≥1
E2F1: ≥3
p21ras: ≥0.5
SLPI: ≥2

The staining intensity results were compared with the known actual clinical outcome data available for each patient, and each slide was then given a final result of true positive (TP), true negative (TN), false positive (FP), false negative (FN), according to the parameters described below. Sensitivity and specificity values for each biomarker were calculated.

Table 3: Slide Classification for Bad Outcome Biomarkers

| | Biomarker Staining | Actual Clinical Outcome* |
|---|---|---|
| **True Positive** | Positive | Bad outcome |
| **True Negative** | Negative | Good outcome |
| **False Positive** | Positive | Good outcome |
| **False Negative** | Negative | Bad outcome |
| *Good clinical outcome = cancer-free survival for at least 5 years<br>Bad clinical outcome = recurrence or death from the underlying cancer within 5 years | | |

Calculations Used

$$Sensitivity = TP/(TP + FN)$$

$$Specificity = TN/(FP + TN)$$

$$\text{Positive Predictive Power (PPP)} = TP/(TP + FP)$$

$$\text{Negative Predictive Power (NPP)} = TN/(FN + TN)$$

Results

The results for each biomarker are summarized below.

Table 4: Summary of Results with Individual Biomarkers

| | Src | MUC-1 | Phospho-p27 | PSMB9 | NDRG-1 | E2F1 | p21ras | SLPI |
|---|---|---|---|---|---|---|---|---|
| TP | 8 | 7 | 7 | 13 | 7 | 3 | 10 | 5 |
| FP | 8 | 4 | 6 | 15 | 14 | 4 | 11 | 7 |
| FN | 35 | 37 | 44 | 30 | 31 | 34 | 30 | 39 |
| TN | 59 | 70 | 76 | 54 | 54 | 57 | 60 | 64 |
| | | | | | | | | |
| Sensitivity | 18.60% | 15.91% | 13.73% | 30.23% | 18.42% | 8.11% | 25.00% | 11.36% |
| Specificity | 88.06% | 94.59% | 92.68% | 78.26% | 79.41% | 93.44% | 84.51% | 90.14% |

**Example 3: Detection of Biomarker Overexpression in Clinical Samples-Combining Biomarkers**

In order to determine if the sensitivity and specificity of the methods of the invention could be improved if multiple biomarkers were combined, the data from Example 2 was subjected to further analysis. Thus, various combinations of biomarkers were considered, and samples that stained positive for any of the biomarkers in the combination of interest were deemed positive. These results were compared with the known actual clinical outcome data available for each patient, and each slide was then given a final result of true positive (TP), true negative (TN), false positive (FP), false negative (FN) as before. Sensitivity, specificity, positive predictive value (PPV), and negative predictive values (NPV) for each combination of biomarkers were calculated.

Results

The results for each combination of biomarkers are summarized below.

Table 5: SLPI, PSMB9, MUC-1, and phospho-p27

| | |
|---|---|
| **TP** | 24 |
| **FP** | 25 |
| **FN** | 23 |
| **TN** | 58 |
| | |
| **Sensitivity** | 51.06% |
| **Specificity** | 69.88% |
| **NPV** | 71.60% |
| **PPV** | 48.98% |

Table 6: SLPI, PSMB9, MUC-1, phospho-p27, and src

| | |
|---|---|
| **TP** | 28 |
| **FP** | 28 |
| **FN** | 24 |
| **TN** | 60 |
| | |
| **Sensitivity** | 53.85% |
| **Specificity** | 68.18% |

(continued)

| | |
|---|---|
| **NPV** | 71.43% |
| **PPV** | 50.00% |

Table 7: SLPI, PSMB9, MUC-1, phospho-p27, src, p21ras, E2F1, and NDRG-1

| | |
|---|---|
| **TP** | 33 |
| **FP** | 41 |
| **FN** | 19 |
| **TN** | 47 |
| | |
| **Sensitivity** | 63.46% |
| **Specificity** | 53.41% |
| **NPV** | 71.21% |
| **PPV** | 44.59% |

**Example 4: Detection of Overexpression of Individual Biomarkers in Clinical Samples Using Marker Analysis Research System (MARS)**

Over 200 patients were analyzed in this study. As summarized in Table 8 this population of patients was quite heterogeneous and exhibited tumors of different stages ranging from T1N0 to T3N0.

Table 8: Patient Population Analyzed

| Stage | Good | Bad | All |
|---|---|---|---|
| T1N0 | 60 | 20 | 80 |
| T1N1 | 6 | 7 | 13 |
| T2N0 | 59 | 39 | 98 |
| T3N0 | 6 | 10 | 16 |
| Totals | **131** | **76** | **207** |

The targeted characteristic of the patients was their good outcome or bad outcome status. In this study, good outcome patients were those still disease-free after five years; bad outcome patients were defined as patients with recurrence, relapse, or death within five years.

Biomarker Selection

The paradigm used for biomarker selection was that biomarker overexpression would capture some of he bad outcome patients and show a very high specificity. Combining different markers would therefore ensure high specificity and gain sensitivity to reach, for example, an 80% sensitivity and 80% specificity. After a multi-step selection process, nine biomarkers were selected for the current study. These markers are shown in Table 9, along with their respective subcellular localization.

Table 9: Biomarkers Analyzed

| Marker Name | Localization |
|---|---|
| E2F1 | Nucleus |
| MUC-1 (IF3.9) | Membrane |
| NDRG-1 (ZYMED CAP43) | Cytoplasm (Nucleus + Membrane) |
| $p21^{ras}$ | Cytoplasm |

(continued)

| Marker Name | Localization |
|---|---|
| p53 | Nucleus |
| Phospho p27 | Cytoplasm (Nucleus) |
| PSMB9 (3A2.4) | Cytoplasm |
| SLPI (5G6.24) | Cytoplasm |
| src | Cytoplasm |

Automated Immunohistochemistry

The patient samples were analyzed by automated immunohistochemistry, essentially as described in Example 1, to identify biomarkers whose overexpression is indicative of a bad cancer prognosis. That is, the goal of this clinical study was to identify biomarkers that can distinguish good and bad outcome patient samples. Antibodies were used to detect the overexpression of the nine biomarkers of interest: SLPI, PSMB9, NDRG-1, E2F1, p21ras, p53, MUC-1, phospho-p27, and src. Samples were also analyzed for ER, PR, Ki67, and Her2/neu (CerbB2) expression.

Slides were prepared as described in Example 1 and subjected to antigen retrieval. Specifically, prepared slides were immersed in an antigen retrieval solution and then placed in a pressure cooker (120-125°C at 17-23 psi) for 5 minutes. The antigen retrieval solutions for each biomarker are listed below in Table 10.

Table 10: Antigen Retrieval Solutions

| Biomarker | Antigen Retrieval Solution |
|---|---|
| SLPI | Citrate pH 6.0 (Dako #S1699) |
| PSMB9 | EDTA (Biocare #CB917L) |
| NDRG-1 | Citrate pH 6.0 (Dako #S1699) |
| E2F1 | EDTA (Biocare #CB917L) |
| p21 ras | citrate buffer pH 6.0 (Dako #S1699 |
| MUC-1 | Citrate pH 6.0 (Dako #S1699) |
| phospho-p27 | deionized water |
| src | Tris pH 9.5 (Biocare CB911M) |

Slides were gradually returned to room temperature deionized water. The slides were rinsed 3 times in TBS/tween®-20 at 2 minutes per wash 200 μl of a biomarker-specific antibody was added to each slide and incubated at room temperature for one hour. Commercial antibodies or monoclonal antibodies, identified by polydoma screening as described herein, directed to the biomarkers of interest were used to detect biomarker overexpression.

Following incubation with the primary antibody, slides were rinsed twice with TBS/tween®-20. 200 μl of labeled polymer (Dako Envision+ HRP-labeled polymer secondary antibody) was then added for 30 minutes at room temperature. Slides were again rinsed 3 times with TBS/tween®-20 prior to the addition of 200 μl of DAB solution for five minutes at room temperature. The slides were then rinsed three times with TBS/tween®-20 and one time with deionized water. 200 μl of hematoxylin was added for 5 minutes. The slides were then rinsed 3 times with deionized water, one time with TBS/tween®-20, and 2 additional times with deionized water. The slides were dehydrated, cleared, and coverslipped as described in Example 1.

Pathologist Evaluation

A board certified pathologist manually scored the slides. p53 expression was scored for staining intensity using a scale of 0, 0.5, 1, 2, or 3, percentage of labeled cells, and a clinical diagnostic score. SLPI and PSMB9 were scored for staining intensity using a scale of 0, 0.5, 1, 2, or 3 and percentage of labeled cells. The pathologist also denoted on the slide the tumor area (ROI) used in making the determination. Up to ten individual 20x fields of view from within the selected regions for each tumor, organized in a single focus, were obtained using MARS. The actual number of images obtained from each sample was dependant on the size of the individual tumor. An Excel spreadsheet containing all of the above scoring information along with the patient outcome, lymph node status, and tumor size was generated. The data was subjected to further analysis as described below.

Data Extraction

Using MARS, the following steps were systematically performed for every file:

- Chromogen separation was optimized for each biomarker using the available slide that showed the best quality stain.
- Segmentation set up was customized for each biomarker according to its subcellular localization (nucleus, cytoplasm or membrane).
- Features were extracted at cell, field of view (FOV), and focus level, within the defined ROI and exported to an output file (XML format).

Data Analysis

A specific program named Multi Marker Analyzer was developed in order to integrate new analysis algorithms and meet the heavy computation needs for this analysis. This software provided a means to load all or a portion of either TMAs or tissue section XML files generated with MARS, to merge data contained in these files using XML files describing the TMA keys (in the case of a TMA analysis) or Excel files giving patient clinical status and patient evaluation (in the case of a tissue section analysis), and all the further analyzes. This merge process included the association of the parameters measured by MARS for each core (or patient) with the information kept in the TMA key (or the Excel file) about the patient: identification number and medical status (good or bad outcome) and the pathologist evaluation if not included in the XML formatted MARS file.

Because some of the samples did not go through the complete experimental process, the number of analyzed patients was smaller than the number of patients reported in Table 8 above and varies from one biomarker to another. The number of tissue sections analyzed for each biomarker is listed below in Table 11. The number of tissue samples analyzed for the conventional breast cancer markers (i.e., ER, PR, Ki67, and Her2/neu (CerbB2)) is in Table 12.

Table 11: Number of Tissue Sections Analyzed for Biomarker Overexpression

| Marker | Bad | Good | Total |
|---|---|---|---|
| E2F1 | 66 | 106 | 172 |
| MUC-1 | 65 | 108 | 173 |
| NDRG1 (CAP 43) | 75 | 115 | 190 |
| p21ras | 72 | 109 | 181 |
| p53 | 71 | 121 | 192 |
| Phospho-p27 | 70 | 115 | 185 |
| PSMB9 | 74 | 118 | 192 |
| SLPI | 75 | 118 | 193 |
| src | 66 | 108 | 174 |

Table 12: Number of Tissue Sections Analyzed for Conventional Breast Cancer Markers

| Marker | Bad | Good | Total |
|---|---|---|---|
| CerbB2 | 69 | 122 | 191 |
| ER | 70 | 123 | 193 |
| Ki67 | 69 | 124 | 193 |
| PR | 69 | 123 | 192 |

Segmentation and Dispatchers Setup

In order to bring MARS analysis closer to the pathologist manner of characterizing slides, only cells considered as being at least 1+ were selected. Table 13 summarizes the segmentation setup used in MARS for this analysis. This segmentation setup lead to the detection of the most stained cells. Segmentation and dispatchers transmittance thresholds were based upon cytologists input. The segmentation setup was pixel-based using 20x images captured with a Dage camera on the computer TPO-RDLAB5.

Table 13: Main segmentation setup parameters

| **Size (pixels)** | **Cell**<br>**Nucleus** | **68**<br>**32** |
|---|---|---|
| **Hematoxylin Contribution** | Nucleus<br>Cytoplasm<br>Membrane | 80%<br>100%<br>0% |
| **Hematoxylin Max. Transmittance** | Nucleus<br>Cytoplasm<br>Membrane | 80%<br>100%<br>100% |
| **DAB Contribution** | Nucleus<br>Cytoplasm<br>Membrane | 30%<br>100%<br>0% |
| **DAB Max. Transmittance** | Nucleus<br>Cytoplasm<br>Membrane | 90%<br>100%<br>100% |

In order to assign the selected cells to categories based upon the biomarker staining intensity in the targeted cellular compartment, valid cells resulting from segmentation were dispatched into 3 categories: 1 (in MARS: NegRef), 2 (in MARS: Test) and 3 (in MARS: PosRef). Table 14 provides MARS features and their values used to perform this dispatch, as a function of the cellular localization of the marker.

Table 14: Dispatcher Settings Resulting in the Assignment of Selected Cells into Category 1, 2 or 3

| **Marker Targeted Cell Compartment** | **Dispatch Step** | **If MARS Feature** | **Is** | **Value (Transmittance)** | **Cell(s)** | **Is** |
|---|---|---|---|---|---|---|
| **Nucleus** | **1** | NUCL_ DYE2_OD_ MEAN | > | 0.161151 (69%) | All | (2 or 3) otherwise 1 |
| | **2** | NUCL_ DYE2_OD_ MEAN | > | 0.29243 (51%) | 2 and 3 | 3 otherwise 2 |
| **Cytoplasmic** | **1** | CYTO_ DYE2_OD_ MEAN | > | 0.173925 (67%) | All | (2 or 3) otherwise 1 |
| | **2** | CYTO_ DYE2_OD_ MEAN | > | 0.29243 (51%) | 2 and 3 | 3 otherwise 2 |
| **Membrane** | **1** | CYTO_ DYE2_OD_ MEAN | > | 0.06048 (87%) | All | (2 or 3) otherwise 1 |
| | | MEMB_ DYE2_OD_ MEAN | > | 0.200659 (63%) | | |
| | | MEMD_AREA | > | 150 pix. | | |
| | **2** | CYTO_ DYE2_OD_ MEAN | > | 0.173925 (67%) | 2 and 3 | 3 otherwise 2 |
| | | MEMB_ DYE2_OD_ MEAN | > | 0.29243 (51%) | | |
| | | MEMB_AREA | > | 150 pix. | | |

An evaluation of category 0 (corresponding to the "expected number of non-stained cells") was performed. The approximate number of these cells was computed using the average tumor cell area (1100 pixels as estimated from the MARS feature called CELL_AREA) obtained from the area of cells with a staining intensity of 1, 2 and 3 cells:

$$N_1 = N_{NegRef}$$

$$N_2 = N_{Test}$$

$$N_3 = N_{PosRef}$$

$$N_{Total} = \max\left(N_1 + N_2 + N_3, \frac{FOCUS_AREA}{1100}\right)$$

$$N_0 = \max(0, N_{Total} - N_1 - N_2 - N_3)$$

Using $N_0$, $N_1$, $N_2$ and $N_3$, the percentage of cells staining 0, 1, 2 and 3 cells were computed. Table 15 gives the name of these new features.

Table 15: Percentage Summary Features

| Percentage of cells from categories | Feature Name |
|---|---|
| 0 | CELL_PERCENT_0 |
| 1 | CELL_PERCENT_1 |
| 2 | CELL_PERCENT_2 |
| 3 | CELL_PERCENT_3 |
| 0 and 1 | CELL_PERCENT_01 |
| 2 and 3 | CELL_PERCENT_23 |
| 0, 1 and 2 | CELL_PERCENT_012 |
| 1, 2 and 3 | CELL_PERCENT_123 |

These features were computed as a simple percentage, e.g. for CELL_PERCENT_0:

$$CELL_PERCENT_0 = \frac{N_0}{N_{Total}} \times 100$$

This study was run with MARS features, these new summary features, and the pathologist scores. USER_ TYPE is the name of the MARS feature for pathologist scoring only.

Multiple Biomarker Analysis

In order to obtain an improved sensitivity/specificity couple, data from multiple biomarkers was combined and analyzed. The specificity target for each biomarker was dependent on the number of biomarkers combined. As an example, a combination of 3 biomarkers will reach 80% specificity if each individual marker specificity is at least of 0.81/3 = 93%. Table 16 provides the list of required specificity values based on the number of biomarkers in the combination, from 1 to 9.

Table 16: Minimum specificity required per biomarker when an overall specificity of 0.8 is targeted for a given combination of up to 9 biomarkers

| Marker Number in combination | Specificity Required Per Marker |
|---|---|
| 1 | 0.8 |
| 2 | 0.894427 |
| 3 | 0.923318 |
| 4 | 0.945742 |
| 5 | 0.956352 |
| 6 | 0.963492 |
| 7 | 0.968625 |
| 8 | 0.972492 |
| 9 | 0.975511 |

Data Interpretation

As used herein, the term "marker performance" encompasses the complete experimental performance that relates to the true biological discriminative power of the marker, as well as to the origin and storage of the biological samples, the staining protocols, the scanning process, the imaging and data mining procedures.

Results

1. Per Biomarker

A. Pathologist Scoring

The threshold giving the best sensitivity/specificity couple was computed when considering only the pathologist scores (USER_TYPE in MARS). The most significant results are summarized in Table 17 when a specificity of 0.75 was targeted.

Table 17: Best Sensitivity and Specificity Couple for Biomarkers (Pathologist Scoring)

| Marker | Threshold | Sensitivity | Specificity |
|---|---|---|---|
| **E2F1** | 1.75 | 0.30 | 0.69 |
| **MUC-1** | 0.75 | 0.21 | 0.81 |
| **NDRG1** | 2.5 | 0.28 | 0.74 |
| **$p21^{rac}$** | 0.25 | 0.05 | 0.98 |
| **p53** | 0.5 | 029 | 0.74 |
| **Phospho-p27** | 1.25 | 0.17 | 0.73 |
| **PSMB9** | 0.75 | 0.10 | 0.94 |
| **SLPI** | 25 | 0.18 | 0.83 |
| **src** | 2.5 | 0.10 | 0.87 |

The threshold giving the best sensitivity/specificity couple was also computed when considering only the pathologist evaluation for conventional markers of the breast panel (i.e., ER, PR, Ki67, and Her2/neu (CerbB2)). The most significant results are summarized in Table 18 when a specificity of 0.75 was targeted.

Table 18: Best Sensitivity and Specificity Couple for Conventional Markers (Pathologist Scoring)

| Marker | Threshold | Sensitivity | Specificity |
|---|---|---|---|
| **CerbB2** | 2.5 | 0.17 | 0.85 |
| **ER** | 0.5 | 0.31 | 0.72 |
| **Ki67** | 0.25 | 0.14 | 0.89 |
| **PR** | 2.5 | 0.23 | 0.69 |

For every biomarker and conventional breast cancer marker (i.e., ER, PR, Ki67, and Her2/neu (CerbB2)), the feature and threshold giving the best sensitivity/specificity couple was computed for the pathologist evaluation alone (USER_TYPE). Corresponding receiver operating characteristics (ROC) curves were prepared (data not shown).

B. Single-Feature Analysis

For every biomarker, the feature and threshold giving the best sensitivity/specificity couple was computed when considering every MARS features defined as being meaningful in respect to the analyzed biomarker. Corresponding ROCs were prepared (data not shown). The feature and threshold giving the best result for each biomarker are summarized in Table 19 when a specificity of 0.75 was targeted.

Table 19: Best Sensitivity and Specificity Couple for Each Biomarker Obtained from MARS Features (Single Feature Algorithm)

| Marker | Feature | Threshold | Sens. | Spec. | Rule |
|---|---|---|---|---|---|
| E2F1 | CELL_ PERCENT_01 | 97.2D165 | 0.575758 | 0.716981 | 8 |
| MUC-1 | CELL_ PERCENT_1 | 21.4664 | 0.415385 | 0.685185 | 1 |
| NDRG1 | CELL_ PERCENT_1 | 16.97263 | 0.386667 | 0.713043 | 8 |
| p21ras | CELL_ PERCENT_ 123 | 61.04522 | 0.402778 | 0.724771 | 1 |
| p53 | CELL_ PERCENT_3 | 0.08363 | 0.422535 | 0.702479 | 8 |
| phospho-p27 | CELL_ PERCENT_1 | 0.442341 | 0.528571 | 0.643478 | 8 |
| PSMB9 | CELL_ PERCENT_ 123 | 30.42549 | 0.391892 | 0.711864 | 1 |
| SLPI | CELL_ PERCENT_ 123 | 0.610623 | 0.493333 | 0.694915 | 1 |
| src | CELL_ PERCENT_1 | 36.80664 | 0.409091 | 0.731481 | 1 |
| *A decision rule of 1 means that patients above the threshold are considered as being positive (i.e. TRUE POSITIVE if bad actual clinical outcome), whereas a decision rule of 8 means that patients above the threshold are considered as being negative (i.e. FALSE NEGATIVE if bad actual clinical outcome). | | | | | |

C. Multiple Feature Analysis

Every percent summary feature was combined two-by-two, and thresholds giving the best sensitivity/specificity couple were computed. The most significant results for each biomarker are provided in Table 20 for a target specificity of 0.75.

Table 20: Best Sensitivity and Specificity Couple for Each Biomarker Obtained from MARS features (Multiple Feature Algorithm)

| Marker | Feature 1 | Feature 2 | Threshold 1 | Threshold 2 | Sensitivity | Specificity | Rule |
|---|---|---|---|---|---|---|---|
| **E2F1** | CELL_ PERCENT _2 | CELL_ PERCENT _3 | 2386008 | 1.275759 | 0.575758 | 0.745283 | 7 |

(continued)

| Marker | Feature 1 | Feature 2 | Threshold 1 | Threshold 2 | Sensitivity | Specificity | Rule |
|---|---|---|---|---|---|---|---|
| **MUC-1** | CELL_ PERCFNT_ 1 | CELL_ PERCENT _3 | 21.26747 | 0.311046 | 0.507692 | 0.685185 | 9 |
| **HDRG1** | CELL_ PERCENT _1 | CELL_ PERCENT _23 | 32.96842 | 0.125399 | 0.48 | 0.713043 | 9 |
| **p21ras** | CELL_ FERCENT_ 3 | CELL_ PERCENT _01 | 0.1695 | 99.97016 | 0.458333 | 0.715596 | 6 |
| **p53** | CELL_ PERCENT _1 | CELL_ PERCENT _123 | 1.667598 | 17.22644 | 0.492958 | 0.710744 | 2 |
| **phospho-p27** | CELL_ PERCENT _1 | CELL_ PERCENT _01 | 0.456606 | 100 | 0.5 | 0.695652 | 8 |
| **PSMB9** | CELL_ PERCENT _1 | CELL_ PERCENT _123 | 47.63697 | 20.25946 | 0.486486 | 0.720339 | 4 |
| **SLPI** | CELL_ PERCENT _0 | CELL_ PERCENT _1 | 62.11591 | 0.414484 | 0.573333 | 0.728814 | 1 |
| **src** | CELL_ PERCENT _2 | CELL_ PERCENT _3 | 16.31145 | 0.082021 | 0.545455 | 0.712963 | 4 |
| *Decision rules correspond to quadrant affection in the 2 features space. | | | | | | | |

2. Combinations of Biomarkers

The complete set of possible combinations of 1 to 9 markers was investigated using successively: the pathologist scoring, one MARS feature, and two MARS features per marker. The sensitivity and specificity were computed according to an FDA-like and a sequence-based interpretation method. "FDA-like" means that any marker ON (1) leads to a bad outcome decision. That is, a combination of markers is considered positive if at least one marker is positive. The sequence-based interpretation relies on sensitivity/specificity of each specific ON/OFF combination. The results obtained with pathologist scoring (Table 21) and percentage features evaluation (Table 22) are presented below.

Table 21: Best Sensitivity/Specificity Couples for Biomarker Combinations Using Different Targeted Specificities (75% and 95%) and Different Interpretation Algorithms (Pathologist Scoring)

| | | | **1 Marker** | **2 Markers** | **3 Markers** | **4 Markers** | **5 Markers** | **6 Markers** | **7 Markers** | **8 Markers** | **9 Markers** |
|---|---|---|---|---|---|---|---|---|---|---|---|
| | **Target Spec** | **Markers** | | | | | | | | | |
| FDA | 0.75 | spec<br>sens | 0.74<br>0.29 | 052<br>0.58 | | | | | | | |
| SEQUENCE | | spec<br>sens | | 0.84<br>0.24 | 0.84<br>0.26 | 0.80<br>0.31 | 0.79<br>0.34 | 0.80<br>0.34 | 0.82<br>0.31 | 0.82<br>0.32 | 0.77<br>0.30 |
| FDA | 0.95 | spec<br>sens | 0.90<br>0.13 | 0.86<br>0.23 | 0.88<br>0.25 | 0.84<br>0.30 | | | | | |
| SEQUENCE | | spec<br>sens | | | | | 0.86<br>0.30 | 0.85<br>0.31 | 0.85<br>0.31 | 0.86<br>0.30 | 0.85<br>0.30 |
| *Each patient is characterized by the pathologist score. | | | | | | | | | | | |

Table 22: Best Sensitivity/Specificity Couples for Biomarker Combinations Using Different Targeted Specificities (75% and 95%) and Different Interpretation Algorithms (Percentage Features)

| **All Fovs** | | | | **1 Marker** | **2 Markers** | **3 Markers** | **4 Markers** | **5 Markers** | **6 Markers** | **7 Markers** | **8 Markers** | **9 Markers** |
|---|---|---|---|---|---|---|---|---|---|---|---|---|
| **% Features** | | **Target Spec** | **Markers** | | | | | | | | | |
| **1** | FDA | 0.75 | spec<br>sens | 0.71<br>0.57 | 0.53<br>0.80 | | | | | | | |
| | SEQUENCE | | spec<br>sens | | 0.96<br>0.33 | 0.88<br>0.47 | 0.80<br>0.60 | 0.81<br>0.58 | 0.81<br>0.55 | 0.81<br>0.62 | 0.80<br>0.58 | 0.80<br>0.59 |
| | FDA | 0.95 | spec<br>sens | 0.93<br>0.18 | 0.87<br>0.34 | 0.83<br>0.44 | 0.81<br>0.50 | | | | | |
| | SEQUENCE | | spec<br>sens | | | | | 0.82<br>0.48 | 0.81<br>0.49 | 0.81<br>0.49 | 0.81<br>0.49 | 0.82<br>0.46 |
| **2** | FDA | 0.75 | spec<br>sens | 0.74<br>0.57 | 0.55<br>0.80 | | | | | | | |
| | SEQUENCE | | spec<br>sens | | 0.97<br>0.39 | 0.85<br>0.61 | 0.82<br>0.66 | 0.86<br>0.65 | 0.84<br>0.69 | 0.84<br>0.71 | 0.86<br>073 | 0.83<br>0.71 |
| | FDA | 0.95 | spec<br>sens | 0.94<br>0.30 | 0.90<br>0.50 | 0.83<br>0.63 | 0.82<br>0.72 | 0.81<br>0.76 | | | | |
| | SEQUENCE | | spec<br>sens | | | | | 0.83<br>0.73 | 0.81<br>0.70 | 0.80<br>0.70 | 0.81<br>0.69 | 0.80<br>0.69 |
| *Each patient is characterized by the percentage of 1, 2 and 3 staining cells. | | | | | | | | | | | | |

Specific examples for combinations of four and six biomarkers are provided in Examples 5.

Analysis Without Data from Infiltrating Lobular Cancer (ILC) Patients

The patient population described in Table 8 was further subdivided based on diagnosis. Specifically, data from patients with infiltrating lobular carcinoma (ILC) was excluded, and the above analysis was performed on the resulting data set. Details of the patient population analyzed in this study are provided in Table 23.

Table 23: Patient Population Analyzed (Without ILC Patients)

| **Stage** | **Good** | **Bad** | **All** |
|---|---|---|---|
| T1N0 | 56 | 19 | 75 |
| T1N1 | 6 | 7 | 13 |
| T2N0 | 54 | 33 | 87 |
| T3N0 | 6 | 7 | 13 |
| Totals | **122** | **66** | **188** |

Results

1. Per Biomarker

A. Pathologist Scoring

Table 24: Best Sensitivity and Specificity Couple for Biomarkers without ILC Patient Data (Pathologist Scoring)

| **Marker** | **Threshold** | **Sensitivity** | **Specificity** |
|---|---|---|---|
| **E2F1** | 1.75 | 0.29 | 0.69 |
| **MUC-1** | 0.75 | 0.26 | 0.80 |
| **NDRG-1** | 2.5 | 0.26 | 0.72 |
| **$p21^{ras}$** | 0.25 | 0.03 | 0.98 |
| **p53** | 0.5 | 0.29 | 0.75 |
| **Phospho-p27** | 1.25 | 0.16 | 0.71 |
| **PSMB9** | 0.75 | 0.12 | 0.93 |
| **SLPI** | 2.5 | 0.22 | 0.81 |
| **src** | 2.5 | 0.10 | 0.86 |

Table 25: Best Sensitivity and Specificity Couple for Conventional Markers without ILC Patient Data (Pathologist Scoring)

| **Marker** | **Threshold** | **Sensitivity** | **Specificity** |
|---|---|---|---|
| **CerbB2** | 2.5 | 0.16 | 0.85 |
| **ER** | 0.5 | 0.38 | 0.71 |
| **Ki67** | 0.25 | 0.14 | 0.88 |
| **PR** | 2.5 | 0.23 | 0.69 |

B. Single-Feature Analysis

Table 26: Best Sensitivity and Specificity Couple for Each Biomarker Obtained from MARS Features without ILC Patient Data (Single Feature Algorithm)

| Marker | Feature | Threshold | Sens. | Spec. | Rule |
|---|---|---|---|---|---|
| **E2F1** | CELL_ PERCENT_23 | 3.19079 | 0.58182 | 0.73469 | 1 |
| **MUC-1** | CELL_ PERCENT_23 | 8.437 | 0.38462 | 0.71717 | 8 |
| **NDRG1** | CELL_ PERCENT_ 123 | 26.13234 | 0.39683 | 0.69811 | 8 |
| **p21ras** | CELL_ PERCENT_ 123 | 61.04522 | 0.45763 | 0.72277 | 1 |
| **p53** | CELL_ PERCENT_3 | 0.08289 | 0.41379 | 0.71171 | 8 |
| **phospho-p27** | CELL_ PERCENT_ 123 | 0.44587 | 0.49153 | 0.64486 | 8 |
| **PSMB9** | CELL_ PERCENT_ 123 | 30.42545 | 0.40323 | 0.71560 | 1 |
| **SLPI** | CELL_ PERCENT_ 123 | 0.57594 | 0.53226 | 0.70370 | 1 |
| **src** | CELL_ PERCENT_23 | 13.08501 | 0.43636 | 0.66000 | 8 |
| *A decision rule of 1 means that patients above the threshold are considered as being positive (i.e., TRUE POSITIVE if bad actual clinical outcome) whereas a decision rule of 8 means that patients above the threshold are considered as being negative (i.e., FALSE NEGATIVE if bad actual clinical outcome). | | | | | |

C. Multiple Feature Analysis

Table 27: Best Sensitivity and Specificity Couple for Each Biomarker Obtained from MARS Features without ILC Patient Data (Multiple Feature Algorithm)

| Marker | Feature 1 | Feature 2 | Threshold 1 | Threshold 2 | Sensitivity | Specificity | Rule |
|---|---|---|---|---|---|---|---|
| **E2F1** | CELL_ PERCENT_ 2 | CELL_ PERCEMT_ 3 | 247761 | 1.2758 | 0.61818 | 0.7449 | 7 |
| **MUC-1** | CELL_ PERCENT_ 1 | CELL_ PERCENT_ 2 | 9.6658 | 13.2244 | 0.51923 | 0.68687 | 9 |
| **NDRG1** | CELL_ PERCENT_ 0 | CELL_ PERCENT_ 123 | 28.32391 | 16.95268 | 0.49206 | 0.70755 | 6 |
| **p21ras** | CELL_ PERCENT_ 3 | CELL_ PERCENT_ 01 | 0.1695 | 99.97219 | 049153 | 0.72277 | 6 |
| **p53** | CELL_ PERCENT_ 0 | CELL_ PERCENT_ 3 | 48.61018 | 0.07805 | 0.46552 | 0.71171 | 6 |

(continued)

| Marker | Feature 1 | Feature 2 | Threshold 1 | Threshold 2 | Sensitivity | Specificity | Rule |
|---|---|---|---|---|---|---|---|
| **phospho-p27** | CELL_ PERCENT_ 1 | CELL_ PERCENT_ 01 | 0.50369 | 100 | 049153 | 0.69159 | 8 |
| **PSMB9** | CELL_ PERCENT_ 123 | CELL_ PERCENT_ 01 | 30.42545 | 99.09092 | 0.45161 | 0.7156 | 11 |
| **SLPI** | CELL_ PERCENT_ 0 | CELL_ PERCENT_ 123 | 62.11591 | 040094 | 0.58065 | 0.75 | 1 |
| **src** | CELL_ PERCENT_ 2 | CELL PERCENT 3 | 16.31145 | 0 08202 | 0.52727 | 0.72 | 4 |
| *Decision rules correspond to quadrant affection in the 2 features space. | | | | | | | |

D. Variations Between Analyses: All Patients v. Without ILC Patients

Variations in the sensitivity and specificity values obtained on a per biomarker basis with the analysis of the complete patient population (Table 8) and the population without ILC patients (Table 23) was determined. The results are presented below in Table 28. The sum column ($d^2$) gives the difference of quadratic distance on an ROC curve, i.e., the overall gain in sensitivity and specificity.

Table 28: Variations in Sensitivity and Specificity Obtained with the Complete Patient Population and Without ILC Patients (Per Biomarker)

| | Pathologist Scoring | | | Single-Feature | | | Multi-Features | | |
|---|---|---|---|---|---|---|---|---|---|
| **Marker** | **Sens.** | **Spec.** | **$d^2$** | **Sens.** | **Spec.** | **$d^2$** | **Sens.** | **Spec.** | **$d^2$** |
| E2F1 | ↓ | - | -0.004 | ↑ | ↑ | 0.018 | ↑ | ↓ | 0.026 |
| MUC-1 | ↑ | ↓ | 0.004 | ↓ | ↑ | 0.013 | ↑ | ↑ | 0.008 |
| NDRG1 | ↓ | ↓ | -0.026 | ↑ | ↓ | -0.008 | ↑ | ↓ | 0.002 |
| p21ras | ↓ | - | -0.001 | ↑ | ↓ | 0026 | ↑ | ↑ | 0.024 |
| p53 | - | ↑ | 0009 | ↓ | ↑ | 0.003 | ↓ | ↑ | -0.015 |
| phospho -p27 | ↓ | ↓ | -0 022 | ↓ | ↑ | -0.022 | ↓ | ↓ | -0.008 |
| PSMB9 | ↑ | ↓ | -0.008 | ↑ | ↑ | 0.009 | ↓ | ↓ | -0.023 |
| SLPI | ↑ | ↓ | -0.010 | ↑ | ↑ | 0.030 | ↑ | ↑ | 0.021 |
| src | - | ↓ | -0.010 | ↑ | ↓ | -0.047 | ↑ | ↑ | -0.005 |

2. Combinations of Biomarkers

A. Pathologist Scoring

Table 29: Best Sensitivity/Specificity Couples for Biomarker Combinations without ILC Patient Data Using Different Targeted Specificities (75% and 95%) and Different Interpretation Algorithms (Pathologist Scoring)

| | | | 1 Marker | 2 Markers | 3 Markers | 4 Markers | 5 Markers | 6 Markers | 7 Markers | 8 Markers | 9 Markers |
|---|---|---|---|---|---|---|---|---|---|---|---|
| | **Target Spec** | **Markers** | | | | | | | | | |
| **FDA** | 0.75 | spec | 0.75 | 0.53 | | | | | | | |
| | | sens | 0.29 | 0.57 | | | | | | | |
| **SEQUENCE** | | spec | | 0.86 | 0.83 | 0.84 | 0.84 | 0.90 | 0.85 | 0.83 | 0.77 |
| | | sens | | 0.24 | 0.35 | 0.37 | 0.35 | 0.36 | 0.35 | 0.37 | 0.27 |
| *Each patient is characterized by the pathologist score. | | | | | | | | | | | |

B. Percentage Features Analysis

Table 30: Best Sensitivity/Specificity Couples for Biomarker Combinations without ILC Patients Using Different Targeted Specificities (75% and 95%) and Different Interpretation Algorithms (Percentage Features)

| **All Fovs** | | | | **1 Marker** | **2 Markers** | **3 Markers** | **4 Markers** | **5 Markers** | **6 Markers** | **7 Markers** | **8 Markers** | **9 Markers** |
|---|---|---|---|---|---|---|---|---|---|---|---|---|
| **% Features** | | **Target Spec** | **Markers** | | | | | | | | | |
| **1** | FDA | 0.75 | spec<br>sens | 0.73<br>0.58 | 0.54<br>0.82 | | | | | | | |
| | SEQUENCE | | spec<br>sens | | 0.95<br>0.36 | 0.89<br>0.48 | 0.81<br>0.58 | 0.88<br>0.58 | 0.85<br>0.56 | 0.82<br>0.62 | 0.82<br>0.57 | 0.78<br>0.50 |
| **2** | FDA | 0.75 | spec<br>sens | 0.74<br>0.60 | 0.58<br>0.83 | | | | | | | |
| | SEQUENCE | | Spec<br>sens | | 0.96<br>0.38 | 0.84<br>0.67 | 0.86<br>0.64 | 0.84<br>0.71 | 0.82<br>0.70 | 0.82<br>0.72 | 0.82<br>0.65 | 0.79<br>0.70 |
| *Each patient is characterized by the percentage of 1, 2 and 3 staining cells. | | | | | | | | | | | | |

Table 30 shows an increase in specificity (0.88 compared to 0.81, see Table 28) when considering a 5 biomarker combination excluding ILC patients with a single percent feature. An increase in sensitivity was observed when using 2 features (0.71 vs. 0.65, see Table 28) for a 5 biomarker sequence analysis when excluding ILC patients from the study.

C. Variations Between Analyses: All Patients v. Without ILC Patients

Variations in the sensitivity and specificity values obtained for biomarker combinations with the analysis of the complete patient population and the population without ILC patients was determined. The results are presented below in Table 31. The sum column ($d^2$) gives the difference of quadratic distance on a ROC curve, i.e., the overall gain in sensitivity and specificity. A slight gain in performance for a 5 biomarker sequence analysis using one or two percentage features was observed when ILC patients were excluded from the study.

Table 31: Variations in Sensitivity and Specificity Obtained with Complete Patient Population and Without ILC Patients (Biomarker Combinations)

| **All Fovs** | | | | **1 Marker** | **2 Markers** | **3 Markers** | **4 Makers** | **5 Markers** | **6 Makers** | **7 Markers** | **8 Markers** | **9 Markers** |
|---|---|---|---|---|---|---|---|---|---|---|---|---|
| **% Features** | | **Target Spec** | **Markers** | | | | | | | | | |
| 1 | FDA | 0.75 | d2 | 0.02 | 0.02 | | | | | | | |
| | SEQUENCE | | d2 | | 0.00 | 0.01 | 0.00 | 0.06 | 0.04 | 0.01 | 0.01 | -0.07 |
| 2 | FDA | 0.75 | d2 | 0.02 | 0.03 | | | | | | | |
| | SEQUENCE | | d2 | | -0.01 | 0.03 | 0.02 | 0.02 | -0.01 | -0.01 | -0.08 | -0.04 |

**Example 5: Specific Biomarker Combinations**

The data obtained in the study described above in example 4 were further analyzed, and specific biomarker combinations were considered. The results obtained with a combination of four (SLPI/p21ras/E2F1/src) and six (SLPI/p21ras/PSMB9/ E2F1/src/phospho-p27) biomarkers are presented below.

Four Biomarker Combination: SLPI/p21ras/E2F1/src

Analysis was performed using only one percentage feature for SLPI, p21ras, E2F1, and src with the thresholds and decision rule defined in Table 32. A 60% sensitivity and an 80% specificity was obtained using the rule: if E2F1 was ON (i.e. 1) and not the only biomarker to be ON, then the patient was considered bad outcome; otherwise, considered good outcome. Figure 1 shows the distribution of the percentage feature as a function of bad and good outcome patients for E2F1. Using a threshold of 2.46% sensitivity and specificity values of 0.54 and 0.75, respectively, were obtained.

Table 32: Percentage Summary Features for Four Biomarker Analysis

| Marker | Feature | Threshold | Rule (1 if) |
|---|---|---|---|
| SLPI | CELL_PERCENT_01 | 99.887874 | < |
| p21ras | CELL_PERCENT_0 | 35.642851 | < |
| E2F1 | CELL_PERCENT_2 | 2.463659 | > |
| src | CELL_PERCENT_1 | 37.624326 | > |

A sequence-based interpretation approach was used to analyze the four biomarker combination. The sequence-based decision rule used was: if E2F1 was ON (i.e. 1) and not the only biomarker to be ON, then the patient was considered bad outcome; otherwise, considered good outcome. The sensitivity and specificity values for all of the possible combinations of the four biomarkers are provided in Table 33. The ROC curve obtained using the sequence interpretation approach for the SLPI/p21ras/E2F1/src combination was prepared (data not shown).

Table 33: Sensitivity and Specificity Couples Using Sequence-based Interpretation Approach for SLPI, p21ras, E2F1 and SRC Combination

| **SLPI-p21ras-E2F1-src** | | | | |
|---|---|---|---|---|
| **Sequence** | **CumulBad** | **CumulGood** | **Sensitivity** | **Specificity** |
| S1111 | 4 | 0 | 0.069 | 1 |
| S1011 | 7 | 0 | 0.1207 | 1 |
| S1110 | 12 | 0 | 0.2069 | 1 |
| S0111 | 14 | 8 | 0.2414 | 0.9184 |
| S1010 | 22 | 12 | 0.3793 | 0.8776 |
| S1101 | 26 | 14 | 0.4483 | 0.8571 |
| S0011 | 31 | 16 | 0.5345 | 0.8367 |
| S0110 | 35 | 19 | 0.6034 | 0.8061 |
| S1001 | 37 | 24 | 0.6379 | 0.7551 |
| S1100 | 37 | 26 | 0.6379 | 0.7347 |
| S0010 | 39 | 37 | 0.6724 | 0.6224 |
| S0101 | 41 | 40 | 0.7069 | 0.5918 |
| S1000 | 46 | 56 | 0.7931 | 0.4286 |
| S0001 | 49 | 63 | 0.8448 | 0.3571 |
| S0100 | 52 | 71 | 0.8966 | 0.2755 |
| S0000 | 58 | 98 | **1** | **0** |
| *A sequence S0110 is read as follows: SLPI=OFF / p21ras=ON / E2F1=ON / src=OFF. | | | | |

An interpretation based on E2F1 alone gave a sensitivity and specificity of 54% and 75%, respectively. A specificity and sensitivity of 60% and 80%, respectively, was obtained using the sequence-based algorithm defined above (i.e., if E2F1 was ON (i.e. 1) and not the only biomarker to be ON, then the patient was considered bad outcome;

otherwise, considered good outcome).

Six Biomarker Combination: SLPI/p21ras/E2F1/src/PSMB9/phospho-p27

Analysis was performed using only one percentage feature for a six biomarker combination of SLPI, p21ras, E2F1, src, PSMB9, and phospho-p27 with the thresholds and decision rules defined in Table 34.

Table 34: Percentage Summary Features for Six Biomarker Analysis

| MarkerName | Feature | Threshold | Sensitivity | Specificity | Rule (1 if) |
|---|---|---|---|---|---|
| SLPI | CELL_PERCENT_123 | 0.576 | 53.2% | 70.4% | > |
| p21ras | CELL_PERCENT_123 | 61.045 | 45.8% | 72.3% | > |
| E2F1 | CELL_PERCENT_23 | 3.191 | 58.2% | 73.5% | > |
| PSMB9 | CELL_PERCENT_123 | 30.425 | 40.3% | 71.6% | > |
| src | CELL_PERCENT_23 | 13.085 | 43.6% | 66.0% | < |
| phospho-p27 | CELL_PERCENT_123 | 0.446 | 49.2% | 64.5% | < |

A sequence-based interpretation approach was used to analyze the six biomarker combination. The sequence-based decision rule used was: If E2F1 was ON (i.e. 1) and either SLPI or 21ras, or E2F1 and any 2 biomarkers, or SLPI and any 2 biomarkers, or any 4 biomarkers or more were ON, then the patient was considered bad outcome; otherwise considered good outcome. The sensitivity and specificity values for all of the possible combinations of the six biomarkers of interest are provided in Table 35. The ROC curve obtained using the sequence interpretation approach for the SLPI/p21ras/E2F1/PSM9/src/phospho-p27 combination are shown in Figure 2.

Table 35: Sensitivity and Specificity Couples Using Sequence-based Interpretation Approach for SLPI, p21ras, E2F1, PSMB9, SRC, and Phospho-p27 Combination

| **SLPI-p21ras-E2F1-PSMB9-src-phospho-p27** | | | | |
|---|---|---|---|---|
| Sequence | CumulBad | CumulGood | Sensitivity | Specificity |
| S111111 | 1 | 0 | 0.0208 | 1 |
| S111101 | 2 | 0 | 0.0417 | 1 |
| S111011 | 2 | 0 | 0.0417 | 1 |
| S111110 | 2 | 0 | 0.0417 | 1 |
| S101111 | 3 | 0 | 0.0625 | 1 |
| S111001 | 4 | 0 | 0.0833 | 1 |
| S111100 | 8 | 0 | 0.1667 | 1 |
| S011111 | 8 | 0 | 0.1667 | 1 |
| S111010 | 9 | 0 | 0.1875 | 1 |
| S101101 | 11 | 0 | 0.2292 | 1 |
| S101011 | 12 | 0 | 0.25 | 1 |
| S110111 | 12 | 0 | 0.25 | 1 |
| S101110 | 12 | 0 | 0.25 | 1 |
| S011101 | 12 | 0 | 0.25 | 1 |
| S111000 | 12 | 0 | 0.25 | 1 |
| S011011 | 13 | 1 | 0.2708 | 0.9885 |
| S011110 | 13 | 1 | 0.2708 | 0.9885 |
| S101001 | 13 | 2 | 0.2708 | 0.977 |
| S110101 | 14 | 2 | 0.2917 | 0.977 |
| S101100 | 15 | 3 | 0.3125 | 0.9655 |
| S001111 | 17 | 3 | 0.3542 | 0.9655 |
| S110011 | 19 | 4 | 0.3958 | 0.954 |
| S101010 | 21 | 4 | 0.4375 | 0.954 |
| S110110 | 21 | 4 | 0.4375 | 0.954 |
| S011001 | 21 | 5 | 0.4375 | 0.9425 |

(continued)

| SLPI-p21ras-E2F1-PSMB9-src-phospho-p27 | | | | |
|---|---|---|---|---|
| Sequence | CumulBad | CumulGood | Sensitivity | Specificity |
| S011100 | 22 | 8 | 0.4583 | 0.908 |
| S011010 | 23 | 10 | 0.4792 | 0.8851 |
| S100111 | 23 | 10 | 0.4792 | 0.8851 |
| S001101 | 23 | 11 | 0.4792 | 0.8736 |
| S110001 | 23 | 11 | 0.4792 | 0.8736 |
| S101000 | 25 | 13 | 0.5208 | 0.8506 |
| S001011 | 26 | 14 | 0.5417 | 0.8391 |
| S110100 | 27 | 14 | 0.5625 | 0.8391 |
| S010111 | 27 | 15 | 0.5625 | 0.8276 |
| S001110 | 28 | 16 | 0.5833 | 0.8161 |
| S110010 | 28 | 18 | 0.5833 | 0.8161 |
| S011000 | 31 | 19 | 0.6458 | 0.7816 |
| S100101 | 31 | 19 | 0.6458 | 0.7816 |
| S100011 | 33 | 20 | 0.6875 | 0.7701 |
| S100110 | 34 | 20 | 0.7083 | 0.7701 |
| S001001 | 34 | 21 | 0.7083 | 0.7586 |
| S010101 | 35 | 22 | 0.7292 | 0.7471 |
| S001100 | 35 | 26 | 0.7292 | 0.7011 |
| S110000 | 36 | 28 | 0.75 | 0.6782 |
| S010011 | 37 | 29 | 0.7708 | 0.6667 |
| S001010 | 37 | 30 | 0.7708 | 0.6552 |
| S010110 | 37 | 30 | 0.7708 | 0.6552 |
| S100001 | 37 | 34 | 0.7708 | 0.6092 |
| S100100 | 38 | 38 | 0.7917 | 0.5632 |
| S000111 | 40 | 39 | 0.8333 | 0.5517 |
| S100010 | 40 | 45 | 0.8333 | 0.4828 |
| S010001 | 41 | 46 | 0.8542 | 0.4713 |
| S001000 | 41 | 46 | 0.8542 | 0.4713 |
| S010100 | 41 | 47 | 0.8542 | 0.4598 |
| S010010 | 41 | 48 | 0.8542 | 0.4483 |
| S000101 | 41 | 51 | 0.8542 | 0.4138 |
| S100000 | 42 | 54 | 0.875 | 0.3793 |
| S000011 | 42 | 59 | 0.875 | 0.3218 |
| S000110 | 42 | 61 | 0.875 | 0.2989 |
| S010000 | 42 | 65 | 0.875 | 0.2529 |
| S000001 | 43 | 70 | 0.8958 | 0.1954 |
| S000100 | 43 | 72 | 0.8958 | 0.1724 |
| S000010 | 44 | 77 | 0.9167 | 0.1149 |
| S000000 | 48 | 87 | 1 | 0 |

A specificity and sensitivity of 70% and 77%, respectively, was obtained using the sequence-based algorithm defined above.

## Example 6: Optimization of Reagents and Staining Conditions for Immunohistochemistry

In order to maximize the signal to noise ratio for detection of expression of a particular biomarker using the immunohistochemistry methods disclosed herein, experiments to select the optimal antigen retrieval solution and conditions, antibody concentration and diluent formulation, and detection chemistry parameters were performed. For each set of experiments, biomarker-specific tissue microarrays (TMAs) were constructed by obtaining cylindrical tissue specimens from regular paraffin blocks, assembling them into a single block, and preparing sections containing multiple tissue specimens. TMAs with 2-3 pre-selected known positive and negative tumors for each breast biomarker were used. Slides were prepared and automated immunohistochemistry was performed essentially as described in Example 1. The following control reagents were used during all of the optimization experiments:

- For the negative control, the application of the primary antibody was replaced with a ready to use universal negative reagent, either non-specific mouse or rabbit IgG.
- EF1-$\alpha$ was used as a positive control.
- A positive marker control slide was run following the optimized labeling parameters established during feasibility for each antibody being tested.
- A biomarker specific TMA containing both positive and negative tumors was used in the testing of each breast marker antibody.

1. Optimization of Antigen Retrieval

A. Antigen Retrieval Solutions

Each antigen retrieval solution listed below was tested using each of the biomarker antibodies of interest. The time and temperatures used here were standard accepted values as defined below.

Table 36: Antigen Retrieval Solutions Tested

| Solution | Time | Temperature | Device |
|---|---|---|---|
| Citrate Buffer pH 6.0 (Dako) | 5 minutes | 120°C | Pressure Cooker |
| Tris Buffer pH 9.5 (Biocare) | 5 minutes | 120°C | Pressure Cooker |
| EDTA pH 8.0 (Biocare) | 20 minutes | 95°C | Steamer |
| L.A.B. (Polysciences) | 20 minutes | 20°C and 60°C | None/oven |
| Antigen Retrieval Glyca Solution (Biogenex) | 5 minutes | 120°C | Pressure Cooker |
| Citrate Buffer Solution, pH 4.0 (Zymed) | 20 minutes | 95°C | Steamer |
| di$H_2O$ | 20 minutes | 120°C | Pressure Cooker |
| Dawn (Protor & Gamble) | 3 minutes | 120°C | Pressure Cooker |
| 2% Glacial Acetic Acid | 10 minutes | 95°C | Steamer |

The slides were scored by a pathologist, and the best performing antigen retrieval solution were determined by comparing the labeling specificity and intensity between positive and negative tumors. If the results were essentially negative, alternative antigen retrieval solutions were screened. If results were positive, i.e. labeling more intense than no antigen retrieval, the top (1-3) solutions were identified and used for antigen retrieval time and temperature testing. The activity of the selected antigen retrieval solutions was verified by labeling a representative sample of positive and negative whole tissue sections.

B. Antigen Retrieval Conditions - Time and Temperature

The best-performing antigen retrieval solutions were tested using the following time and temperature criteria:

Table 37: Antigen Retrieval Time and Temperature Conditions Tested

| Temp | 3 minutes | 5 minutes | 10 minutes | 20 minutes | 30 minutes | 4 hours | Overnight |
|---|---|---|---|---|---|---|---|
| 2-8°C | | | | | | * | * |
| 25°C | | | | | | * | * |
| 37°C | | | | | | * | * |
| 60°C | | | | | | * | * |
| 95°C/ST | | | * | * | * | | |

(continued)

| Temp | 3 minutes | 5 minutes | 10 minutes | 20 minutes | 30 minutes | 4 hours | Overnight |
|---|---|---|---|---|---|---|---|
| 120°C/PC | * | * | * | | | | |

The slides were scored by a pathologist, and the best-performing antigen retrieval time and temperature combinations were determined by comparing the labeling specificity and intensity between positive and negative tumors. The activity of the selected antigen retrieval solutions and time and temperature combinations was verified by labeling a representative sample of positive and negative whole tissue sections utilizing the controls listed above.

2. Optimization of Antibody Dilution and Diluent Formulations

A. Antibody Dilution

Each breast cancer biomarker antibody was tested over a range of antibody dilutions. Table 38 provides an example of antibody dilutions tested for the SLPI 5G6.24 antibody. All other breast biomarker antibodies were tested in a similar manner.

Table 38: Antibody Dilutions Tested

| Antibody | IgG concentration | μg/slide (200 μl/slide) | Dilution |
|---|---|---|---|
| SLPI 5G6.24 | 3.5mg/ml (3.5μg/ul) | 3.5 | 1:200 |
| | | 1.75 | 1:400 |
| | | 1.17 | 1:600 |
| | | 0.88 | 1:800 |
| | | 0.7 | 1:1000 |
| | | 0.47 | 1:1500 |

The slides were scored by a pathologist, and the labeling intensities between controls, known positive, and known negative tumors were assessed. The labeling data was analyzed to determine both the upper and lower limits of the antibody dilutions that maintained the desired labeling intensity and the width of the utility range for each antibody. If the initial dilution range tested did not result in the identification of the upper and lower limits, additional antibody dilutions were tested.

B. Antibody Diluent Formulation

Various antibody diluents were tested using each of the breast biomarker antibodies of interest. The table below provides a description of the diluent parameters that were tested.

Table 39: Antibody Diluents Tested

| | | | |
|---|---|---|---|
| PBS pH7.4 | | | |
| PBS pH 7.4 | 0.1% tween®20 | | |
| PBS pH 7.4 | | 1%BSA | |
| PBS pH 7.4 | | | 0.05% $NaN_3$ |
| PBS pH 7.4 | 0.1% tween®20 | 1% BSA | |
| PBS pH 7.4 | 0.1% tween®20 | | 0.05% $NaN_3$ |
| PBS pH 7.4 | | 1% BSA | 0.05% $NaN_3$ |
| PBS pH 7.4 | 0.1% tween®20 | 1% BSA | 0.05% $NaN_3$ |

The slides were scored by a pathologist for labeling intensity. The effectiveness of the diluent formulation was determined by comparing the labeling grade of the biomarkers control slide to the experimental slides. Those that resulted in the most specific and highest signal to noise ratio by comparing the labeling of positive and negative tumors were carried forward. The diluent formulations (approximately one to three) that resulted in the optimal labeling intensity were carried forward into further optimization and stability studies. The activity of the selected diluents was verified by labeling a representative sample of positive and negative whole breast cancer tissue sections.

3. Optimization of Detection Chemistry

Each of the breast biomarker antibodies was tested utilizing the DAKO Envision+ detection kit over the range of times and concentrations listed below.

Table 40: Detection Chemistry Time and Concentration Conditions Tested

| **Concentration** | **Time** | | |
|---|---|---|---|
| | 10 minutes | 30 minutes | 60 minutes |
| 1.0X Concentration | | | |
| 0.75X Concentration | | | |
| 0.5X Concentration | | | |

The slides were scored by a pathologist, and the labeling intensities between controls, known positive, and known negative tumors were assessed. The activity of the selected detection chemistry time and concentration combinations was verified by labeling a representative sample of positive and negative whole breast cancer tissue sections.

Results

A significantly improved signal to noise ratio was observed with optimized staining reagent conditions (data not shown).

Example 7: Real-time PCR Detection of Biomarkers in Clinical Samples

TaqMan® real-time PCR was performed with the ABI Prism 7700 Sequence Detection System (Applied Biosystems, Foster City, CA). The primers and probes were designed with the aid of the Primer Express™ program, version 1.5 (Applied Biosystems, Foster City, CA), for specific amplification of the targeted breast staging markers (e.g., DARPP32 and NDRG-1) in this study. The sequence information on primers and probes is shown below:

DARPP32:

Forward Primer Name: DARPP32_t1-F
Sequence: TACACACCACCTTCGCTGAAAG (SEQ ID NO:33)

Reverse Primer Name: DARPP32_t1-R
Sequence: GGCCTGGTTCTCATTCAAATTG (SEQ ID NO:34)

TaqMan Probe Name: DARPP32_t1-Probe
Sequence: CGCATTGCTGAGTCTCACCTGCAGTC (SEQ ID NO:35)

Forward Primer Name: DARPP32_t2-F
Sequence: CAGCCTTACAGAGACTGGA.AAAGAA (SEQ ID NO:36)

Reverse Primer Name: DARPP32_t2-R
Sequence: GAGGCTCAGGGACCCAAAG (SEQ ID NO:37)

TaqMan Probe Name: DARPP32_t2-Probe
Sequence: CCAAACCAAGGCCCCCAGAGAGGT (SEQ ID NO:38)

NDRG-1:

Forward Primer Name: NDRG-1-F
Sequence: CCTACCGCCAGCACATTGT (SEQ ID NO:39)

Reverse Primer Name: NDRG-1-R
Sequence: GCTGTTGTAGGCATTGATGAACA (SEQ ID NO:40)

TaqMan Probe Name: NDRG-1-Probe
Sequence: AATGACATGAACCCCGGCAACCTG (SEQ ID NO:41)

The probes were labeled with a fluorescent dye FAM (6-carboxyfluorescein) on the 5' base, and a quenching dye TAMRA (6-carboxytetramethylrhodamine) on the 3' base. The sizes of the amplicons were around 100 bp.18S ribosomal RNA was applied as endogenous control. 18S rRNA probe was labeled with a fluorescent dye VIC. Pre-Developed 18S rRNA primer/probe mixture was purchased from Applied Biosystems (P/N: 4310893E). 20 frozen breast tissues (i.e., 6 tumors with bad outcome, 12 tumors with good outcome, and 2 normal tissues) were analyzed in this study. In this study, good outcome was defined as remaining cancer-free for at least 5 years; bad outcome was defined as suffering disease relapse, recurrence, or death within 5 years. 5 μg of total RNA extracted from the frozen breast tissues was quantitatively converted into the single stranded cDNA form with random hexamers (not with oligo-dT) by using the High-Capacity cDNA Archive Kit (Applied Biosystems, P/N: 4322171). The following reaction reagents were prepared:

| 20X Master Mix of Primers/Probe (in 200 μl) | |
|---|---|
| 180 μM Forward primer | 20 μl |
| 180 μM Reverse primer | 20 μl |
| 100 μM TaqMan probe | 10 μl |
| $H_2O$ | 150μl |
| Final Reaction Mix (25 μl / well) | |
| 20X master mix of primers/probe | 1.25 μl |
| 2X TaqMan Universal PCR master mix (P/N: 4304437) | 12.5 μl |
| cDNA template | 5.0 μl |
| $H_2O$ | 6.25 μl |

20X TaqMan Universal PCR Master Mix was purchased from Applied Biosystems (P/N: 4304437). The final primer and probe concentrations, in a total volume of 25 μl, were 0.9 μM and 0.25 μM, respectively. 10ng of total RNA was applied to each well of the reaction. The amplification conditions were 2 min at 50°C, 10 min at 95°C, and a two-step cycle of 95°C for 15 seconds and 60°C for 60 seconds for a total of 40 cycles. At least three no-template control reaction mixtures were included in each run. All experiments were performed in triplicate.

At the end of each reaction, the recorded fluorescence intensity was used for the following calculations: $Rn^+$ is the Rn value of a reaction containing all components, $Rn^-$ is the Rn value of an unreacted sample (baseline value or the value detected in NTC). ΔRn is the difference between $Rn^+$ and $Rn^-$. It is an indicator of the magnitude of the signal generated by the PCR. Expression level of a target gene was computed by comparative $C_T$ method. This method uses no known amount of standard but compares the relative amount of the target sequence to the reference values chosen (18S rRNA was selected as a reference in this study). See the Applied Biosystems' TaqMan Human Endogenous Control Plate Protocol that contains detailed instructions regarding MS Excel based data analysis.

Results

The results obtained with each biomarker and with the specific primers are listed below in tabular form. Results obtained with normal breast tissue samples are designated N; those obtained with breast cancer samples are labeled T.

Table 41: DARPP32 TaqMan® Results

| Samples | t1 | t2 | t1t2 |
|---|---|---|---|
| **2T** | **0.18** | **0.5** | **0.54** |
| **7T** | **5.7** | **23.5** | **62.5** |
| **12T** | **73.5** | **16.9** | **84.2** |
| **13T** | **1.2** | **1.1** | **2.2** |
| **21T** | **5.8** | **6.1** | **16.1** |
| **24T** | **4.2** | **2.9** | **7.9** |
| 26T | 0.6 | 0.3 | 1.9 |
| 1T | 0.02 | 0.2 | 0.1 |
| 3T | 0.4 | 0.04 | 0.8 |
| 4T | 2.5 | 1 | 4.8 |
| 5T | 1.2 | 0.5 | 3.7 |
| 6T | 0.9 | 0.6 | 2.6 |
| 9T | 0.3 | 0.6 | 0.5 |
| 10T | 0.1 | 0.2 | 0.3 |
| 11T | 0.7 | 0.1 | 0.9 |
| 19T | 0.8 | 0.3 | 1.6 |
| 22T | 0.6 | 0.6 | 1.6 |
| 23T | 0.5 | 0.4 | 1.2 |
| 25T | 0.2 | 0.1 | 0.3 |
| 1N | 1.1 | 1.3 | 2 |
| 8N | 0.7 | 0.3 | 1.3 |
| Bad Mean: | 15.10 | 8.50 | 28.91 |
| Good Mean | 0.69 | 0.39 | 1.53 |
| t-test P= | 0.046 | 0.004 | 0.007 |

DARPP32 has two transcripts: t1 and t2. TaqMan® data showed that both t1 and t2 were overexpressed in the breast tumors with bad outcomes (in bold) as compared with those with good outcomes.

Table 42: NDRG-1 TaqMan® Results

| Samples | NDRG-1 |
|---|---|
| **2T** | **2.8** |
| **7T** | **12.8** |
| **12T** | **5.5** |
| **13T** | **6.4** |
| **21T** | **2.4** |
| **24T** | **6.7** |
| 26T | 2.3 |
| 1T | 4.1 |
| 3T | 4.2 |

(continued)

| Samples | NDRG-1 |
|---|---|
| 4T | 2.8 |
| 5T | 3.2 |
| 6T | 1.3 |
| 9T | 3.1 |
| 10T | 3.7 |
| 11T | 1.6 |
| 19T | 3.4 |
| 22T | 5.5 |
| 23T | 1.6 |
| 25T | 3.1 |
| 1N | 0.9 |
| 8N | 0.5 |
| Bad Mean: | 6.10 |
| Good Mean: | 3.13 |
| t-test P= | 0.021 |

NDRG-1 has one transcript. TaqMan data showed that NDRG-1 was overexpressed in the breast tumors with bad outcomes (in bold) as compared with those with good outcomes.

**Example 8: Detection of Biomarker Overexpression in a Chemo-Naïve Patient Population with 10-Year Clinical Follow-up (Five Biomarker Panel)**

Breast tumor tissue samples collected at or near the time of initial diagnosis from 255 early-stage breast cancer patients were analyzed for biomarker overexpression in this study. Ten-year clinical follow-up data was available for all patients in the study. None of the patients received cytotoxic chemotherapy at any time during their treatment for breast cancer. The clinical demographics, distribution, and standard histopathological parameters (e.g., ER/PR hormone receptor status, histological grade, etc.) for the patient population are summarized below in Table 43.

Table 43: Clinical Characteristics of Chemo-Naive Patient Population

| Characteristics | Overall |
|---|---|
| Age at diagnosis (years) | n = 255 |
| Mean (std) | 64.0 (10.6) |
| Range | 30 - 85 |
| Age group distribution | |
| <40 | 6 (2.4%) |
| 40-<50 | 23 (9.0%) |
| 50-<60 | 48 (18.8%) |
| 60-<70 | 87(34.1%) |
| >=70 | 91 (35.7%) |
| Tumor size (cm) | n = 255 |
| Mean (std) | 2.1 (1.19) |

(continued)

| | |
|---|---|
| Age group distribution | |
| Range | 0.3-11.0 |
| Tumor size group | |
| <1.0 | 16 (6.3%) |
| 1.0-<2.0 | 104(40.8%) |
| 2.0-<4.0 | 122 (47.8%) |
| >=4.0 | 13(5.1%) |
| Lymph node status | n = 255 |
| Negative | 232 (91.0%) |
| Positive | 23 (9.0%) |
| Histological Grade | n = 244 |
| 1 | 38 (15.6%) |
| 2 | 135 (55.3% |
| 3 | 71 (29.1%) |
| ER Status | n = 249 |
| Negative | 64 (25.7%) |
| Positive | 185 (74.3%) |
| Her2/neu status | n = 249 |
| Negative | 176 (70.7%) |
| Positive | 73 (29.3%) |

Detection of expression of a five biomarker panel comprising SLPI, src, PSMB9, p21ras, and E2F1 was performed essentially as described above. That is, breast tumor samples were prepared and stained for biomarker expression using the Dako Autostainer, as described above in Example 1. Biomarker overexpression was determined using the imaging analysis described in Example 4.

The prognostic performance of the 5 biomarker panel was assessed utilizing a Cox Proportional Hazards Model analysis. See, for example Spruance *et al., supra.* The prognostic value of each biomarker and/or histological characteristic to identify the patients who suffered disease recurrence or death within ten years over the patients disease-free after ten years was calculated. In the analysis without the biomarker panel, age and tumor size were found to be independent prognostic factors with a p value < 0.05. When the biomarkers were added to this analysis, they exhibited the highest statistically significant independent prognostic utility with a p value of <0.0001. The results of the Cox Proportional Hazard analysis are summarized below in Table 44.

Table 44: Results of Cox Proportional Hazard Analysis with Chemo-Naïve Patient Population (SLPI, src. PSMB9. p21ras, and E2F1 Biomarker Panel)

| Variable | P Value | Hazard Ratio | (95% CI) |
|---|---|---|---|
| Analysis (without Biomarkers) | | | |
| Age at Diagnosis | 0.0002 | 1.05 | (1.02, 1.08) |
| Tumor Size | 0.0066 | 1.28 | (1.07,1.53) |
| ER | 0.2506 | 1.40 | (0.79,2.50) |
| Total Grade | 0.0674 | 1.39 | (0.98, 1.99) |

(continued)

| | | | |
|---|---|---|---|
| Analysis (with Biomarkers)* | | | |
| Age at Diagnosis | 0.0004 | 1.05 | (1.02, 1.08) |
| Tumor Size | 0.0318 | 1.21 | (1.02, 1.44) |
| ER | 0.0134 | 2.20 | (1.18, 4.12) |
| Total Grade | 0.0845 | 1.37 | (0.96, 1.96) |
| TPO Marker | <0.0001 | 1.92 | (1.47,2.50) |
| Age at diagnosis was continuous variable and the biomarker was ordinary variable with 0 or 1, 2, 3, 4 (0=none positive marker, 1 =one positive marker, or 2, 3, 4 positive marker). | | | |

The prognostic performance of the SLPI, src, PSMB9, p21ras, and E2F1 biomarker panel is graphically presented in the Kaplan-Meier plot of Figure 3. The x-axis represents years from initial diagnosis, and the y axis is the percentage of disease-free survival. The corresponding graph for the general breast cancer population independent of biomarker analysis is presented in Figure 4. These plot demonstrate the ability of this biomarker panel to risk stratify this early stage breast cancer patient population for disease recurrence and/or death due to primary disease. The risk of reccurance and/or death due to primary disease increases as the number of biomarkers that are overexpressed in the patient samples increases. The disease-free survival rates of the patient subgroups identified by the number of overexpressed biomarkers are statistically significant from each other with a p value of <0.001, as determined by log-rank test for comparison of 0 positive, 1 positive, 2 positive, 3 or more positive biomarker groups. A biomarker that is classified as overexpressed by the imaging analyses described herein is deemed "positive."

Because one of the most important clinical features of a breast cancer patient's diagnosis relates to estrogen receptor (ER) status, the prognostic performance of the SLPI, src, PSMB9, p21ras, and E2F1 biomarker panel was further assessed using the Cox Proportional Hazard analysis in the ER-positive and -negative patient subgroups. Clinical management and prognosis of these two subgroups is different because ER-positive patients are candidates for tamoxifen therapy whereas ER negative patients are not. The results of the analysis are summarized below in Table 45. The data indicate that the five biomarkers of interest have prognostic utility in both the ER positive and negative breast cancer patient subgroups. Therefore, while the biomakers SLPI, src, PSMB9, p21ras, and E2F1 are indicative of prognosis independent of the patient's ER status, these biomarkers also correlate with ER status.

Table 45: Results of Cox Proportional Hazard Analysis with Chemo-Naïve Patient Population (SLPI, src, PSMB9, p21ras, and E2F1 Biomarker Panel in ER Positive and Negative Patient Subgroups)

| ER Positive | Variable | P Value | Hazard Ratio | (95% CI) |
|---|---|---|---|---|
| | Analysis Without Biomarker | | | |
| | Age at Diagnosis | 0.0012 | 1.05 | (1.02, 1.09) |
| | Tumor Size | 0.0237 | 1.25 | (1.03, 1.51) |
| | HER2 | 0.5732 | 1.18 | (0.66,2.13) |
| | Total Grade | 0.0566 | 1.47 | (0.99, 2.20) |
| | Analysis with Biomarker* | | | |
| | Age at Diagnosis | 0.0009 | 1.06 | (1.03, 1.10) |
| | Tumor Size | 0.0753 | 1.19 | (0.98, 1.43) |
| | HER2 | 0.8523 | 1.06 | (0.58, 1.93) |
| | Total Grade | 0.0440 | 1.50 | (1.01, 2.23) |
| | TPO Marker | <0.0001 | 1.98 | (1.46, 2.69) |
| ER Negative | Analysis without Biomarker | | | |

(continued)

| | | | | |
|---|---|---|---|---|
| | Age at Diagnosis | 0.0771 | 1.04 | (1.00, 1.09) |
| | Tumor Size | 0.1527 | 1.51 | (0.86,2.64) |
| | HER2 | 0.2562 | 0.55 | (0.19, 1.55) |
| | Total Grade | 0.9883 | 1.01 | (0.45, 2.24) |
| | Analysis with Biomarker* | | | |
| | Age at Diagnosis | 0.3467 | 1.03 | (0.97, 1.08) |
| | Tumor Size | 0.1854 | 1.44 | (0.84, 2.48) |
| | HER2 | 0.6577 | 0.78 | (0.27, 2.30) |
| | Total Grade | 0.7327 | 0.86 | (0.38, 1.99) |
| | TPO Marker | 0.0089 | 1.91 | (1.18,3.09) |
| Age at diagnosis was continuous variable and the TPO marker was ordinary variable with 0 or 1, 2, 3, 4 (0=none positive marker, 1=one positive marker, or 2, 3, 4 positive marker). | | | | |

**Example 9: Detection of Biomarker Overexpression in a Chemo-Naïve Patient Population with 10-Year Clinical Follow-up (Six Biomarker Panel)**

Breast tumor tissue samples from 100 patients (50 good outcome; 50 bad outcome patients) from the chemo-naïve patient population described in Example 8 were analyzed for biomarker overexpression of six biomarkers of interest (SLPI, src, PSMB9, $p21^{ras}$, E2F1, and MUC-1). Detection of expression of the six biomarker panel was performed by automated immunohistochemistry essentially as described above except that an alternate staining platform, the Ventana BenchMark XT, was used in place of the Dako Autostainer. A standard manual for operating the Ventana BenchMark XT is readily available from the manufacturer. Additional modifications to the immunohistochemistry parameters used with the Ventana BenchMark XT staining platform are summarized in Table 46 below. Biomarker overexpression was determined as before using the imaging analysis described in Example 4.

Table 46: Immunohistochemistry Parameters for Biomarker Staining with the Ventana BenchMark XT Staining Platform

| Biomarker | Antibody Concentration (ug/ml) | Antigen Retrieval Solution | Antigen Retrieval Time | Antibody Incubation Temp | Antibody Incubation Time | Block and Amplification |
|---|---|---|---|---|---|---|
| SLPI | 3.6 | CC1 | Extende d | RT | 1hr | None |
| E2F1 | 2.0 | CC1 | Extende d | 37°C | 16 min | Pro & Biotin Amp |
| SRC | 40 | CC2 | Standard | 37°C | 1 hr | None |
| $p21^{ras}$ | 13.7 | CC1 | Short | 37°C | 12 min | None |
| PSMB9 | 6.5 | CC2 | Standard | RT | 1 hr | None |
| MUC1 | 5.0 | CC1 | Extende d | 37°C | 1hr | None |
| *CC1 and CC2 refer to cell conditioning reagents commercially available from Ventana. With respect to antigen retrieval times: short = 30 min; standard = 60 min; and extended = 90 min.* | | | | | | |

The prognostic performance of the 6 biomarker panel was assessed utilizing a Cox Proportional Hazards Model analysis, as above. The prognostic value of each biomarker and/or histological characteristic to identify the patients who suffered disease recurrence or death within ten years over the patients disease-free after 10 years was calculated. The biomarkers of interest (SLPI, src, PSMB9, $p21^{ras}$, E2F1, and MUC-1) exhibited statistically significant prognostic utility with a p value of 0.0220. The results of the Cox Proportional Hazard analysis are summarized below in Table 47.

Table 47: Results of Cox Proportional Hazard Analysis with Chemo-Naïve Patient Population (SLPI, src, PSMB9, p21ras. E2F1 , and MUC-1 Biomarker Panel)

| *Variable* | P Value | Hazard Ratio | 95% Hazard Ratio confidence Limits | |
|---|---|---|---|---|
| Age at Diagnosis | 0.0523 | 1.032 | 1.000 | 1.066 |
| Tumor Size | 0.0180 | 1.319 | 1.049 | 1.658 |
| Her2 | 0.2619 | 0.640 | 0.293 | 1.396 |
| ER | 0.4539 | 1.359 | 0.609 | 3.035 |
| Total Grade | 0.7693 | 1.075 | 0.661 | 1.749 |
| Biomarkers (SLPI, src, PSMB9, p21ras, and E2F1 Biomarker Panel) | 0.0220 | 1.335 | 1.042 | 1.709 |

The prognostic performance of the SLPI, src, PSMB9, p21ras, E2F1, and MUC-1 biomarker panel is graphically presented in the Kaplan-Meier plot of Figure 5. The x-axis represents years from initial diagnosis, and the y axis is the percentage of disease-free survival. This plot demonstrates the ability of this biomarker panel to risk stratify this early stage breast cancer patient population for disease recurrence and/or death due to primary disease. The risk of reccurance and/or death due to primary disease increases as the number of biomarkers that are overexpressed in the patient samples increases. The disease-free survival rates of the patient subgroups identified by the number of overexpressed biomarkers are statistically significant from each other with a p value of <0.0065, as determined by log-rank test for comparison of 0 positive, 1 positive, 2 positive, 3 or more positive biomarker groups. As described above, a biomarker that is classified as overexpressed by the imaging analyses described herein is deemed "positive."

Table 48: Biomarker Nucleotide and Amino Acid Sequence Information

| | **Nucleotide Sequence** | | **Amino Acid Sequence** | |
|---|---|---|---|---|
| **Biomarker Name** | **Accession No.** | **Sequence Identiffer** | **Accession No.** | **Sequence Identifier** |
| SLPI | NM_003064 | SEQ ID NO:1 | NP 003055 | SEQ ID NO:2 |
| DARPP-32 | NM_032192 | SEQ ID NO:3 | NP 115568 | SEQ ID NO:4 |
| MGC14832 | NM_032339 | SEQ ID NO:5 | NP 115715 | SEQ ID NO:6 |
| NDRG-1 | NM_006096 | SEQ ID NO:7 | NP 006087 | SEQ ID NO:8 |
| PSMB9 | NM_002800 | SEQ ID NO:9 | NP 002791 | SEQ ID NO:10 |
| p27 | NM_004064 | SEQ ID NO:11 | NP 004055 | SEQ ID NO:12 |
| E2F1 | NM_005225 | SEQ ID NO:13 | NP 005216 | SEQ ID NO:14 |
| MCM6 | NM_005915 | SEQ ID NO:15 | NP 005906 | SEQ ID NO:16 |
| MCM2 | D83987 | SEQ ID NO:17 | BAA12177 | SEQ ID NO:18 |
| MUC-1 | NM_182741 | SEQ ID NO:19 | NP 877418 | SEQ ID NO:20 |
| p21ras | NM_005343 | SEQ ID NO:21 | NP 005334 | SEQ ID NO:22 |
| Src | NM_005417 | SEQ ID NO:23 | NP 005408 | SEQ ID NO:24 |
| TGF-beta3 | BC018503 | SEQ ID NO:25 | AAH18503 | SEQ ID NO:26 |
| PDGFRalpha | M21574 | SEQ ID NO:27 | AAA96715 | SEQ ID NO:28 |
| Myc | V00568 | SEQ ID NO:29 | CAA23831 | SEQ ID NO:30 |
| SERHL | NM_014509 | SEQ ID NO:31 | NP 055324 | SEQ ID NO:32 |

**Claims**

**1.** A method for evaluating the prognosis of a breast cancer patient, said method comprising:

a) contacting a sample from a patient with at least five antibodies that each specifically bind to a distinct biomarker protein, wherein a first antibody specifically binds to SLPI, wherein a second antibody specifically binds to src, wherein a third antibody specifically binds to PSMB9, wherein a fourth antibody specifically binds to p21ras, and wherein a fifth antibody specifically binds to E2F1;
b) detecting binding of said antibodies to said biomarker proteins;
c) determining if said biomarker proteins are overexpressed in said sample, wherein overexpression of at least two biomarker proteins is indicative of a poor prognosis; and,
d) thereby evaluating the prognosis of said breast cancer patient.

**2.** The method of claim 1, wherein the method further comprises determining if one or more additional biomarker proteins is overexpressed in the sample, wherein the one or more additional biomarker proteins is selected from the group consisting of MUC-1, DARPP-32, phospho-p27, MGC 14832, myc, TGFβ-3, SERHL, PDGFRα, NDRG-1, MCM2, and MCM6.

**3.** The method of claim 1 or 2, wherein the patient is lymph node-negative.

**4.** The method of any of the preceding claims, wherein said method for evaluating the prognosis of a breast cancer patient further comprises assessment of clinical information.

5. The method of claim 4, wherein said clinical information comprises tumor size, tumor grade, lymph node status, and family history.

6. The method of any of the preceding claims, wherein said method is used to develop a treatment strategy for said breast cancer patient.

7. The method of any of the preceding claims, wherein said method for evaluating the prognosis of a breast cancer patient is coupled with analysis of Her2/neu expression levels.

8. The method of any of the preceding claims, wherein said method for evaluating the prognosis of a breast cancer patient is coupled with analysis of estrogen receptor or progesterone receptor status of the patient.

9. The method of any of the preceding claims, wherein said method for evaluating the prognosis of a breast cancer patient is independent of estrogen receptor status of the patient.

10. The method of any of the preceding claims, wherein said method is used to evaluate the prognosis of an estrogen receptor-positive or an estrogen receptor-negative breast cancer patient.

11. The method of any of the preceding claims, wherein said breast cancer patient has early-stage breast cancer.

12. A kit comprising a first antibody that specifically binds to SLPI, a second antibody that specifically binds to src, a third antibody that specifically binds to PSMB9, a fourth antibody that specifically binds to p21ras, and a fifth antibody that specifically binds to E2F1.

13. The kit of claim 12 further comprising at least one antibody that specifically binds to MUC-1, DARPP-32, phospho-p27, MGC 14832, myc, TGF$\beta$-3, SERHL, PDGFR$\alpha$, NDRG-1, MCM2, or MCM6.

14. The kit of claim 12 or 13, wherein said kit further comprises chemicals for the detection of antibody binding.

15. The kit of any one of claims 12-14, wherein said kit further comprises a positive control sample.

## Patentansprüche

1. Verfahren zur Bewertung der Prognose eines Brustkrebs-Patienten, welches Verfahren umfasst:

   a) Kontaktieren einer Probe von einem Patienten mit wenigstens fünf Antikörpern, die jeweils spezifisch an ein unterschiedliches Biomarker-Protein binden, wobei ein erster Antikörper spezifisch an SLPI bindet, wobei ein zweiter Antikörper spezifisch an src bindet, wobei ein dritter Antikörper spezifisch an PSMB9 bindet, wobei ein vierter Antikörper spezifisch an p21 ras bindet und wobei ein fünfter Antikörper spezifisch an E2F1 bindet;
   b) Nachweisen der Bindung dieser Antikörper an diese Biomarker-Proteine;
   c) Bestimmen, ob diese Biomarker-Proteine in dieser Probe überexprimiert sind, wobei die Überexpression wenigstens zweier Biomarker-Proteine auf eine schlechte Prognose schließen lässt; und
   d) **dadurch** Bewerten der Prognose dieses Brustkrebs-Patienten.

2. Verfahren nach Anspruch 1, wobei das Verfahren außerdem das Bestimmen umfasst, ob ein oder mehrere weitere Biomarker-Proteine in der Probe überexprimiert sind, wobei die ein oder mehreren Biomarker-Proteine ausgewählt sind aus der Gruppe, bestehend aus MUC-1, DARPP-32, Phospho-p27, MGC 14832, myc, TGF$\beta$-3, SERHL, PDG-FR$\alpha$, NDRG-1, MCM2 und MCM6.

3. Verfahren nach Anspruch 1 oder 2, wobei der Patient Lymphknoten-negativ ist.

4. Verfahren nach jedem der vorangehenden Ansprüche, wobei das Verfahren zur Bewertung der Prognose eines Brustkrebs-Patienten außerdem die Erhebung der klinischen Informationen umfasst.

5. Verfahren nach Anspruch 4, wobei die klinischen Informationen Tumorgröße, Tumorgrad, Lymphknoten-Status und Familiengeschichte (Anamnese) umfassen.

**6.** Verfahren nach jedem der vorangehenden Ansprüche, wobei das Verfahren zur Entwicklung einer Behandlungsstrategie für diesen Brustkrebs-Patienten eingesetzt wird.

**7.** Verfahren nach jedem der vorangehenden Ansprüche, wobei das Verfahren zur Bewertung der Prognose eines Brustkrebs-Patienten mit der Analyse der Her2/neu-Expressionshöhen gekoppelt ist.

**8.** Verfahren nach jedem der vorangehenden Ansprüche, wobei das Verfahren zur Bewertung der Prognose eines Brustkrebs-Patienten mit der Analyse des Östrogenrezeptor- oder Progesteronrezeptor-Status des Patienten gekoppelt ist.

**9.** Verfahren nach jedem der vorangehenden Ansprüche, wobei das Verfahren zur Bewertung der Prognose eines Brustkrebs-Patienten unabhängig vom Östrogenrezeptor-Status des Patienten ist.

**10.** Verfahren nach jedem der vorangehenden Ansprüche, wobei das Verfahren zur Bewertung der Prognose eines Östrogenrezeptor-positiven oder eines Östrogenrezeptor-negativen Brustkrebs-Patienten eingesetzt wird.

**11.** Verfahren nach jedem der vorangehenden Ansprüche, wobei der Brustkrebs-Patient einen Brustkrebs im Frühstadium aufweist.

**12.** Kit, umfassend einen ersten Antikörper, der spezifisch an SLPI bindet, einen zweiten Antikörper, der spezifisch an src bindet, einen dritten Antikörper, der spezifisch an PSMB9 bindet, einen vierten Antikörper, der spezifisch an p21 ras bindet, und einen fünften Antikörper, der spezifisch an E2F1 bindet.

**13.** Kit nach Anspruch 12, außerdem umfassend wenigstens einen Antikörper, der spezifisch an MUC-1, DARPP-32, Phospho-p27, MGC 14832, myc, TGFβ-3, SERHL, PDGFRα, NDRG-1, MCM2 oder MCM6 bindet.

**14.** Kit nach Anspruch 12 oder 13, wobei dieser Kit außerdem Chemikalien für den Nachweis der Antikörper-Bindung umfasst.

**15.** Kit nach jedem der Ansprüche 12-14, wobei dieser Kit außerdem eine positive Kontrollprobe umfasst.

## Revendications

**1.** Procédé pour évaluer le pronostic d'une patiente ayant un cancer du sein, ledit procédé consistant à :

a) mettre en contact un échantillon provenant de la patiente avec au moins cinq anticorps, chacun se liant spécifiquement à une protéine de biomarquage distincte, un premier anticorps se liant spécifiquement à SLPI, un deuxième anticorps se liant spécifiquement à src, un troisième anticorps se liant spécifiquement à PSMB9, un quatrième anticorps se liant spécifiquement à p21ras, et un cinquième anticorps se liant spécifiquement à E2F1 ;
b) détecter la liaison desdits anticorps auxdites protéines de biomarquage ;
c) déterminer si lesdites protéines de biomarquage sont surexprimées dans ledit échantillon, une surexpression d'au moins deux protéines de biomarquage étant indicative d'un pronostic pessimiste ; et
d) évaluer en conséquence le pronostic de ladite patiente ayant un cancer du sein.

**2.** Procédé selon la revendication 1, lequel procédé comprend en outre l'étape consistant à déterminer si une ou plusieurs protéines de biomarquage additionnelles sont surexprimées dans l'échantillon, la ou les protéines de biomarquage additionnelles étant choisies dans le groupe constitué par MUC-1, DARPP-32, phospho-p27, MGC 14832, myc, TGFβ-3, SERHL, PDGFRα, NDRG-1, MCM2, et MCM6.

**3.** Procédé selon la revendication 1 ou 2, dans lequel la patiente est négative au niveau des ganglions lymphatiques.

**4.** Procédé selon l'une quelconque des revendications précédentes, ledit procédé pour évaluer le pronostic d'une patiente ayant un cancer du sein comprenant en outre la détermination d'informations cliniques.

**5.** Procédé selon la revendication 4, dans lequel lesdites informations cliniques comprennent la taille des tumeurs, le grade des tumeurs, l'état des ganglions lymphatiques, et l'historique familial.

**6.** Procédé selon l'une quelconque des revendications précédentes, ledit procédé étant utilisé pour développer une stratégie de traitement pour ladite patiente ayant un cancer du sein.

**7.** Procédé selon l'une quelconque des revendications précédentes, ledit procédé pour évaluer le pronostic d'une patiente ayant un cancer du sein étant couplé à une analyse des niveaux d'expression de Her2/neu.

**8.** Procédé selon l'une quelconque des revendications précédentes, ledit procédé pour évaluer le pronostic d'une patiente ayant un cancer du sein étant couplé à une analyse de l'état des récepteurs d'oestrogène ou des récepteurs de progestérone de la patiente.

**9.** Procédé selon l'une quelconque des revendications précédentes, ledit procédé pour évaluer le pronostic d'une patiente ayant un cancer du sein étant indépendant de l'état des récepteurs d'oestrogène de la patiente.

**10.** Procédé selon l'une quelconque des revendications précédentes, ledit procédé étant utilisé pour évaluer le pronostic d'une patiente ayant un cancer du sein positif aux récepteurs d'oestrogène ou négatif aux récepteurs d'oestrogène.

**11.** Procédé selon l'une quelconque des revendications précédentes, dans lequel ladite patiente ayant un cancer du sein a un cancer du sein à un stade précoce.

**12.** Kit comprenant un premier anticorps qui se lie spécifiquement à SLPI, un deuxième anticorps qui se lie spécifiquement à src, un troisième anticorps qui se lie spécifiquement à PSMB9, un quatrième anticorps qui se lie spécifiquement à p21ras, et un cinquième anticorps qui se lie spécifiquement à E2F1.

**13.** Kit selon la revendication 12, comprenant en outre au moins un anticorps qui se lie spécifiquement à MUC-1, DARPP-32, phospho-p27, MGC 14832, myc, TGF$\beta$-3, SERHL, PDGFR$\alpha$, NDRG-1, MCM2, ou MCM6.

**14.** Kit selon la revendication 12 ou 13, ledit kit comprenant en outre des produits chimiques pour la détection d'une liaison à un anticorps.

**15.** Kit selon l'une quelconque des revendications 12 à 14, ledit kit comprenant en outre un échantillon témoin positif.

FIG. 1

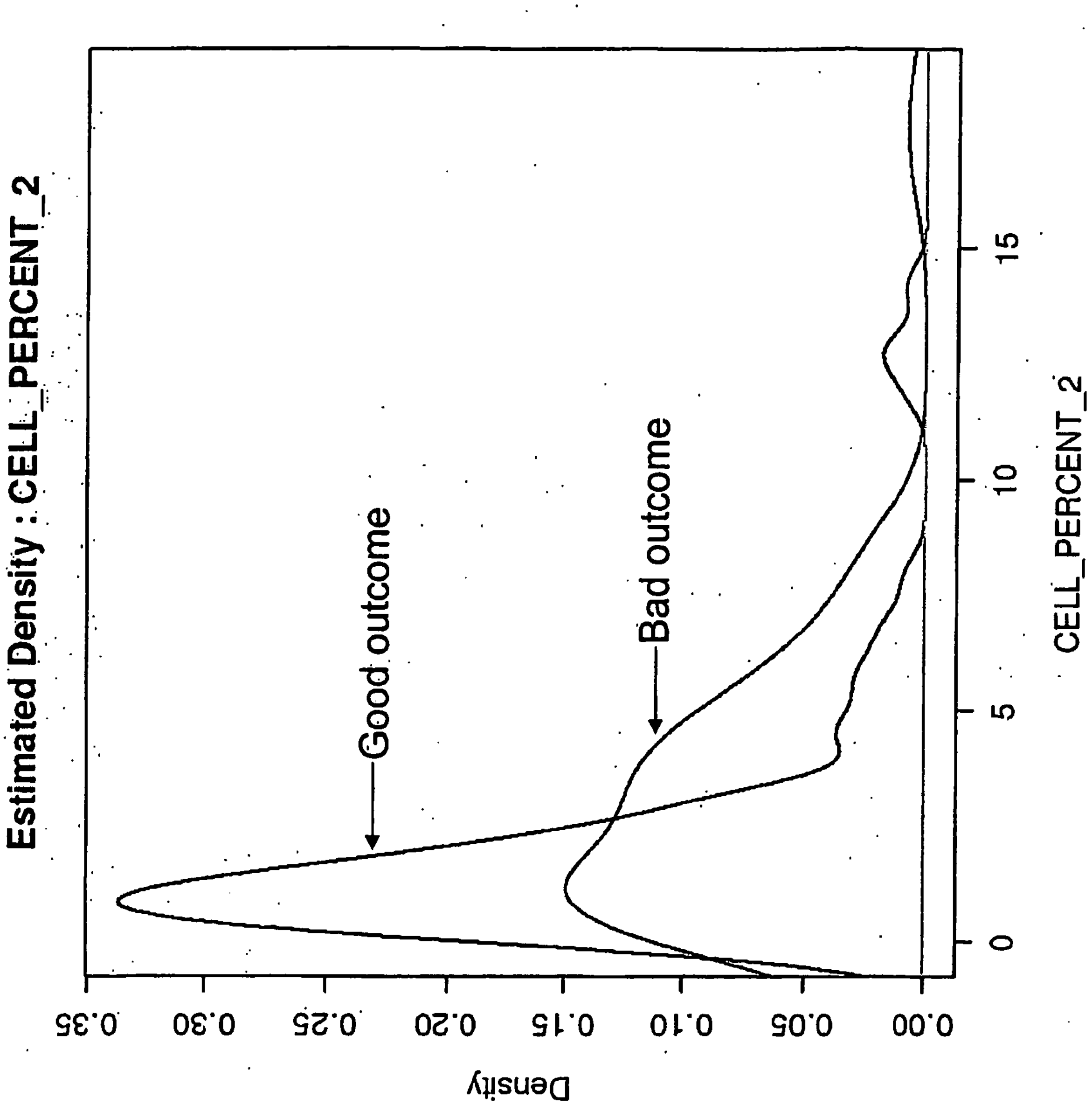
Estimated Density : CELL_PERCENT_2
Good outcome
Bad outcome
Density
0.00
0.05
0.10
0.15
0.20
0.25
0.30
0.35
0
5
10
15
CELL_PERCENT_2

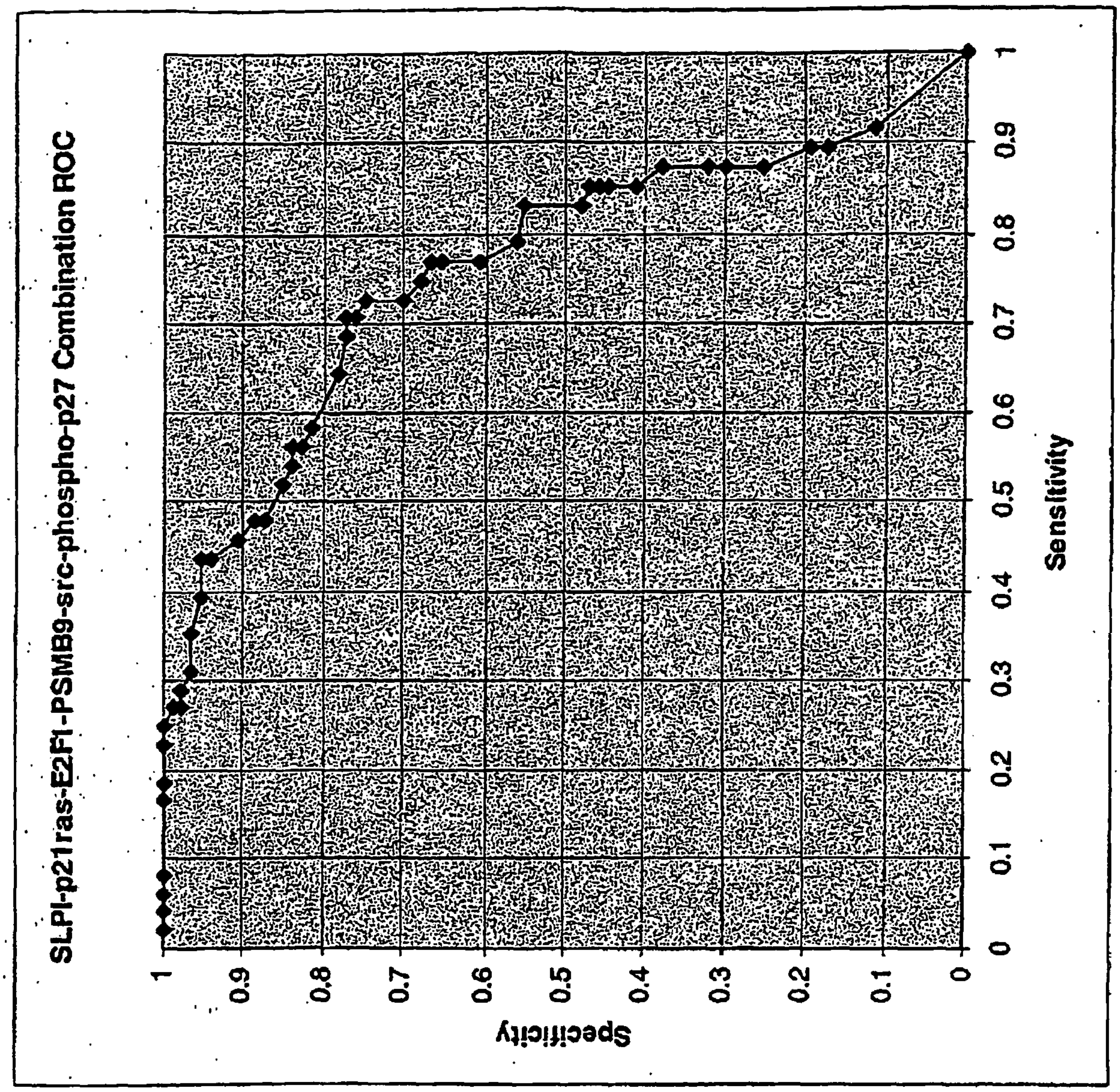
SLPI-p21ras-E2F1-PSMB9-src-phospho-p27 Combination ROC
Specificity
1
0.9
0.8
0.7
0.6
0.5
0.4
0.3
0.2
0.1
0
Sensitivity
0
0.1
0.2
0.3
0.4
0.5
0.6
0.7
0.8
0.9
1

FIG. 2

FIG. 3

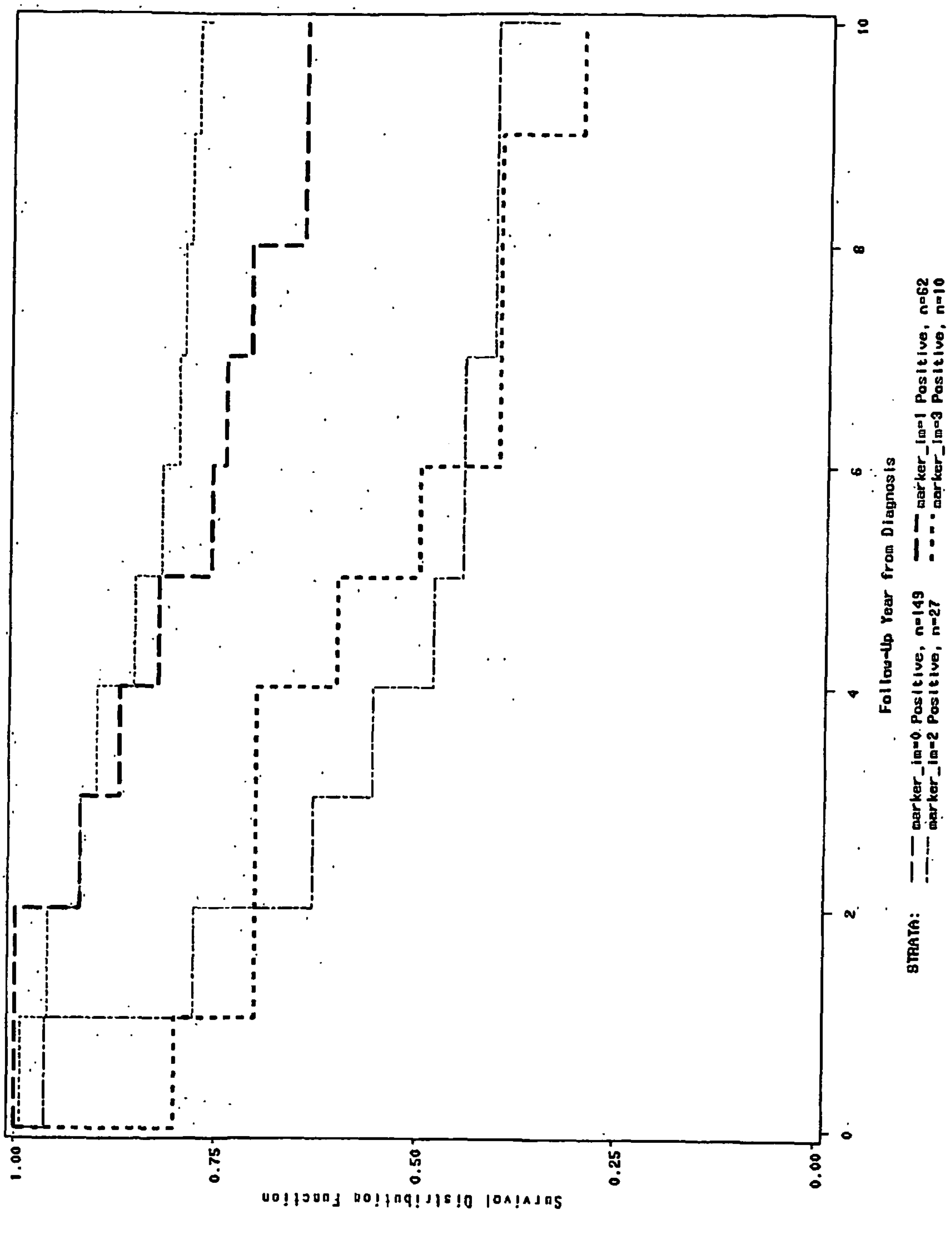
Survival Distribution Function
1.00
0.75
0.50
0.25
0.00
0
2
4
6
8
10
Follow-Up Year from Diagnosis
STRATA:
marker_im=0 Positive, n=149
marker_im=1 Positive, n=62
marker_im=2 Positive, n=27
marker_im=3 Positive, n=10

FIG. 4

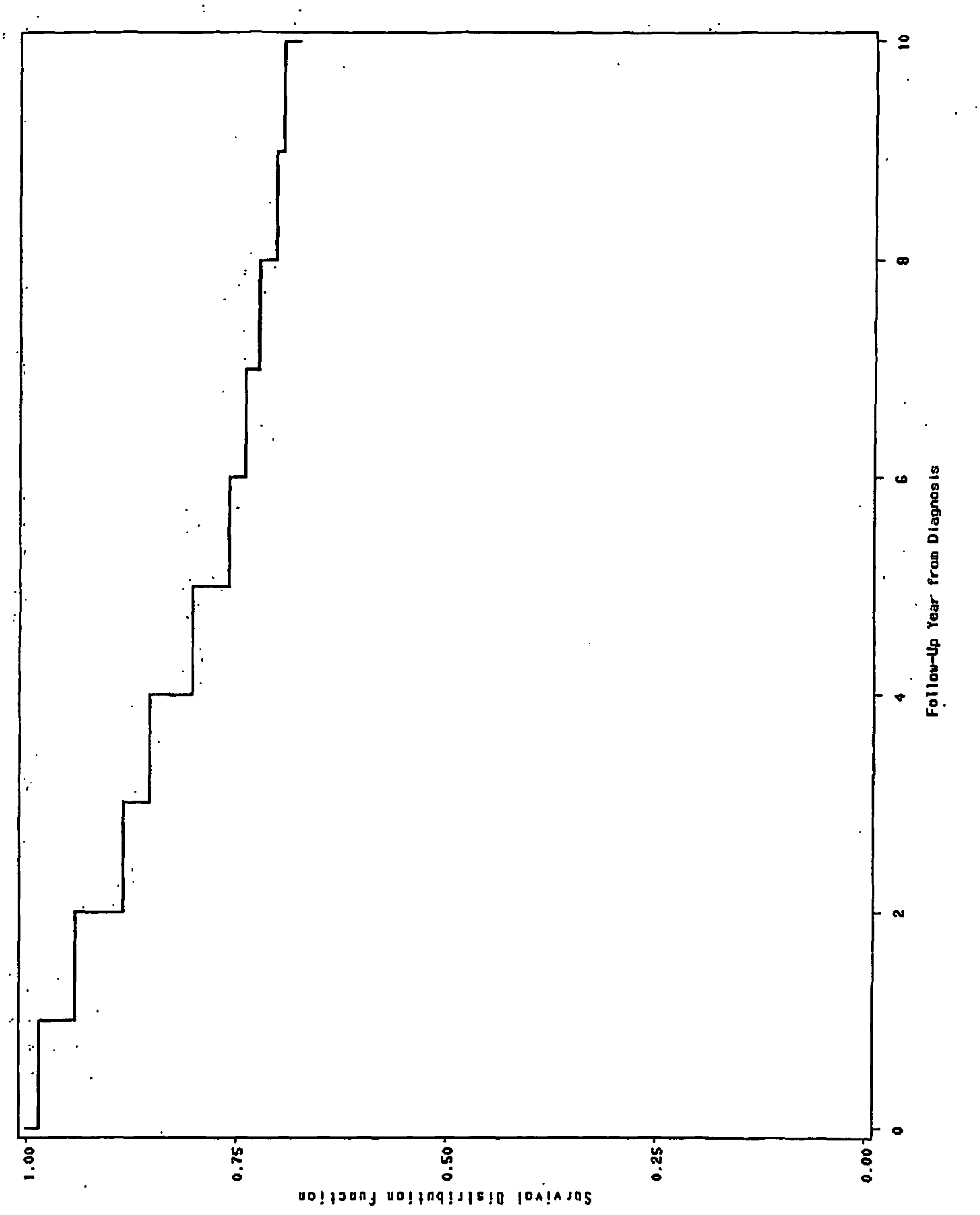
Survival Distribution Function
1.00
0.75
0.50
0.25
0.00
Follow-Up Year from Diagnosis
0
2
4
6
8
10

FIG. 5

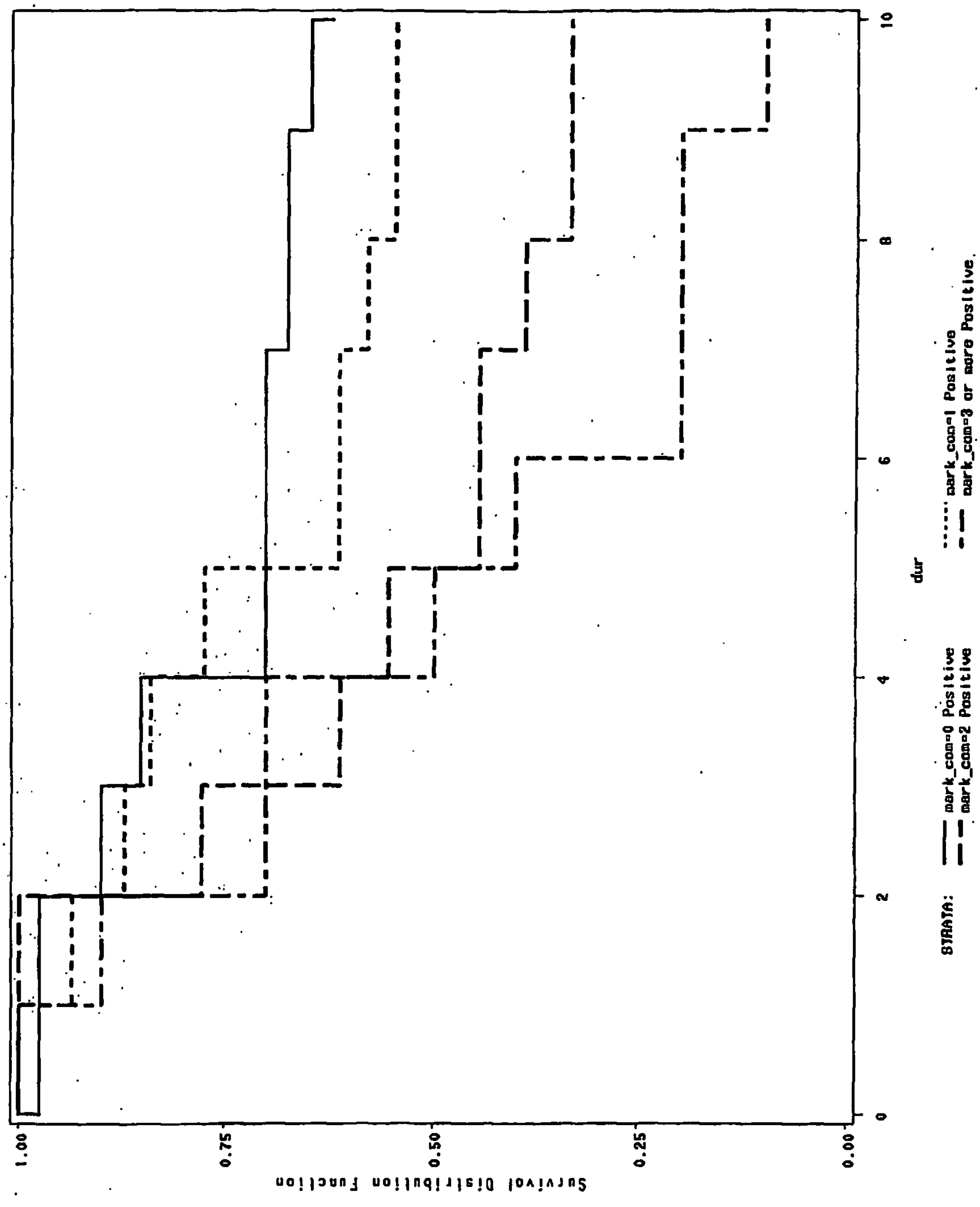
Survival Distribution Function
1.00
0.75
0.50
0.25
0.00
0
2
4
6
8
10
dur
STRATA:
mark_com=0 Positive
mark_com=2 Positive
mark_com=1 Positive
mark_com=3 or more Positive

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5545403 A
- US 5545405 A
- US 5998144 A
- US 5223409 A
- WO 9218619 A
- WO 9117271 A
- WO 9220791 A
- WO 9215679 A
- WO 9301288 A
- WO 9201047 A
- WO 9209690 A
- WO 9002809 A
- US 4708871 A
- US 4873192 A
- EP 75444 A
- US 4820504 A
- US 60612073 B
- US 60611965 B


### Non-patent literature cited in the description


- Molecular Oncology of Breast Cancer. Jones and Bartlett Publishers
- **Fitzgibbons et al.** *Arch. Pathol..Lab. Med.,* 2000, vol. 124, 966-978
- **Ross et al.** *The Oncologist,* 2003, 307-325
- **Spruance et al.** *Antimicrob. Agents & Chemo.,* 2004, vol. 48, 2787-2792
- **Goldhirsch et al.** *J. Clin. Oncol.,* 2001, vol. 19, 3817-3827
- **Correll ; Zoll.** *Human Genetics,* 1988, vol. 79, 225-259
- **Tong et al.** *Nature,* 1989, vol. 337, 90-93
- **Watson et al.** *Breast Cancer Res. Treat.,* 1991, vol. 17, 161-169
- **Dati et al.** *Int. J. Cancer,* 1991, vol. 47, 833-838
- **Archer et al.** *Br. J. Cancer,* 1995, vol. 72, 1259-1266
- **Bland et al.** *Ann. Surg.,* 1995, vol. 221, 706-718
- **Shackney et al.** *Clin. Cancer Res.,* 1998, vol. 4, 913-928
- **Gohring et al.** *Tumor Biol.,* 1999, vol. 20, 173-183
- **Freeman et al.** *Clin. Cancer Res.,* 1999, vol. 5, 2121-2132
- **Lei et al.** *J. Cell Sci.,* 2001, vol. 114, 1447-1454
- **Ishimi et al.** *Eur. J. Biochem.,* 2003, vol. 270, 1089-1101
- Tumor Marker Protocols. Humana Press, Inc, 1998
- Antigen Retrieval Techniques: Immunohistochemistry and Molecular Morphology. Eaton Publishing, 2000
- **Zapata et al.** *Protein Eng.,* 1995, vol. 8 (10), 1057-1062
- **Kohler et al.** *Nature,* 1975, vol. 256, 495-496
- **Fuchs et al.** *Bio/Technology,* 1991, vol. 9, 1370-1372
- **Hay et al.** *Hum. Antibod. Hybridomas,* 1992, vol. 3, 81-85
- **Huse et al.** *Science,* 1989, vol. 246, 1275-1281
- **Griffiths et al.** *EMBO J.,* 1993, vol. 12, 725-734
- **Kohler ; Milstein.** *Nature,* 1975, vol. 256, 495-497
- **Kozbor et al.** *Immunol. Today,* 1983, vol. 4, 72
- **Cole et al.** Monoclonal Antibodies and Cancer Therapy. Alan R. Liss, Inc, 1985, 77-96
- Current Protocols in Immunology. John Wiley & Sons, Inc, 1994
- **Galfre et al.** *Nature,* 1977, vol. 266, 55052
- **Kenneth.** Monoclonal Antibodies: A New Dimension In Biological Analyses. Plenum Publishing Corp, 1980
- **Lerner.** *Yale J. Biol. Med.,* 1981, vol. 54, 387-402
- Techniques in Molecular Biology. MacMillan Publishing Company, 1983
- **Kunkel.** *Proc. Natl. Acad. Sci. USA,* 1985, vol. 82, 488-492
- **Kunkel et al.** *Methods Enzymol.,* 1987, vol. 154, 367-382
- **Sambrook et al.** Molecular Cloning: A Laboratory Manual. Cold Spring Harbor, 1989
- **Dayhoff et al.** Atlas of Protein Sequence and Structure. Natl. Biomed. Res. Found, 1978

- **Smith ; Waterman.** *Adv. Appl. Math.,* 1981, vol. 2, 482-489
- Cell Biology & Laboratory Handbook. Academic Press
- Tumor Marker Protocols. Humana Press, Inc,

SEQUENCE LISTING

<110> TriPath Imaging, Inc.
Fischer, Timothy J.
Whitehead, Clark M.
Malinowski, Douglas P.
Marcelpoil, Raphael
Morel, Didier

<120> Methods and Compositions for Evaluating Breast Cancer Prognosis

<130> 46143/296716

<150> 60/612,073
<151> 2004-09-22

<150> 60/611,965
<151> 2004-09-22

<160> 41
<170> FastSEQ for Windows Version 4.0

<210> 1
<211> 598
<212> DNA
<213> Homo sapiens

<220>
<221> CDS
<222> (23)...(421)

<400> 1

```
cagagtcact cctgccttca cc atg aag tcc agc ggc ctc ttc ccc ttc ctg   52
                         Met Lys Ser Ser Gly Leu Phe Pro Phe Leu
                         1               5                   10

gtg ctg ctt gcc ctg gga act ctg gca cct tgg gct gtg gaa ggc tct   100
Val Leu Leu Ala Leu Gly Thr Leu Ala Pro Trp Ala Val Glu Gly Ser
                15                  20                  25

gga aag tcc ttc aaa gct gga gtc tgt cct cct aag aaa tct gcc cag   148
Gly Lys Ser Phe Lys Ala Gly Val Cys Pro Pro Lys Lys Ser Ala Gln
            30                  35                  40

tgc ctt aga tac aag aaa cct gag tgc cag agt gac tgg cag tgt cca   196
Cys Leu Arg Tyr Lys Lys Pro Glu Cys Gln Ser Asp Trp Gln Cys Pro
        45                  50                  55

ggg aag aag aga tgt tgt cct gac act tgt ggc atc aaa tgc ctg gat   244
Gly Lys Lys Arg Cys Cys Pro Asp Thr Cys Gly Ile Lys Cys Leu Asp
    60                  65                  70

cct gtt gac acc cca aac cca aca agg agg aag cct ggg aag tgc cca   292
Pro Val Asp Thr Pro Asn Pro Thr Arg Arg Lys Pro Gly Lys Cys Pro
75                  80                  85                  90

gtg act tat ggc caa tgt ttg atg ctt aac ccc ccc aat ttc tgt gag   340
Val Thr Tyr Gly Gln Cys Leu Met Leu Asn Pro Pro Asn Phe Cys Glu
                95                  100                 105

atg gat ggc cag tgc aag cgt gac ttg aag tgt tgc atg ggc atg tgt   388
Met Asp Gly Gln Cys Lys Arg Asp Leu Lys Cys Cys Met Gly Met Cys


            110                 115                 120

ggg aaa tcc tgc gtt tcc cct gtg aaa gct tga ttcctgccat atggaggagg 441
Gly Lys Ser Cys Val Ser Pro Val Lys Ala  *
        125                 130

ctctggagtc ctgctctgtg tggtccaggt cctttccacc ctgagacttg gctccaccac 501
tgatatcctc ctttggggaa aggcttggca cacagcaggc tttcaagaag tgccagttga 561
tcaatgaata aataaacgag cctatttctc tttgcac                           598
```

<210> 2
<211> 132
<212> PRT
<213> Homo sapiens

<400> 2

```
Met Lys Ser Ser Gly Leu Phe Pro Phe Leu Val Leu Leu Ala Leu Gly
 1               5                  10                  15
Thr Leu Ala Pro Trp Ala Val Glu Gly Ser Gly Lys Ser Phe Lys Ala
            20                  25                  30
Gly Val Cys Pro Pro Lys Lys Ser Ala Gln Cys Leu Arg Tyr Lys Lys
        35                  40                  45
Pro Glu Cys Gln Ser Asp Trp Gln Cys Pro Gly Lys Lys Arg Cys Cys
    50                  55                  60
Pro Asp Thr Cys Gly Ile Lys Cys Leu Asp Pro Val Asp Thr Pro Asn
65                  70                  75                  80
Pro Thr Arg Arg Lys Pro Gly Lys Cys Pro Val Thr Tyr Gly Gln Cys
                85                  90                  95
Leu Met Leu Asn Pro Pro Asn Phe Cys Glu Met Asp Gly Gln Cys Lys
            100                 105                 110
Arg Asp Leu Lys Cys Cys Met Gly Met Cys Gly Lys Ser Cys Val Ser
        115                 120                 125
Pro Val Lys Ala
    130
```

<210> 3
<211> 1513
<212> DNA
<213> Homo sapiens

<220>
<221> CDS
<222> (236)...(742)

<400> 3

```
attcacttct cacaaggact gggtgaagag ttctgcagcc ttacagagac tggaaaagaa 60
gcccaaacca aggcccccag agaggtcccc caggcccctt tgggtccctg agcctcagct 120
ggaggtgggg ggtgcctgca gtgcgctggc tcagtctcct tctgaaaagc tggatccagc 180
ttgtttgaag cccttgagct gatcttagat ccggcgcagg agaccaacgc ctgcc atg  238
                                                             Met
                                                              1

ctg ttc cgg ctc tca gag cac tcc tca cca gag gag gaa gcc tcc ccc   286
Leu Phe Arg Leu Ser Glu His Ser Ser Pro Glu Glu Glu Ala Ser Pro
            5                   10                  15

cac cag aga gcc tca gga gag ggg cac cat ctc aag tcg aag aga ccc   334
His Gln Arg Ala Ser Gly Glu Gly His His Leu Lys Ser Lys Arg Pro
        20                  25                  30

aac ccc tgt gcc tac aca cca cct tcg ctg aaa gct gtg cag cgc att   382
Asn Pro Cys Ala Tyr Thr Pro Pro Ser Leu Lys Ala Val Gln Arg Ile
    35                  40                  45

gct gag tct cac ctg cag tct atc agc aat ttg aat gag aac cag gcc   430
```

```
Ala Glu Ser His Leu Gln Ser Ile Ser Asn Leu Asn Glu Asn Gln Ala
 50                 55                  60                  65

tca gag gag gag gat gag ctg ggg gag ctt cgg gag ctg ggt tat cca     478
Ser Glu Glu Glu Asp Glu Leu Gly Glu Leu Arg Glu Leu Gly Tyr Pro
                70                  75                  80

aga gag gaa gat gag gag gaa gag gag gat gat gaa gaa gag gaa gaa     526
Arg Glu Glu Asp Glu Glu Glu Glu Glu Asp Asp Glu Glu Glu Glu Glu
            85                  90                  95

gaa gag gac agc cag gct gaa gtc ctg aag gtc atc agg cag tct gct     574
Glu Glu Asp Ser Gln Ala Glu Val Leu Lys Val Ile Arg Gln Ser Ala
        100                 105                 110

ggg caa aag aca acc tgt ggc cag ggt ctg gaa ggg ccc tgg gag cgc     622
Gly Gln Lys Thr Thr Cys Gly Gln Gly Leu Glu Gly Pro Trp Glu Arg
    115                 120                 125

cca ccc cct ctg gat gag tcc gag aga gat gga ggc tct gag gac caa     670
Pro Pro Pro Leu Asp Glu Ser Glu Arg Asp Gly Gly Ser Glu Asp Gln
130                 135                 140                 145

gtg gaa gac cca gca cta agt gag cct ggg gag gaa cct cag cgc cct     718
Val Glu Asp Pro Ala Leu Ser Glu Pro Gly Glu Glu Pro Gln Arg Pro
                150                 155                 160

tcc ccc tct gag cct ggc aca tag gcacccagcc tgcatctccc aggaggaagt    772
Ser Pro Ser Glu Pro Gly Thr  *
            165

ggaggggaca tcgctgttcc ccagaaaccc actctatcct caccctgttt tgtgctcttc 832
ccctcgcctg ctagggctgc ggcttctgac ttctagaaga ctaaggctgg tctgtgtttg 892
cttgtttgcc cacctttggc tgatacccag agaacctggg cacttgctgc ctgatgccca 952
cccctgccag tcattcctcc attcacccag cgggaggtgg gatgtgagac agcccacatt 1012
ggaaaatcca gaaaaccggg aacagggatt tgcccttcac aattctactc cccagatcct 1072
ctcccctgga cacaggagac ccacagggca ggaccctaag atctggggaa aggaggtcct 1132
gagaaccttg aggtaccctt agatcctttt ctacccactt tcctatggag gattccaagt 1192
caccacttct ctcaccggct tctaccaggg tccaggacta aggcgttttt ctccatagcc 1252
tcaacatttt gggaatcttc ccttaatcac ccttgctcct cctgggtgcc tggaagatgg 1312
actggcagag acctctttgt tgcgttttgt gctttgatgc caggaatgcc gcctagttta 1372
tgtccccggt ggggcacaca gcggggggcg ccaggttttc cttgtccccc agctgctctg 1432
cccctttccc cttcttccct gactccaggc ctgaacccct cccgtgctgt aataaatctt 1492
tgtaaataac aaaaaaaaaa a                                           1513
```

<210> 4
<211> 168
<212> PRT
<213> Homo sapiens

<400> 4

```
Met Leu Phe Arg Leu Ser Glu His Ser Ser Pro Glu Glu Glu Ala Ser
 1               5                  10                  15
Pro His Gln Arg Ala Ser Gly Glu Gly His His Leu Lys Ser Lys Arg
            20                  25                  30
Pro Asn Pro Cys Ala Tyr Thr Pro Pro Ser Leu Lys Ala Val Gln Arg
        35                  40                  45
Ile Ala Glu Ser His Leu Gln Ser Ile Ser Asn Leu Asn Glu Asn Gln
    50                  55                  60
Ala Ser Glu Glu Glu Asp Glu Leu Gly Glu Leu Arg Glu Leu Gly Tyr
65                  70                  75                  80
Pro Arg Glu Glu Asp Glu Glu Glu Glu Glu Asp Asp Glu Glu Glu Glu
                85                  90                  95
Glu Glu Glu Asp Ser Gln Ala Glu Val Leu Lys Val Ile Arg Gln Ser
            100                 105                 110
Ala Gly Gln Lys Thr Thr Cys Gly Gln Gly Leu Glu Gly Pro Trp Glu
        115                 120                 125
Arg Pro Pro Pro Leu Asp Glu Ser Glu Arg Asp Gly Gly Ser Glu Asp
    130                 135                 140
Gln Val Glu Asp Pro Ala Leu Ser Glu Pro Gly Glu Glu Pro Gln Arg
145                 150                 155                 160
Pro Ser Pro Ser Glu Pro Gly Thr
                165
```

<210> 5
<211> 748
<212> DNA
<213> Homo sapiens

<220>
<221> CDS
<222> (8)...(355)

<400> 5

```
ggccgcg atg agc ggg gag ccg ggg cag acg tcc gta gcg ccc cct ccc    49
        Met Ser Gly Glu Pro Gly Gln Thr Ser Val Ala Pro Pro Pro
        1               5                   10

gag gag gtc gag ccg ggc agt ggg gtc cgc atc gtg gtg gag tac tgt    97
Glu Glu Val Glu Pro Gly Ser Gly Val Arg Ile Val Val Glu Tyr Cys
15                  20                  25                  30

gaa ccc tgc ggc ttc gag gcg acc tac ctg gag ctg gcc agt gct gtg    145
Glu Pro Cys Gly Phe Glu Ala Thr Tyr Leu Glu Leu Ala Ser Ala Val
                35                  40                  45

aag gag cag tat ccg ggc atc gag atc gag tcg cgc ctc ggg ggc aca    193
Lys Glu Gln Tyr Pro Gly Ile Glu Ile Glu Ser Arg Leu Gly Gly Thr
            50                  55                  60

ggt gcc ttt gag ata gag ata aat gga cag ctg gtg ttc tcc aag ctg    241
Gly Ala Phe Glu Ile Glu Ile Asn Gly Gln Leu Val Phe Ser Lys Leu
        65                  70                  75

gag aat ggg ggc ttt ccc tat gag aaa gat ctc att gag gcc atc cga    289
Glu Asn Gly Gly Phe Pro Tyr Glu Lys Asp Leu Ile Glu Ala Ile Arg
    80                  85                  90

aga gcc agt aat gga gaa acc cta gaa aag atc acc aac agc cgt cct    337
Arg Ala Ser Asn Gly Glu Thr Leu Glu Lys Ile Thr Asn Ser Arg Pro
95                  100                 105                 110

ccc tgc gtc atc ctg tga ctgcacagga ctctgggttc ctgctctgtt           385
Pro Cys Val Ile Leu  *
                115

ctggggtcca aaccttggtc tccctttggt cctgctggga gctccccctg cctctttccc 445
ctacttagct ccttagcaaa gagaccctgg cctccacttt gccctttggg tacaaagaag 505
gaatagaaga ttccgtggcc ttgggggcag gagagagaca ctctccatga acacttctcc 565
agccacctca taccccttc ccagggtaag tgcccacgaa agcccagtcc actcttcgcc 625
tcggtaatac ctgtctgatg ccacagattt tatttattct cccctaaccc agggcaatgt 685
cagctattgg cagtaaagtg gcgctacaaa cactaaaaaa aaaaaaaaaa aaaaaaaaaa 745
aaa                                                               748
```

<210> 6
<211> 115
<212> PRT
<213> Homo sapiens

<400> 6

```
Met Ser Gly Glu Pro Gly Gln Thr Ser Val Ala Pro Pro Pro Glu Glu
```

```
1               5                   10                  15
Val Glu Pro Gly Ser Gly Val Arg Ile Val Val Glu Tyr Cys Glu Pro
            20                  25                  30
Cys Gly Phe Glu Ala Thr Tyr Leu Glu Leu Ala Ser Ala Val Lys Glu
        35                  40                  45
Gln Tyr Pro Gly Ile Glu Ile Glu Ser Arg Leu Gly Gly Thr Gly Ala
    50                  55                  60
Phe Glu Ile Glu Ile Asn Gly Gln Leu Val Phe Ser Lys Leu Glu Asn
65                  70                  75                  80
Gly Gly Phe Pro Tyr Glu Lys Asp Leu Ile Glu Ala Ile Arg Arg Ala
                85                  90                  95
Ser Asn Gly Glu Thr Leu Glu Lys Ile Thr Asn Ser Arg Pro Pro Cys
            100                 105                 110
Val Ile Leu
        115
```

<210> 7
<211> 3020
<212> DNA
<213> Homo sapiens

<220>
<221> CDS
<222> (111)...(1295)

<400> 7

```
tgaagctcgt cagttcacca tccgccctcg gcttccgcgg ggcgctgggc cgccagcctc 60
ggcaccgtcc tttcctttct ccctcgcgtt aggcaggtga cagcagggac atg tct   116
                                                      Met Ser
                                                      1

cgg gag atg cag gat gta gac ctc gct gag gtg aag cct ttg gtg gag   164
Arg Glu Met Gln Asp Val Asp Leu Ala Glu Val Lys Pro Leu Val Glu
        5                   10                  15

aaa ggg gag acc atc acc ggc ctc ctg caa gag ttt gat gtc cag gag   212
Lys Gly Glu Thr Ile Thr Gly Leu Leu Gln Glu Phe Asp Val Gln Glu
    20                  25                  30

cag gac atc gag act tta cat ggc tct gtt cac gtc acg ctg tgt ggg   260
Gln Asp Ile Glu Thr Leu His Gly Ser Val His Val Thr Leu Cys Gly
35                  40                  45                  50

act ccc aag gga aac cgg cct gtc atc ctc acc tac cat gac atc ggc   308
Thr Pro Lys Gly Asn Arg Pro Val Ile Leu Thr Tyr His Asp Ile Gly
                55                  60                  65

atg aac cac aaa acc tgc tac aac ccc ctc ttc aac tac gag gac atg   356
Met Asn His Lys Thr Cys Tyr Asn Pro Leu Phe Asn Tyr Glu Asp Met
            70                  75                  80

cag gag atc acc cag cac ttt gcc gtc tgc cac gtg gac gcc cct ggc   404
Gln Glu Ile Thr Gln His Phe Ala Val Cys His Val Asp Ala Pro Gly
        85                  90                  95

cag cag gac ggc gca gcc tcc ttc ccc gca ggg tac atg tac ccc tcc   452
Gln Gln Asp Gly Ala Ala Ser Phe Pro Ala Gly Tyr Met Tyr Pro Ser
    100                 105                 110

atg gat cag ctg gct gaa atg ctt cct gga gtc ctt caa cag ttt ggg   500
Met Asp Gln Leu Ala Glu Met Leu Pro Gly Val Leu Gln Gln Phe Gly
115                 120                 125                 130

ctg aaa agc att att ggc atg gga aca gga gca ggc gcc tac acc cta   548
Leu Lys Ser Ile Ile Gly Met Gly Thr Gly Ala Gly Ala Tyr Thr Leu
```

```
                135                     140                     145

act cga ttt gct cta aac aac cct gag atg gtg gag ggc ctt gtc ctt     596
Thr Arg Phe Ala Leu Asn Asn Pro Glu Met Val Glu Gly Leu Val Leu
            150                 155                 160

atc aac gtg aac cct tgt gcg gaa ggc tgg atg gac tgg gcc gcc tcc     644
Ile Asn Val Asn Pro Cys Ala Glu Gly Trp Met Asp Trp Ala Ala Ser
        165                 170                 175

aag atc tca gga tgg acc caa gct ctg ccg gac atg gtg gtg tcc cac     692
Lys Ile Ser Gly Trp Thr Gln Ala Leu Pro Asp Met Val Val Ser His
    180                 185                 190

ctt ttt ggg aag gaa gaa atg cag agt aac gtg gaa gtg gtc cac acc     740
Leu Phe Gly Lys Glu Glu Met Gln Ser Asn Val Glu Val Val His Thr
195                 200                 205                 210

tac cgc cag cac att gtg aat gac atg aac ccc ggc aac ctg cac ctg     788
Tyr Arg Gln His Ile Val Asn Asp Met Asn Pro Gly Asn Leu His Leu
                215                 220                 225

ttc atc aat gcc tac aac agc cgg cgc gac ctg gag att gag cga cca     836
Phe Ile Asn Ala Tyr Asn Ser Arg Arg Asp Leu Glu Ile Glu Arg Pro
            230                 235                 240

atg ccg gga acc cac aca gtc acc ctg cag tgc cct gct ctg ttg gtg     884
Met Pro Gly Thr His Thr Val Thr Leu Gln Cys Pro Ala Leu Leu Val
        245                 250                 255

gtt ggg gac agc tcg cct gca gtg gat gcc gtg gtg gag tgc aac tca     932
Val Gly Asp Ser Ser Pro Ala Val Asp Ala Val Val Glu Cys Asn Ser
    260                 265                 270

aaa ttg gac cca aca aag acc act ctc ctc aag atg gcg gac tgt ggc     980
Lys Leu Asp Pro Thr Lys Thr Thr Leu Leu Lys Met Ala Asp Cys Gly
275                 280                 285                 290

ggc ctc ccg cag atc tcc cag ccg gcc aag ctc gct gag gcc ttc aag    1028
Gly Leu Pro Gln Ile Ser Gln Pro Ala Lys Leu Ala Glu Ala Phe Lys
                295                 300                 305

tac ttc gtg cag ggc atg gga tac atg ccc tcg gct agc atg acc cgc    1076
Tyr Phe Val Gln Gly Met Gly Tyr Met Pro Ser Ala Ser Met Thr Arg
            310                 315                 320

ctg atg cgg tcc cgc aca gcc tct ggt tcc agc gtc act tct ctg gat    1124
Leu Met Arg Ser Arg Thr Ala Ser Gly Ser Ser Val Thr Ser Leu Asp
        325                 330                 335

ggc acc cgc agc cgc tcc cac acc agc gag ggc acc cga agc cgc tcc    1172
Gly Thr Arg Ser Arg Ser His Thr Ser Glu Gly Thr Arg Ser Arg Ser
    340                 345                 350

cac acc agc gag ggc acc cgc agc cgc tcg cac acc agc gag ggg gcc    1220
His Thr Ser Glu Gly Thr Arg Ser Arg Ser His Thr Ser Glu Gly Ala
355                 360                 365                 370

cac ctg gac atc acc ccc aac tcg ggt gct gct ggg aac agc gcc ggg    1268
His Leu Asp Ile Thr Pro Asn Ser Gly Ala Ala Gly Asn Ser Ala Gly
                375                 380                 385

ccc aag tcc atg gag gtc tcc tgc tag gcggcctgcc cagctgccgc          1315
Pro Lys Ser Met Glu Val Ser Cys  *
            390
```

```
ccccggactc tgatctctgt agtggccccc tcctccccgg cccctttcg ccccctgcct 1375
gccatactgc gcctaactcg gtattaatcc aaagcttatt ttgtaagagt gagctctggt 1435
ggagacaaat gaggtctatt acgtgggtgc cctctccaaa ggcggggtgg cggtggacca 1495
aaggaaggaa gcaagcatct ccgcatcgca tcctcttcca ttaaccagtg gccggttgcc 1555
actctcctcc cctccctcag agacaccaaa ctgccaaaaa caagacgcgt agcagcacac 1615
acttcacaaa gccaagccta ggccgccctg agcatcctgg ttcaaacggg tgcctggtca 1675
gaaggccagc cgcccacttc ccgtttcctc tttaactgag gagaagctga tccagctttc 1735
cggaaacaaa atccttttct tcatttgggg aggggggtaa tagtgacatg caggcacctc 1795
ttttaaacag gcaaaacagg aaggggggaaa aggtgggatt catgtcgagg ctagaggcat 1855
ttggaacaac aaatctacgt agttaacttg aagaaaccga tttttaaagt tggtgcatct 1915
agaaagcttt gaatgcagaa gcaaacaagc ttgatttttc tagcatcctc ttaatgtgca 1975
gcaaaagcag gcaacaaaat ctcctggctt tacagacaaa aatatttcag caaacgttgg 2035
gcatcatggt ttttgaaggc tttagttctg ctttctgcct ctcctccaca gccccaacct 2095
cccacccctg atacatgagc cagtgattat tcttgttcag ggagaagatc atttagattt 2155
gttttgcatt ccttagaatg gagggcaaca ttccacagct gccctggctg tgatgagtgt 2215
ccttgcaggg gccggagtag gagcactggg gtgggggcgg aattggggtt actcgatgta 2275
agggattcct tgttgttgtg ttgagatcca gtgcagttgt gatttctgtg gatcccagct 2335
tggtccagga atttgagag attggcttaa atccagtttt caatcttcga cagctgggct 2395
ggaacgtgaa ctcagtagct gaacctgtct gacccggtca cgttcttgga tcctcagaac 2455
tctttgctct tgtcggggtg ggggtgggaa ctcacgtggg gagcggtggc tgagaaaatg 2515
taaggattct ggaatacata ttccatggac tttccttccc tctcctgctt cctcttttcc 2575
tgctccctaa cctttcgccg aatggggcag acaaacactg acgtttctgg gtggccagtg 2635
cggctgccag gttcctgtac tactgccttg tacttttcat tttggctcac cgtggatttt 2695
ctcataggaa gtttggtcag agtgaattga atattgtaag tcagccactg ggacccgagg 2755
atttctggga ccccgcagtt gggaggagga agtagtccag ccttccaggt gggcgtgaga 2815
ggcaatgact cgttacctgc cgcccatcac cttggaggcc ttccctggcc ttgagtagaa 2875
aagtcgggga tcggggcaag agaggctgag tacggatggg aaactattgt gcacaagtct 2935
ttccagagga gtttcttaat gagatatttg tatttatttc cagaccaata aatttgtaac 2995
tttgcaaaaa aaaaaaaaaa aaaaa                                       3020
```

<210> 8
<211> 394
<212> PRT
<213> Homo sapiens

<400> 8

Met Ser Arg Glu Met Gln Asp Val Asp Leu Ala Glu Val Lys Pro Leu
1               5                   10                  15
Val Glu Lys Gly Glu Thr Ile Thr Gly Leu Leu Gln Glu Phe Asp Val
            20                  25                  30
Gln Glu Gln Asp Ile Glu Thr Leu His Gly Ser Val His Val Thr Leu
        35                  40                  45
Cys Gly Thr Pro Lys Gly Asn Arg Pro Val Ile Leu Thr Tyr His Asp
    50                  55                  60
Ile Gly Met Asn His Lys Thr Cys Tyr Asn Pro Leu Phe Asn Tyr Glu
65                  70                  75                  80
Asp Met Gln Glu Ile Thr Gln His Phe Ala Val Cys His Val Asp Ala
                85                  90                  95
Pro Gly Gln Gln Asp Gly Ala Ala Ser Phe Pro Ala Gly Tyr Met Tyr
            100                 105                 110
Pro Ser Met Asp Gln Leu Ala Glu Met Leu Pro Gly Val Leu Gln Gln
        115                 120                 125
Phe Gly Leu Lys Ser Ile Ile Gly Met Gly Thr Gly Ala Gly Ala Tyr
    130                 135                 140
Thr Leu Thr Arg Phe Ala Leu Asn Asn Pro Glu Met Val Glu Gly Leu
145                 150                 155                 160
Val Leu Ile Asn Val Asn Pro Cys Ala Glu Gly Trp Met Asp Trp Ala
                165                 170                 175
Ala Ser Lys Ile Ser Gly Trp Thr Gln Ala Leu Pro Asp Met Val Val
            180                 185                 190
Ser His Leu Phe Gly Lys Glu Glu Met Gln Ser Asn Val Glu Val Val
        195                 200                 205
His Thr Tyr Arg Gln His Ile Val Asn Asp Met Asn Pro Gly Asn Leu
    210                 215                 220
His Leu Phe Ile Asn Ala Tyr Asn Ser Arg Arg Asp Leu Glu Ile Glu
225                 230                 235                 240

Arg Pro Met Pro Gly Thr His Thr Val Thr Leu Gln Cys Pro Ala Leu
                245                 250                 255
Leu Val Val Gly Asp Ser Ser Pro Ala Val Asp Ala Val Val Glu Cys
            260                 265                 270
Asn Ser Lys Leu Asp Pro Thr Lys Thr Thr Leu Leu Lys Met Ala Asp
        275                 280                 285
Cys Gly Gly Leu Pro Gln Ile Ser Gln Pro Ala Lys Leu Ala Glu Ala
    290                 295                 300
Phe Lys Tyr Phe Val Gln Gly Met Gly Tyr Met Pro Ser Ala Ser Met
305                 310                 315                 320
Thr Arg Leu Met Arg Ser Arg Thr Ala Ser Gly Ser Ser Val Thr Ser
                325                 330                 335
Leu Asp Gly Thr Arg Ser Arg Ser His Thr Ser Glu Gly Thr Arg Ser
            340                 345                 350
Arg Ser His Thr Ser Glu Gly Thr Arg Ser Arg Ser His Thr Ser Glu
        355                 360                 365
Gly Ala His Leu Asp Ile Thr Pro Asn Ser Gly Ala Ala Gly Asn Ser
    370                 375                 380
Ala Gly Pro Lys Ser Met Glu Val Ser Cys
385                 390

<210> 9
<211> 778
<212> DNA
<213> Homo sapiens

<220>
<221> CDS
<222> (52)...(711)

<400> 9

```
caggttggaa accagtgccc caggcggcga ggagagcggt gccttgcagg g atg ctg    57
                                                         Met Leu
                                                          1

cgg gcg gga gca cca acc ggg gac tta ccc cgg gcg gga gaa gtc cac    105
Arg Ala Gly Ala Pro Thr Gly Asp Leu Pro Arg Ala Gly Glu Val His
        5                  10                  15

acc ggg acc acc atc atg gca gtg gag ttt gac ggg ggc gtt gtg atg    153
Thr Gly Thr Thr Ile Met Ala Val Glu Phe Asp Gly Gly Val Val Met
    20                  25                  30

ggt tct gat tcc cga gtg tct gca ggc gag gcg gtg gtg aac cga gtg    201
Gly Ser Asp Ser Arg Val Ser Ala Gly Glu Ala Val Val Asn Arg Val
 35                  40                  45                  50

ttt gac aag ctg tcc ccg ctg cac gag cgc atc tac tgt gca ctc tct    249
Phe Asp Lys Leu Ser Pro Leu His Glu Arg Ile Tyr Cys Ala Leu Ser
                 55                  60                  65

ggt tca gct gct gat gcc caa gcc gtg gcc gac atg gcc gcc tac cag    297
Gly Ser Ala Ala Asp Ala Gln Ala Val Ala Asp Met Ala Ala Tyr Gln
             70                  75                  80

ctg gag ctc cat ggg ata gaa ctg gag gaa cct cca ctt gtt ttg gct    345
Leu Glu Leu His Gly Ile Glu Leu Glu Glu Pro Pro Leu Val Leu Ala
        85                  90                  95

gct gca aat gtg gtg aga aat atc agc tat aaa tat cga gag gac ttg    393
Ala Ala Asn Val Val Arg Asn Ile Ser Tyr Lys Tyr Arg Glu Asp Leu
    100                 105                 110

tct gca cat ctc atg gta gct ggc tgg gac caa cgt gaa gga ggt cag    441
Ser Ala His Leu Met Val Ala Gly Trp Asp Gln Arg Glu Gly Gly Gln


115                 120                 125                 130

gta tat gga acc ctg gga gga atg ctg act cga cag cct ttt gcc att    489
Val Tyr Gly Thr Leu Gly Gly Met Leu Thr Arg Gln Pro Phe Ala Ile
                135                 140                 145

ggt ggc tcc ggc agc acc ttt atc tat ggt tat gtg gat gca gca tat    537
Gly Gly Ser Gly Ser Thr Phe Ile Tyr Gly Tyr Val Asp Ala Ala Tyr
            150                 155                 160

aag cca ggc atg tct ccc gag gag tgc agg cgc ttc acc aca gac gct    585
Lys Pro Gly Met Ser Pro Glu Glu Cys Arg Arg Phe Thr Thr Asp Ala
        165                 170                 175

att gct ctg gcc atg agc cgg gat ggc tca agc ggg ggt gtc atc tac    633
Ile Ala Leu Ala Met Ser Arg Asp Gly Ser Ser Gly Gly Val Ile Tyr
    180                 185                 190

ctg gtc act att aca gct gcc ggt gtg gac cat cga gtc atc ttg ggc    681
Leu Val Thr Ile Thr Ala Ala Gly Val Asp His Arg Val Ile Leu Gly
195                 200                 205                 210

aat gaa ctg cca aaa ttc tat gat gag tga accttcccca gacttctctt      731
Asn Glu Leu Pro Lys Phe Tyr Asp Glu  *
                215

tcttattttg taataaactc tctagggcca aaaaaaaaaa aaaaaaa                778
```

<210> 10

<211> 219
<212> PRT
<213> Homo sapiens

<400> 10

```
Met Leu Arg Ala Gly Ala Pro Thr Gly Asp Leu Pro Arg Ala Gly Glu
 1               5                  10                  15
Val His Thr Gly Thr Thr Ile Met Ala Val Glu Phe Asp Gly Gly Val
            20                  25                  30
Val Met Gly Ser Asp Ser Arg Val Ser Ala Gly Glu Ala Val Val Asn
        35                  40                  45
Arg Val Phe Asp Lys Leu Ser Pro Leu His Glu Arg Ile Tyr Cys Ala
    50                  55                  60
Leu Ser Gly Ser Ala Ala Asp Ala Gln Ala Val Ala Asp Met Ala Ala
65                  70                  75                  80
Tyr Gln Leu Glu Leu His Gly Ile Glu Leu Glu Glu Pro Pro Leu Val
                85                  90                  95
Leu Ala Ala Ala Asn Val Val Arg Asn Ile Ser Tyr Lys Tyr Arg Glu
            100                 105                 110
Asp Leu Ser Ala His Leu Met Val Ala Gly Trp Asp Gln Arg Glu Gly
        115                 120                 125
Gly Gln Val Tyr Gly Thr Leu Gly Gly Met Leu Thr Arg Gln Pro Phe
    130                 135                 140
Ala Ile Gly Gly Ser Gly Ser Thr Phe Ile Tyr Gly Tyr Val Asp Ala
145                 150                 155                 160
Ala Tyr Lys Pro Gly Met Ser Pro Glu Glu Cys Arg Arg Phe Thr Thr
                165                 170                 175
Asp Ala Ile Ala Leu Ala Met Ser Arg Asp Gly Ser Ser Gly Gly Val
            180                 185                 190
Ile Tyr Leu Val Thr Ile Thr Ala Ala Gly Val Asp His Arg Val Ile
        195                 200                 205
Leu Gly Asn Glu Leu Pro Lys Phe Tyr Asp Glu
    210                 215
```

<210> 11
<211> 2422
<212> DNA
<213> Homo sapiens

<220>
<221> CDS
<222> (466)...(1062)

<400> 11

```
gtcagcctcc cttccaccgc catattgggc cactaaaaaa agggggctcg tcttttcggg 60
gtgtttttct ccccctcccc tgtccccgct tgctcacggc tctgcgactc cgacgccggc 120
aaggtttgga gagcggctgg gttcgcggga cccgcgggct tgcacccgcc cagactcgga 180
cgggctttgc caccctctcc gcttgcctgg tcccctctcc tctccgccct cccgctcgcc 240
agtccatttg atcagcggag actcggcggc cgggccgggg cttccccgca gcccctgcgc 300
gctcctagag ctcgggccgt ggctcgtcgg ggtctgtgtc ttttggctcc gagggcagtc 360
gctgggcttc cgagaggggt tcgggccgcg taggggcgct ttgttttgtt cggttttgtt 420
tttttgagag tgcgagagag gcggtcgtgc agacccggga gaaag atg tca aac gtg 477
                                                  Met Ser Asn Val
                                                  1

cga gtg tct aac ggg agc cct agc ctg gag cgg atg gac gcc agg cag    525
Arg Val Ser Asn Gly Ser Pro Ser Leu Glu Arg Met Asp Ala Arg Gln
5                   10                  15                  20

gcg gag cac ccc aag ccc tcg gcc tgc agg aac ctc ttc ggc ccg gtg    573
Ala Glu His Pro Lys Pro Ser Ala Cys Arg Asn Leu Phe Gly Pro Val
                25                  30                  35

gac cac gaa gag tta acc cgg gac ttg gag aag cac tgc aga gac atg    621
Asp His Glu Glu Leu Thr Arg Asp Leu Glu Lys His Cys Arg Asp Met
            40                  45                  50

gaa gag gcg agc cag cgc aag tgg aat ttc gat ttt cag aat cac aaa    669
Glu Glu Ala Ser Gln Arg Lys Trp Asn Phe Asp Phe Gln Asn His Lys
        55                  60                  65

ccc cta gag ggc aag tac gag tgg caa gag gtg gag aag ggc agc ttg    717
Pro Leu Glu Gly Lys Tyr Glu Trp Gln Glu Val Glu Lys Gly Ser Leu
    70                  75                  80

ccc gag ttc tac tac aga ccc ccg cgg ccc ccc aaa ggt gcc tgc aag    765
Pro Glu Phe Tyr Tyr Arg Pro Pro Arg Pro Pro Lys Gly Ala Cys Lys
85                  90                  95                  100

gtg ccg gcg cag gag agc cag gat gtc agc ggg agc cgc ccg gcg gcg    813
Val Pro Ala Gln Glu Ser Gln Asp Val Ser Gly Ser Arg Pro Ala Ala
                105                 110                 115

cct tta att ggg gct ccg gct aac tct gag gac acg cat ttg gtg gac    861
Pro Leu Ile Gly Ala Pro Ala Asn Ser Glu Asp Thr His Leu Val Asp
            120                 125                 130

cca aag act gat ccg tcg gac agc cag acg ggg tta gcg gag caa tgc    909
Pro Lys Thr Asp Pro Ser Asp Ser Gln Thr Gly Leu Ala Glu Gln Cys
        135                 140                 145

gca gga ata agg aag cga cct gca acc gac gat tct tct act caa aac    957
Ala Gly Ile Arg Lys Arg Pro Ala Thr Asp Asp Ser Ser Thr Gln Asn
    150                 155                 160

aaa aga gcc aac aga aca gaa gaa aat gtt tca gac ggt tcc cca aat    1005
Lys Arg Ala Asn Arg Thr Glu Glu Asn Val Ser Asp Gly Ser Pro Asn
165                 170                 175                 180

gcc ggt tct gtg gag cag acg ccc aag aag cct ggc ctc aga aga cgt    1053
Ala Gly Ser Val Glu Gln Thr Pro Lys Lys Pro Gly Leu Arg Arg Arg
                185                 190                 195
```

caa acg taa acagctcgaa ttaagaatat gtttccttgt ttatcagata 1102
Gln Thr *

catcactgct tgatgaagca aggaagatat acatgaaaat tttaaaaata catatcgctg 1162
acttcatgga atggacatcc tgtataagca ctgaaaaaca acaacacaat aacactaaaa 1222
ttttaggcac tcttaaatga tctgcctcta aaagcgttgg atgtagcatt atgcaattag 1282
gtttttcctt atttgcttca ttgtactacc tgtgtatata gtttttacct tttatgtagc 1342
acataaactt tggggaaggg agggcagggt ggggctgagg aactgacgtg gagcggggta 1402
tgaagagctt gctttgattt acagcaagta gataaatatt tgacttgcat gaagagaagc 1462
aatttggggg aagggtttga attgttttct ttaaagatgt aatgtccctt tcagagacag 1522
ctgatacttc atttaaaaaa atcacaaaaa tttgaacact ggctaaagat aattgctatt 1582
tatttttaca agaagtttat tctcatttgg gagatctggt gatctcccaa gctatctaaa 1642
gtttgttaga tagctgcatg tggctttttt aaaaaagcaa cagaaaccta tcctcactgc 1702
cctccccagt ctctcttaaa gttggaattt accagttaat tactcagcag aatggtgatc 1762
actccaggta gtttggggca aaaatccgag gtgcttggga gttttgaatg ttaagaattg 1822
accatctgct tttattaaat ttgttgacaa aattttctca ttttcttttc acttcgggct 1882
gtgtaaacac agtcaaaata attctaaatc cctcgatatt tttaaagatc tgtaagtaac 1942
ttcacattaa aaaatgaaat atttttttaat ttaaagctta ctctgtccat ttatccacag 2002
gaaagtgtta ttttaaaagg aaggttcatg tagagaaaag cacacttgta ggataagtga 2062
aatggatact acatctttaa acagtatttc attgcctgtg tatggaaaaa ccatttgaag 2122
tgtacctgtg tacataactc tgtaaaaaca ctgaaaaatt atactaactt atttatgtta 2182
aaagattttt tttaatctag acaatataca agccaaagtg gcatgttttg tgcatttgta 2242
aatgctgtgt tgggtagaat aggttttccc ctcttttgtt aaataatatg gctatgctta 2302
aaaggttgca tactgagcca agtataattt tttgtaatgt gtgaaaaaga tgccaattat 2362
tgttacacat taagtaatca ataaagaaaa cttccatagc taaaaaaaaa aaaaaaaaaa 2422

<210> 12
<211> 198
<212> PRT
<213> Homo sapiens

<400> 12

Met Ser Asn Val Arg Val Ser Asn Gly Ser Pro Ser Leu Glu Arg Met
1 5 10 15
Asp Ala Arg Gln Ala Glu His Pro Lys Pro Ser Ala Cys Arg Asn Leu
20 25 30
Phe Gly Pro Val Asp His Glu Glu Leu Thr Arg Asp Leu Glu Lys His
35 40 45
Cys Arg Asp Met Glu Glu Ala Ser Gln Arg Lys Trp Asn Phe Asp Phe
50 55 60
Gln Asn His Lys Pro Leu Glu Gly Lys Tyr Glu Trp Gln Glu Val Glu
65 70 75 80
Lys Gly Ser Leu Pro Glu Phe Tyr Tyr Arg Pro Pro Arg Pro Pro Lys
85 90 95
Gly Ala Cys Lys Val Pro Ala Gln Glu Ser Gln Asp Val Ser Gly Ser
100 105 110
Arg Pro Ala Ala Pro Leu Ile Gly Ala Pro Ala Asn Ser Glu Asp Thr
115 120 125
His Leu Val Asp Pro Lys Thr Asp Pro Ser Asp Ser Gln Thr Gly Leu
130 135 140
Ala Glu Gln Cys Ala Gly Ile Arg Lys Arg Pro Ala Thr Asp Asp Ser
145 150 155 160
Ser Thr Gln Asn Lys Arg Ala Asn Arg Thr Glu Glu Asn Val Ser Asp
165 170 175
Gly Ser Pro Asn Ala Gly Ser Val Glu Gln Thr Pro Lys Lys Pro Gly
180 185 190
Leu Arg Arg Arg Gln Thr
195

<210> 13
<211> 2486

<212> DNA
<213> Homo sapiens

<220>
<221> CDS
<222> (124)...(1437)

<400> 13

```
ccgggacttt gcaggcagcg gcggccgggg gcggagcggg atcgagccct cgccgaggcc 60
tgccgccatg ggcccgcgcc gccgccgccg cctgtcaccc gggccgcgcg ggccgtgagc 120
gtc atg gcc ttg gcc ggg gcc cct gcg ggc ggc cca tgc gcg ccg gcg   168
    Met Ala Leu Ala Gly Ala Pro Ala Gly Gly Pro Cys Ala Pro Ala
     1               5                  10                  15

ctg gag gcc ctg ctc ggg gcc ggc gcg ctg cgg ctg ctc gac tcc tcg   216
Leu Glu Ala Leu Leu Gly Ala Gly Ala Leu Arg Leu Leu Asp Ser Ser
                20                  25                  30

cag atc gtc atc atc tcc gcc gcg cag gac gcc agc gcc ccg ccg gct   264
Gln Ile Val Ile Ile Ser Ala Ala Gln Asp Ala Ser Ala Pro Pro Ala
            35                  40                  45

ccc acc ggc ccc gcg gcg ccc gcc gcc ggc ccc tgc gac cct gac ctg   312
Pro Thr Gly Pro Ala Ala Pro Ala Ala Gly Pro Cys Asp Pro Asp Leu
        50                  55                  60

ctg ctc ttc gcc aca ccg cag gcg ccc cgg ccc aca ccc agt gcg ccg   360
Leu Leu Phe Ala Thr Pro Gln Ala Pro Arg Pro Thr Pro Ser Ala Pro
    65                  70                  75

cgg ccc gcg ctc ggc cgc ccg ccg gtg aag cgg agg ctg gac ctg gaa   408
Arg Pro Ala Leu Gly Arg Pro Pro Val Lys Arg Arg Leu Asp Leu Glu
80                  85                  90                  95

act gac cat cag tac ctg gcc gag agc agt ggg cca gct cgg ggc aga   456
Thr Asp His Gln Tyr Leu Ala Glu Ser Ser Gly Pro Ala Arg Gly Arg
                100                 105                 110

ggc cgc cat cca gga aaa ggt gtg aaa tcc ccg ggg gag aag tca cgc   504
Gly Arg His Pro Gly Lys Gly Val Lys Ser Pro Gly Glu Lys Ser Arg
            115                 120                 125

tat gag acc tca ctg aat ctg acc acc aag cgc ttc ctg gag ctg ctg   552
Tyr Glu Thr Ser Leu Asn Leu Thr Thr Lys Arg Phe Leu Glu Leu Leu
        130                 135                 140

agc cac tcg gct gac ggt gtc gtc gac ctg aac tgg gct gcc gag gtg   600
Ser His Ser Ala Asp Gly Val Val Asp Leu Asn Trp Ala Ala Glu Val
    145                 150                 155

ctg aag gtg cag aag cgg cgc atc tat gac atc acc aac gtc ctt gag   648
Leu Lys Val Gln Lys Arg Arg Ile Tyr Asp Ile Thr Asn Val Leu Glu
160                 165                 170                 175

ggc atc cag ctc att gcc aag aag tcc aag aac cac atc cag tgg ctg   696
Gly Ile Gln Leu Ile Ala Lys Lys Ser Lys Asn His Ile Gln Trp Leu
                180                 185                 190

ggc agc cac acc aca gtg ggc gtc ggc gga cgg ctt gag ggg ttg acc   744
Gly Ser His Thr Thr Val Gly Val Gly Gly Arg Leu Glu Gly Leu Thr
            195                 200                 205

cag gac ctc cga cag ctg cag gag agc gag cag cag ctg gac cac ctg   792
Gln Asp Leu Arg Gln Leu Gln Glu Ser Glu Gln Gln Leu Asp His Leu
        210                 215                 220

atg aat atc tgt act acg cag ctg cgc ctg ctc tcc gag gac act gac   840
```

```
Met Asn Ile Cys Thr Thr Gln Leu Arg Leu Leu Ser Glu Asp Thr Asp
    225                 230                 235

agc cag cgc ctg gcc tac gtg acg tgt cag gac ctt cgt agc att gca    888
Ser Gln Arg Leu Ala Tyr Val Thr Cys Gln Asp Leu Arg Ser Ile Ala
240                 245                 250                 255

gac cct gca gag cag atg gtt atg gtg atc aaa gcc cct cct gag acc    936
Asp Pro Ala Glu Gln Met Val Met Val Ile Lys Ala Pro Pro Glu Thr
                260                 265                 270

cag ctc caa gcc gtg gac tct tcg gag aac ttt cag atc tcc ctt aag    984
Gln Leu Gln Ala Val Asp Ser Ser Glu Asn Phe Gln Ile Ser Leu Lys
            275                 280                 285

agc aaa caa ggc ccg atc gat gtt ttc ctg tgc cct gag gag acc gta    1032
Ser Lys Gln Gly Pro Ile Asp Val Phe Leu Cys Pro Glu Glu Thr Val
        290                 295                 300

ggt ggg atc agc cct ggg aag acc cca tcc cag gag gtc act tct gag    1080
Gly Gly Ile Ser Pro Gly Lys Thr Pro Ser Gln Glu Val Thr Ser Glu
    305                 310                 315

gag gag aac agg gcc act gac tct gcc acc ata gtg tca cca cca cca    1128
Glu Glu Asn Arg Ala Thr Asp Ser Ala Thr Ile Val Ser Pro Pro Pro
320                 325                 330                 335

tca tct ccc ccc tca tcc ctc acc aca gat ccc agc cag tct cta ctc    1176
Ser Ser Pro Pro Ser Ser Leu Thr Thr Asp Pro Ser Gln Ser Leu Leu
                340                 345                 350

agc ctg gag caa gaa ccg ctg ttg tcc cgg atg ggc agc ctg cgg gct    1224
Ser Leu Glu Gln Glu Pro Leu Leu Ser Arg Met Gly Ser Leu Arg Ala
            355                 360                 365

ccc gtg gac gag gac cgc ctg tcc ccg ctg gtg gcg gcc gac tcg ctc    1272
Pro Val Asp Glu Asp Arg Leu Ser Pro Leu Val Ala Ala Asp Ser Leu
        370                 375                 380

ctg gag cat gtg cgg gag gac ttc tcc ggc ctc ctc cct gag gag ttc    1320
Leu Glu His Val Arg Glu Asp Phe Ser Gly Leu Leu Pro Glu Glu Phe
    385                 390                 395

atc agc ctt tcc cca ccc cac gag gcc ctc gac tac cac ttc ggc ctc    1368
Ile Ser Leu Ser Pro Pro His Glu Ala Leu Asp Tyr His Phe Gly Leu
400                 405                 410                 415

gag gag ggc gag ggc atc aga gac ctc ttc gac tgt gac ttt ggg gac    1416
Glu Glu Gly Glu Gly Ile Arg Asp Leu Phe Asp Cys Asp Phe Gly Asp
                420                 425                 430

ctc acc ccc ctg gat ttc tga cagggcttgg agggaccagg gtttccagag       1467
Leu Thr Pro Leu Asp Phe  *
            435

tagctcacct tgtctctgca gccctggagc cccctgtccc tggccgtcct cccagcctgt 1527
ttggaaacat ttaatttata cccctctcct ctgtctccag aagcttctag ctctggggtc 1587
tggctaccgc taggaggctg agcaagccag gaagggaagg agtctgtgtg gtgtgtatgt 1647
gcatgcagcc tacacccaca cgtgtgtacc gggggtgaat gtgtgtgagc atgtgtgtgt 1707
gcatgtaccg gggaatgaag gtgaacatac acctctgtgt gtgcactgca gacacgcccc 1767
agtgtgtcca catgtgtgtg catgagtcca tctctgcgcg tggggggggct ctaactgcac 1827
tttcggccct tttgctcgtg ggtcccacaa aggcccaggg cagtgcctgc tcccagaatc 1887
tggtgctctg accaggccag gtggggaggc tttggctggc tgggcgtgta ggacggtgag 1947
agcacttctg tcttaaaggt tttttctgat tgaagcttta atggagcgtt atttatttat 2007
cgaggcctct ttggtgagcc tggggaatca gcaaaagggg aggaggggtg tggggttgat 2067
accccaactc cctctaccct tgagcaaggg caggggtccc tgagctgttc ttctgcccca 2127
```

```
tactgaagga actgaggcct gggtgattta tttattggga aagtgaggga gggagacaga 2187
ctgactgaca gccatgggtg gtcagatggt ggggtgggcc ctctccaggg ggccagttca 2247
gggcccagct gccccccagg atggatatga gatgggagag gtgagtgggg gaccttcact 2307
gatgtgggca ggaggggtgg tgaaggcctc ccccagccca gaccctgtgg tccctcctgc 2367
agtgtctgaa gcgcctgcct ccccactgct ctgccccacc ctccaatctg cactttgatt 2427
tgcttcctaa cagctctgtt ccctcctgct ttggttttaa taaatatttt gatgacgtt  2486
```

<210> 14
<211> 437
<212> PRT
<213> Homo sapiens

<400> 14

Met Ala Leu Ala Gly Ala Pro Ala Gly Gly Pro Cys Ala Pro Ala Leu
1 5 10 15
Glu Ala Leu Leu Gly Ala Gly Ala Leu Arg Leu Leu Asp Ser Ser Gln
20 25 30
Ile Val Ile Ile Ser Ala Ala Gln Asp Ala Ser Ala Pro Pro Ala Pro
35 40 45
Thr Gly Pro Ala Ala Pro Ala Ala Gly Pro Cys Asp Pro Asp Leu Leu
50 55 60
Leu Phe Ala Thr Pro Gln Ala Pro Arg Pro Thr Pro Ser Ala Pro Arg
65 70 75 80
Pro Ala Leu Gly Arg Pro Pro Val Lys Arg Arg Leu Asp Leu Glu Thr
85 90 95
Asp His Gln Tyr Leu Ala Glu Ser Ser Gly Pro Ala Arg Gly Arg Gly
100 105 110
Arg His Pro Gly Lys Gly Val Lys Ser Pro Gly Glu Lys Ser Arg Tyr
115 120 125
Glu Thr Ser Leu Asn Leu Thr Thr Lys Arg Phe Leu Glu Leu Leu Ser
130 135 140
His Ser Ala Asp Gly Val Val Asp Leu Asn Trp Ala Ala Glu Val Leu
145 150 155 160
Lys Val Gln Lys Arg Arg Ile Tyr Asp Ile Thr Asn Val Leu Glu Gly
165 170 175
Ile Gln Leu Ile Ala Lys Lys Ser Lys Asn His Ile Gln Trp Leu Gly
180 185 190
Ser His Thr Thr Val Gly Val Gly Gly Arg Leu Glu Gly Leu Thr Gln
195 200 205
Asp Leu Arg Gln Leu Gln Glu Ser Glu Gln Gln Leu Asp His Leu Met
210 215 220
Asn Ile Cys Thr Thr Gln Leu Arg Leu Leu Ser Glu Asp Thr Asp Ser
225 230 235 240
Gln Arg Leu Ala Tyr Val Thr Cys Gln Asp Leu Arg Ser Ile Ala Asp
245 250 255
Pro Ala Glu Gln Met Val Met Val Ile Lys Ala Pro Pro Glu Thr Gln
260 265 270
Leu Gln Ala Val Asp Ser Ser Glu Asn Phe Gln Ile Ser Leu Lys Ser
275 280 285
Lys Gln Gly Pro Ile Asp Val Phe Leu Cys Pro Glu Glu Thr Val Gly
290 295 300
Gly Ile Ser Pro Gly Lys Thr Pro Ser Gln Glu Val Thr Ser Glu Glu
305 310 315 320
Glu Asn Arg Ala Thr Asp Ser Ala Thr Ile Val Ser Pro Pro Pro Ser
325 330 335
Ser Pro Pro Ser Ser Leu Thr Thr Asp Pro Ser Gln Ser Leu Leu Ser
340 345 350
Leu Glu Gln Glu Pro Leu Leu Ser Arg Met Gly Ser Leu Arg Ala Pro
355 360 365
Val Asp Glu Asp Arg Leu Ser Pro Leu Val Ala Ala Asp Ser Leu Leu
370 375 380
Glu His Val Arg Glu Asp Phe Ser Gly Leu Leu Pro Glu Glu Phe Ile
385 390 395 400
Ser Leu Ser Pro Pro His Glu Ala Leu Asp Tyr His Phe Gly Leu Glu
405 410 415
Glu Gly Glu Gly Ile Arg Asp Leu Phe Asp Cys Asp Phe Gly Asp Leu
420 425 430
Thr Pro Leu Asp Phe
435

<210> 15
<211> 3744
<212> DNA
<213> Homo sapiens

<220>
<221> CDS
<222> (56)...(2521)

<400> 15

```
ccacgcgtcc ggtggcggtc gagcgtggcg taggcgaatc ctcggcacta agcat atg   58
                                                              Met
                                                               1

gac ctc gcg gcg gca gcg gag ccg ggc gcc ggc agc cag cac ctg gag   106
Asp Leu Ala Ala Ala Ala Glu Pro Gly Ala Gly Ser Gln His Leu Glu
                5                   10                  15

gtc cgc gac gag gtg gcc gag aag tgc cag aaa ctg ttc ctg gac ttc   154
Val Arg Asp Glu Val Ala Glu Lys Cys Gln Lys Leu Phe Leu Asp Phe
            20                  25                  30

ttg gag gag ttt cag agc agc gat gga gaa att aaa tac ttg caa tta   202
Leu Glu Glu Phe Gln Ser Ser Asp Gly Glu Ile Lys Tyr Leu Gln Leu
        35                  40                  45

gca gag gaa ctg att cgt cct gag aga aac aca ttg gtt gtg agt ttt   250
Ala Glu Glu Leu Ile Arg Pro Glu Arg Asn Thr Leu Val Val Ser Phe
    50                  55                  60                  65

gtg gac ctg gaa caa ttt aac cag caa ctt tcc acc acc att caa gag   298
Val Asp Leu Glu Gln Phe Asn Gln Gln Leu Ser Thr Thr Ile Gln Glu
                    70                  75                  80

gag ttc tat aga gtt tac cct tac ctg tgt cgg gcc ttg aaa aca ttc   346
Glu Phe Tyr Arg Val Tyr Pro Tyr Leu Cys Arg Ala Leu Lys Thr Phe
                85                  90                  95

gtc aaa gac cgt aaa gag atc cct ctt gcc aag gat ttt tat gtt gca   394
Val Lys Asp Arg Lys Glu Ile Pro Leu Ala Lys Asp Phe Tyr Val Ala
            100                 105                 110

ttc caa gac ctg cct acc aga cac aag att cga gag ctc acc tca tcc   442
Phe Gln Asp Leu Pro Thr Arg His Lys Ile Arg Glu Leu Thr Ser Ser
        115                 120                 125

aga att ggt ttg ctc act cgc atc agt ggg cag gtg gtg cgg act cac   490
Arg Ile Gly Leu Leu Thr Arg Ile Ser Gly Gln Val Val Arg Thr His
130                 135                 140                 145

cca gtt cac cca gag ctt gtg agc gga act ttt ctg tgc ttg gac tgt   538
Pro Val His Pro Glu Leu Val Ser Gly Thr Phe Leu Cys Leu Asp Cys
                150                 155                 160

cag aca gtg atc agg gat gta gaa cag cag ttc aaa tac aca cag cca   586
Gln Thr Val Ile Arg Asp Val Glu Gln Gln Phe Lys Tyr Thr Gln Pro
            165                 170                 175

aac atc tgc cga aat cca gtt tgt gcc aac agg agg aga ttc tta ctg   634
Asn Ile Cys Arg Asn Pro Val Cys Ala Asn Arg Arg Arg Phe Leu Leu
        180                 185                 190
```

```
gat aca aat aaa tca aga ttt gtt gat ttt caa aag gtt cgt att caa    682
Asp Thr Asn Lys Ser Arg Phe Val Asp Phe Gln Lys Val Arg Ile Gln
    195                 200                 205

gag acc caa gct gag ctt cct cga ggg agt atc ccc cgc agt tta gaa    730
Glu Thr Gln Ala Glu Leu Pro Arg Gly Ser Ile Pro Arg Ser Leu Glu
210                 215                 220                 225

gta att tta agg gct gaa gct gtg gaa tca gct caa gct ggt gac aag    778
Val Ile Leu Arg Ala Glu Ala Val Glu Ser Ala Gln Ala Gly Asp Lys
                230                 235                 240

tgt gac ttt aca ggg aca ctg att gtt gtg cct gac gtc tcc aag ctt    826
Cys Asp Phe Thr Gly Thr Leu Ile Val Val Pro Asp Val Ser Lys Leu
            245                 250                 255

agc aca cca gga gca cgt gca gaa act aat tcc cgt gtc agt ggt gtt    874
Ser Thr Pro Gly Ala Arg Ala Glu Thr Asn Ser Arg Val Ser Gly Val
        260                 265                 270

gat gga tat gag aca gaa ggc att cga gga ctc cgg gcc ctt ggt gtt    922
Asp Gly Tyr Glu Thr Glu Gly Ile Arg Gly Leu Arg Ala Leu Gly Val
    275                 280                 285

agg gac ctt tct tat agg ctg gtc ttt ctt gcc tgc tgt gtt gcg cca    970
Arg Asp Leu Ser Tyr Arg Leu Val Phe Leu Ala Cys Cys Val Ala Pro
290                 295                 300                 305

acc aac cca agg ttt ggg ggg aaa gag ctc aga gat gag gaa cag aca    1018
Thr Asn Pro Arg Phe Gly Gly Lys Glu Leu Arg Asp Glu Glu Gln Thr
                310                 315                 320

gct gag agc att aag aac caa atg act gtg aaa gaa tgg gag aaa gtg    1066
Ala Glu Ser Ile Lys Asn Gln Met Thr Val Lys Glu Trp Glu Lys Val
            325                 330                 335

ttt gag atg agt caa gat aaa aat cta tac cac aat ctt tgt acc agc    1114
Phe Glu Met Ser Gln Asp Lys Asn Leu Tyr His Asn Leu Cys Thr Ser
        340                 345                 350

ctg ttc cct act ata cat ggc aat gat gaa gta aaa cgg ggt gtc ctg    1162
Leu Phe Pro Thr Ile His Gly Asn Asp Glu Val Lys Arg Gly Val Leu
    355                 360                 365

ctg atg ctc ttt ggt ggc gtt cca aag aca aca gga gaa ggg acc tct    1210
Leu Met Leu Phe Gly Gly Val Pro Lys Thr Thr Gly Glu Gly Thr Ser
370                 375                 380                 385

ctt cga ggg gac ata aat gtt tgc att gtt ggt gac cca agt aca gct    1258
Leu Arg Gly Asp Ile Asn Val Cys Ile Val Gly Asp Pro Ser Thr Ala
                390                 395                 400

aag agc caa ttt ctc aag cac gtg gag gag ttc agc ccc aga gct gtc    1306
Lys Ser Gln Phe Leu Lys His Val Glu Glu Phe Ser Pro Arg Ala Val
            405                 410                 415

tac acc agt ggt aaa gcg tcc agt gct gct ggc tta aca gca gct gtt    1354
Tyr Thr Ser Gly Lys Ala Ser Ser Ala Ala Gly Leu Thr Ala Ala Val
        420                 425                 430

gtg aga gat gaa gaa tct cat gag ttt gtc att gag gct gga gct ttg    1402
Val Arg Asp Glu Glu Ser His Glu Phe Val Ile Glu Ala Gly Ala Leu
    435                 440                 445

atg ttg gct gat aat ggt gtg tgt tgt att gat gaa ttt gat aag atg    1450
```

```
Met Leu Ala Asp Asn Gly Val Cys Cys Ile Asp Glu Phe Asp Lys Met
450                 455                 460                 465

gac gtg cgg gat caa gtt gct att cat gaa gct atg gaa cag cag acc     1498
Asp Val Arg Asp Gln Val Ala Ile His Glu Ala Met Glu Gln Gln Thr
                470                 475                 480

ata tcc atc act aaa gca gga gtg aag gct act ctg aac gcc cgg acg     1546
Ile Ser Ile Thr Lys Ala Gly Val Lys Ala Thr Leu Asn Ala Arg Thr
            485                 490                 495

tcc att ttg gca gca gca aac cca atc agt gga cac tat gac aga tca     1594
Ser Ile Leu Ala Ala Ala Asn Pro Ile Ser Gly His Tyr Asp Arg Ser
        500                 505                 510

aaa tca ttg aaa cag aat ata aat ttg tca gct ccc atc atg tcc cga     1642
Lys Ser Leu Lys Gln Asn Ile Asn Leu Ser Ala Pro Ile Met Ser Arg
    515                 520                 525

ttc gat ctc ttc ttt atc ctt gtg gat gaa tgt aat gag gtt aca gat     1690
Phe Asp Leu Phe Phe Ile Leu Val Asp Glu Cys Asn Glu Val Thr Asp
530                 535                 540                 545

tat gcc att gcc agg cgc ata gta gat ttg cat tca aga att gag gaa     1738
Tyr Ala Ile Ala Arg Arg Ile Val Asp Leu His Ser Arg Ile Glu Glu
                550                 555                 560

tca att gat cgt gtc tat tcc ctc gat gat atc aga aga tat ctt ctc     1786
Ser Ile Asp Arg Val Tyr Ser Leu Asp Asp Ile Arg Arg Tyr Leu Leu
            565                 570                 575

ttt gca aga cag ttt aaa ccc aag att tcc aaa gag tca gag gac ttc     1834
Phe Ala Arg Gln Phe Lys Pro Lys Ile Ser Lys Glu Ser Glu Asp Phe
        580                 585                 590

att gtg gag caa tat aaa cat ctc cgc cag aga gat ggt tct gga gtg     1882
Ile Val Glu Gln Tyr Lys His Leu Arg Gln Arg Asp Gly Ser Gly Val
    595                 600                 605

acc aag tct tca tgg agg att aca gtg cga cag ctt gag agc atg att     1930
Thr Lys Ser Ser Trp Arg Ile Thr Val Arg Gln Leu Glu Ser Met Ile
610                 615                 620                 625

cgt ctc tct gaa gct atg gct cgg atg cac tgc tgt gat gag gtc caa     1978
Arg Leu Ser Glu Ala Met Ala Arg Met His Cys Cys Asp Glu Val Gln
                630                 635                 640

cct aaa cat gtg aag gaa gct ttc cgg tta ctg aat aaa tca atc atc     2026
Pro Lys His Val Lys Glu Ala Phe Arg Leu Leu Asn Lys Ser Ile Ile
            645                 650                 655

cgt gtg gaa aca cct gat gtc aat cta gat caa gag gaa gag atc cag     2074
Arg Val Glu Thr Pro Asp Val Asn Leu Asp Gln Glu Glu Glu Ile Gln
        660                 665                 670

atg gag gta gat gag ggt gcc ggt ggc atc aat ggt cat gct gac agc     2122
Met Glu Val Asp Glu Gly Ala Gly Gly Ile Asn Gly His Ala Asp Ser
    675                 680                 685

cct gct cct gtg aac ggg atc aat ggc tac aat gaa gac ata aat caa     2170
Pro Ala Pro Val Asn Gly Ile Asn Gly Tyr Asn Glu Asp Ile Asn Gln
690                 695                 700                 705

gag tct gct ccc aaa gcc tcc tta agg ctg ggc ttc tct gag tac tgc     2218
Glu Ser Ala Pro Lys Ala Ser Leu Arg Leu Gly Phe Ser Glu Tyr Cys
                710                 715                 720
```

```
cga atc tct aac ctt att gtg ctt cac ctc aga aag gtg gaa gaa gaa    2266
Arg Ile Ser Asn Leu Ile Val Leu His Leu Arg Lys Val Glu Glu Glu
            725                 730                 735

gag gac gag tca gca tta aag agg agc gag ctt gtt aac tgg tac ttg    2314
Glu Asp Glu Ser Ala Leu Lys Arg Ser Glu Leu Val Asn Trp Tyr Leu
        740                 745                 750

aag gaa atc gaa tca gag ata gac tct gaa gaa gaa ctt ata aat aaa    2362
Lys Glu Ile Glu Ser Glu Ile Asp Ser Glu Glu Glu Leu Ile Asn Lys
    755                 760                 765

aaa aga atc ata gag aaa gtt att cat cga ctc aca cac tat gat cat    2410
Lys Arg Ile Ile Glu Lys Val Ile His Arg Leu Thr His Tyr Asp His
770                 775                 780                 785

gtt cta att gag ctc acc cag gct gga ttg aaa ggc tcc aca gag gga    2458
Val Leu Ile Glu Leu Thr Gln Ala Gly Leu Lys Gly Ser Thr Glu Gly
                790                 795                 800

agt gag agc tat gaa gaa gat ccc tac ttg gta gtt aac cct aac tac    2506
Ser Glu Ser Tyr Glu Glu Asp Pro Tyr Leu Val Val Asn Pro Asn Tyr
            805                 810                 815

ttg ctc gaa gat tga gatagtgaaa gtaactgacc agagctgagg aactgtggca    2561
Leu Leu Glu Asp  *
        820

cagcacctcg tggcctggag cctggctgga gctctgctag ggacagaagt gtttctggaa 2621
gtgatgcttc caggatttgt tttcagaaac aagaattgag ttgatggtcc tatgtgtcac 2681
attcatcaca ggtttcatac caacacaggc ttcagcactt cctttggtgt gtttcctgtc 2741
ccagtgaagt tggaaccaaa taatgtgtag tctctataac caataccttt gttttcatgt 2801
gtaagaaaag gcccattact tttaaggtat gtgctgtcct attgagcaaa taactttttt 2861
tcaattgcca gctactgctt ttattcatca aaataaaata acttgttctg aagttgtcta 2921
ttggatttct ttctactgta ccctgattat tacttccatc tacttctgaa tgtgagactt 2981
tccctttttg cttaacctgg agtgaagagg tagaactgtg gtattatgga tgaggtttct 3041
atgagaagga gtcattagag aactcatatg aaagctagag gccttagaga tgactttcca 3101
aggttaattc cagttttttt tttttttaag tttataaaag tttattatac tttttaaaa 3161
ttactcttta gtaatttatt ttacttctgt gtcctaaggg taatttctca ggattgtttt 3221
caaattgctt ttttagggga aataggtcat ttgctatatt acaagcaatc cccaaatttt 3281
atggtcttcc aggaaaagtt attaccgttt atgatactaa cagttcctga gacttagcta 3341
tgatcagtat gttcatgagg tggagcagtt cctgtgttgc agcttttaac aacagatggc 3401
attcattaaa tcacaaagta tgttaaaggt cacaaaagca aaataactgt ctgaggctaa 3461
ggcccacgtg ggacagtcta atacccatga gtactcaact tgccttgatg tctgagcttt 3521
ccagtgcaat gtgaatttga gcagccagaa atctattagt agaaagcaag acagattaat 3581
ataggttaaa acaatgattt aaatatgttt ctcccaataa ttatctcttt ccctggaatc 3641
aacttgtatg aaaccttgtc aaaatgtact ccacaagtat gtacaattaa gtattttaaa 3701
aataaatggc aaacattaaa aaaaaaaaaa aaaaaaaaaa aaa                   3744
```

<210> 16
<211> 821
<212> PRT
<213> Homo sapiens

<400> 16

```
Met Asp Leu Ala Ala Ala Ala Glu Pro Gly Ala Gly Ser Gln His Leu
 1              5                   10                  15
Glu Val Arg Asp Glu Val Ala Glu Lys Cys Gln Lys Leu Phe Leu Asp
            20                  25                  30
Phe Leu Glu Glu Phe Gln Ser Ser Asp Gly Glu Ile Lys Tyr Leu Gln
        35                  40                  45
Leu Ala Glu Glu Leu Ile Arg Pro Glu Arg Asn Thr Leu Val Val Ser
    50                  55                  60
Phe Val Asp Leu Glu Gln Phe Asn Gln Gln Leu Ser Thr Thr Ile Gln
65                  70                  75                  80
```

Glu Glu Phe Tyr Arg Val Tyr Pro Tyr Leu Cys Arg Ala Leu Lys Thr
85 90 95
Phe Val Lys Asp Arg Lys Glu Ile Pro Leu Ala Lys Asp Phe Tyr Val
100 105 110
Ala Phe Gln Asp Leu Pro Thr Arg His Lys Ile Arg Glu Leu Thr Ser
115 120 125
Ser Arg Ile Gly Leu Leu Thr Arg Ile Ser Gly Gln Val Val Arg Thr
130 135 140
His Pro Val His Pro Glu Leu Val Ser Gly Thr Phe Leu Cys Leu Asp
145 150 155 160
Cys Gln Thr Val Ile Arg Asp Val Glu Gln Gln Phe Lys Tyr Thr Gln
165 170 175
Pro Asn Ile Cys Arg Asn Pro Val Cys Ala Asn Arg Arg Arg Phe Leu
180 185 190
Leu Asp Thr Asn Lys Ser Arg Phe Val Asp Phe Gln Lys Val Arg Ile
195 200 205
Gln Glu Thr Gln Ala Glu Leu Pro Arg Gly Ser Ile Pro Arg Ser Leu
210 215 220
Glu Val Ile Leu Arg Ala Glu Ala Val Glu Ser Ala Gln Ala Gly Asp
225 230 235 240
Lys Cys Asp Phe Thr Gly Thr Leu Ile Val Val Pro Asp Val Ser Lys
245 250 255
Leu Ser Thr Pro Gly Ala Arg Ala Glu Thr Asn Ser Arg Val Ser Gly
260 265 270
Val Asp Gly Tyr Glu Thr Glu Gly Ile Arg Gly Leu Arg Ala Leu Gly
275 280 285
Val Arg Asp Leu Ser Tyr Arg Leu Val Phe Leu Ala Cys Cys Val Ala
290 295 300
Pro Thr Asn Pro Arg Phe Gly Gly Lys Glu Leu Arg Asp Glu Glu Gln
305 310 315 320
Thr Ala Glu Ser Ile Lys Asn Gln Met Thr Val Lys Glu Trp Glu Lys
325 330 335
Val Phe Glu Met Ser Gln Asp Lys Asn Leu Tyr His Asn Leu Cys Thr
340 345 350
Ser Leu Phe Pro Thr Ile His Gly Asn Asp Glu Val Lys Arg Gly Val
355 360 365
Leu Leu Met Leu Phe Gly Gly Val Pro Lys Thr Thr Gly Glu Gly Thr
370 375 380
Ser Leu Arg Gly Asp Ile Asn Val Cys Ile Val Gly Asp Pro Ser Thr
385 390 395 400
Ala Lys Ser Gln Phe Leu Lys His Val Glu Glu Phe Ser Pro Arg Ala
405 410 415
Val Tyr Thr Ser Gly Lys Ala Ser Ser Ala Ala Gly Leu Thr Ala Ala
420 425 430
Val Val Arg Asp Glu Glu Ser His Glu Phe Val Ile Glu Ala Gly Ala
435 440 445
Leu Met Leu Ala Asp Asn Gly Val Cys Cys Ile Asp Glu Phe Asp Lys
450 455 460
Met Asp Val Arg Asp Gln Val Ala Ile His Glu Ala Met Glu Gln Gln
465 470 475 480
Thr Ile Ser Ile Thr Lys Ala Gly Val Lys Ala Thr Leu Asn Ala Arg
485 490 495
Thr Ser Ile Leu Ala Ala Ala Asn Pro Ile Ser Gly His Tyr Asp Arg
500 505 510
Ser Lys Ser Leu Lys Gln Asn Ile Asn Leu Ser Ala Pro Ile Met Ser
515 520 525
Arg Phe Asp Leu Phe Phe Ile Leu Val Asp Glu Cys Asn Glu Val Thr
530 535 540
Asp Tyr Ala Ile Ala Arg Arg Ile Val Asp Leu His Ser Arg Ile Glu
545 550 555 560
Glu Ser Ile Asp Arg Val Tyr Ser Leu Asp Asp Ile Arg Arg Tyr Leu
565 570 575
Leu Phe Ala Arg Gln Phe Lys Pro Lys Ile Ser Lys Glu Ser Glu Asp
580 585 590
Phe Ile Val Glu Gln Tyr Lys His Leu Arg Gln Arg Asp Gly Ser Gly
595 600 605

```
Val Thr Lys Ser Ser Trp Arg Ile Thr Val Arg Gln Leu Glu Ser Met
    610                 615                 620
Ile Arg Leu Ser Glu Ala Met Ala Arg Met His Cys Cys Asp Glu Val
625                 630                 635                 640
Gln Pro Lys His Val Lys Glu Ala Phe Arg Leu Leu Asn Lys Ser Ile
                645                 650                 655
Ile Arg Val Glu Thr Pro Asp Val Asn Leu Asp Gln Glu Glu Glu Ile
            660                 665                 670
Gln Met Glu Val Asp Glu Gly Ala Gly Gly Ile Asn Gly His Ala Asp
        675                 680                 685
Ser Pro Ala Pro Val Asn Gly Ile Asn Gly Tyr Asn Glu Asp Ile Asn
    690                 695                 700
Gln Glu Ser Ala Pro Lys Ala Ser Leu Arg Leu Gly Phe Ser Glu Tyr
705                 710                 715                 720
Cys Arg Ile Ser Asn Leu Ile Val Leu His Leu Arg Lys Val Glu Glu
                725                 730                 735
Glu Glu Asp Glu Ser Ala Leu Lys Arg Ser Glu Leu Val Asn Trp Tyr
            740                 745                 750
Leu Lys Glu Ile Glu Ser Glu Ile Asp Ser Glu Glu Glu Leu Ile Asn
        755                 760                 765
Lys Lys Arg Ile Ile Glu Lys Val Ile His Arg Leu Thr His Tyr Asp
    770                 775                 780
His Val Leu Ile Glu Leu Thr Gln Ala Gly Leu Lys Gly Ser Thr Glu
785                 790                 795                 800
Gly Ser Glu Ser Tyr Glu Glu Asp Pro Tyr Leu Val Val Asn Pro Asn
                805                 810                 815
Tyr Leu Leu Glu Asp
            820
```

<210> 17
<211> 3371
<212> DNA
<213> Homo sapiens

<220>
<221> CDS
<222> (25)...(2712)

<400> 17

```
gcggaatcat cggaatcctt cacc atg gca tcc agc ccg gcc cag cgt cgg     51
                           Met Ala Ser Ser Pro Ala Gln Arg Arg
                            1               5

cga ggc aat gat cct ctc acc tcc agc cct ggc cga agc tcc cgg cgt    99
Arg Gly Asn Asp Pro Leu Thr Ser Ser Pro Gly Arg Ser Ser Arg Arg
 10                  15                  20                  25

act gat gcc ctc acc tcc agc cct ggc cgt gac ctt cca cca ttt gag   147
Thr Asp Ala Leu Thr Ser Ser Pro Gly Arg Asp Leu Pro Pro Phe Glu
                 30                  35                  40

gat gag tcc gag ggg ctc cta ggc aca gag ggg ccc ctg gag gaa gaa   195
Asp Glu Ser Glu Gly Leu Leu Gly Thr Glu Gly Pro Leu Glu Glu Glu
             45                  50                  55

gag gat gga gag gag ctc att gga gat ggc atg gaa agg gac tac cgc   243
Glu Asp Gly Glu Glu Leu Ile Gly Asp Gly Met Glu Arg Asp Tyr Arg
         60                  65                  70

gcc atc cca gag ctg gac gcc tat gag gcc gag gga ctg gct ctg gat   291
Ala Ile Pro Glu Leu Asp Ala Tyr Glu Ala Glu Gly Leu Ala Leu Asp
     75                  80                  85

gat gag gac gta gag gag ctg acg gcc agt cag agg gag gca gca gag   339
Asp Glu Asp Val Glu Glu Leu Thr Ala Ser Gln Arg Glu Ala Ala Glu
```

```
 90                  95                  100                 105

cgg gcc atg cgg cag cgt gac cgg gag gct ggc cgg ggc ctg ggc cgc    387
Arg Ala Met Arg Gln Arg Asp Arg Glu Ala Gly Arg Gly Leu Gly Arg
                110                 115                 120

atg cgc cgt ggg ctc ctg tat gac agc gat gag gag gac gag gag cgc    435
Met Arg Arg Gly Leu Leu Tyr Asp Ser Asp Glu Glu Asp Glu Glu Arg
            125                 130                 135

cct gcc cgc aag cgc cgc cag gtg gag cgg gcc acg gag gac ggc gag    483
Pro Ala Arg Lys Arg Arg Gln Val Glu Arg Ala Thr Glu Asp Gly Glu
        140                 145                 150

gag gac gag gag atg att gag agc atc gag aac ctg gag gat ctc aaa    531
Glu Asp Glu Glu Met Ile Glu Ser Ile Glu Asn Leu Glu Asp Leu Lys
    155                 160                 165

ggc cac tct gtg cgc gag tgg gtg agc atg gcg ggc ccc cgg ctg gag    579
Gly His Ser Val Arg Glu Trp Val Ser Met Ala Gly Pro Arg Leu Glu
170                 175                 180                 185

atc cac cac cgc ttc aag aac ttc ctg cgc act cac gtc gac agc cac    627
Ile His His Arg Phe Lys Asn Phe Leu Arg Thr His Val Asp Ser His
                190                 195                 200

ggc cac aac gtc ttc aag gag cgc atc agc gac atg tgc aaa gag aac    675
Gly His Asn Val Phe Lys Glu Arg Ile Ser Asp Met Cys Lys Glu Asn
            205                 210                 215

cgt gag agc ctg gtg gtg aac tat gag gac ttg gca gcc agg gag cac    723
Arg Glu Ser Leu Val Val Asn Tyr Glu Asp Leu Ala Ala Arg Glu His
        220                 225                 230

gtg ctg gcc tac ttc ctg cct gag gca ccg gcg gag ctg ctg cag atc    771
Val Leu Ala Tyr Phe Leu Pro Glu Ala Pro Ala Glu Leu Leu Gln Ile
    235                 240                 245

ttt gat gag gct gcc ctg gag gtg gta ctg gcc atg tac ccc aag tac    819
Phe Asp Glu Ala Ala Leu Glu Val Val Leu Ala Met Tyr Pro Lys Tyr
250                 255                 260                 265

gac cgc atc acc aac cac atc cat gtc cgc atc tcc cac ctg cct ctg    867
Asp Arg Ile Thr Asn His Ile His Val Arg Ile Ser His Leu Pro Leu
                270                 275                 280

gtg gag gag ctg cgc tcg ctg agg cag ctg cat ctg aac cag ctg atc    915
Val Glu Glu Leu Arg Ser Leu Arg Gln Leu His Leu Asn Gln Leu Ile
            285                 290                 295

cgc acc agt ggg gtg gtg acc agc tgc act ggc gtc ctg ccc cag ctc    963
Arg Thr Ser Gly Val Val Thr Ser Cys Thr Gly Val Leu Pro Gln Leu
        300                 305                 310

agc atg gtc aag tac aac tgc aac aag tgc aat ttc gtc ctg ggt cct    1011
Ser Met Val Lys Tyr Asn Cys Asn Lys Cys Asn Phe Val Leu Gly Pro
    315                 320                 325

ttc tgc cag tcc cag aac cag gag gtg aaa cca ggc tcc tgt cct gag    1059
Phe Cys Gln Ser Gln Asn Gln Glu Val Lys Pro Gly Ser Cys Pro Glu
330                 335                 340                 345

tgc cag tcg gcc ggc ccc ttt gag gtc aac atg gag gag acc atc tat    1107
Cys Gln Ser Ala Gly Pro Phe Glu Val Asn Met Glu Glu Thr Ile Tyr
                350                 355                 360
```

```
cag aac tac cag cgt atc cga atc cag gag agt cca ggc aaa gtg gcg    1155
Gln Asn Tyr Gln Arg Ile Arg Ile Gln Glu Ser Pro Gly Lys Val Ala
            365                 370                 375

gct ggc cgg ctg ccc cgc tcc aag gac gcc att ctc ctc gca gat ctg    1203
Ala Gly Arg Leu Pro Arg Ser Lys Asp Ala Ile Leu Leu Ala Asp Leu
        380                 385                 390

gtg gac agc tgc aac gca gga gac gag ata gag ctg act ggc atc tat    1251
Val Asp Ser Cys Asn Ala Gly Asp Glu Ile Glu Leu Thr Gly Ile Tyr
    395                 400                 405

cac aac aac tat gat ggc tcc ctc aac act gcc aat ggc ttc cct gtc    1299
His Asn Asn Tyr Asp Gly Ser Leu Asn Thr Ala Asn Gly Phe Pro Val
410                 415                 420                 425

ttt gcc act gtc atc cta gcc aac cac gtg gcc aag aag gac aac aag    1347
Phe Ala Thr Val Ile Leu Ala Asn His Val Ala Lys Lys Asp Asn Lys
                430                 435                 440

gtt gct gta ggg gaa ctg acc gat gaa gat gtg aag atg atc act agc    1395
Val Ala Val Gly Glu Leu Thr Asp Glu Asp Val Lys Met Ile Thr Ser
            445                 450                 455

ctc tcc aag gat cag cag atc gga gag aag atc ttt gcc agc att gct    1443
Leu Ser Lys Asp Gln Gln Ile Gly Glu Lys Ile Phe Ala Ser Ile Ala
        460                 465                 470

cct tcc atc tat ggt cat gaa gac atc aag aga ggc ctg gct ctg gcc    1491
Pro Ser Ile Tyr Gly His Glu Asp Ile Lys Arg Gly Leu Ala Leu Ala
    475                 480                 485

ctg ttc gga ggg gag ccc aaa aac cca ggt ggc aag cac aag gta cgt    1539
Leu Phe Gly Gly Glu Pro Lys Asn Pro Gly Gly Lys His Lys Val Arg
490                 495                 500                 505

ggt gat atc aac gtg ctc ttg tgc gga gac cct ggc aca gcg aag tcg    1587
Gly Asp Ile Asn Val Leu Leu Cys Gly Asp Pro Gly Thr Ala Lys Ser
                510                 515                 520

cag ttt ctc aag tat att gag aaa gtg tcc agc cga gcc atc ttc acc    1635
Gln Phe Leu Lys Tyr Ile Glu Lys Val Ser Ser Arg Ala Ile Phe Thr
            525                 530                 535

act ggc cag ggg gcg tcg gct gtg ggc ctc acg gcg tat gtc cag cgg    1683
Thr Gly Gln Gly Ala Ser Ala Val Gly Leu Thr Ala Tyr Val Gln Arg
        540                 545                 550

cac cct gtc agc agg gag tgg acc ttg gag gct ggg gcc ctg gtt ctg    1731
His Pro Val Ser Arg Glu Trp Thr Leu Glu Ala Gly Ala Leu Val Leu
    555                 560                 565

gct gac cga gga gtg tgt ctc att gat gaa ttt gac aag atg aat gac    1779
Ala Asp Arg Gly Val Cys Leu Ile Asp Glu Phe Asp Lys Met Asn Asp
570                 575                 580                 585

cag gac aga acc agc atc cat gag gcc atg gag caa cag agc atc tcc    1827
Gln Asp Arg Thr Ser Ile His Glu Ala Met Glu Gln Gln Ser Ile Ser
                590                 595                 600

atc tcg aag gct ggc atc gtc acc tcc ctg cag gct cgc tgc acg gtc    1875
Ile Ser Lys Ala Gly Ile Val Thr Ser Leu Gln Ala Arg Cys Thr Val
            605                 610                 615

att gct gcc gcc aac ccc ata gga ggg cgc tac gac ccc tcg ctg act    1923
Ile Ala Ala Ala Asn Pro Ile Gly Gly Arg Tyr Asp Pro Ser Leu Thr
```

620 625 630

ttc tct gag aac gtg gac ctc aca gag ccc atc atc tca cgc ttt gac 1971
Phe Ser Glu Asn Val Asp Leu Thr Glu Pro Ile Ile Ser Arg Phe Asp
635 640 645

atc ctg tgt gtg gtg agg gac acc gtg gac cca gtc cag gac gag atg 2019
Ile Leu Cys Val Val Arg Asp Thr Val Asp Pro Val Gln Asp Glu Met
650 655 660 665

ctg gcc cgc ttc gtg gtg ggc agc cac gtc aga cac cac ccc agc aac 2067
Leu Ala Arg Phe Val Val Gly Ser His Val Arg His His Pro Ser Asn
670 675 680

aag gag gag gag ggg ctg gcc aat ggc agc gct gct gag ccc gcc atg 2115
Lys Glu Glu Glu Gly Leu Ala Asn Gly Ser Ala Ala Glu Pro Ala Met
685 690 695

ccc aac acg tat ggc gtg gag ccc ctg ccc cag gag gtc ctg aag aag 2163
Pro Asn Thr Tyr Gly Val Glu Pro Leu Pro Gln Glu Val Leu Lys Lys
700 705 710

tac atc atc tac gcc aag gag agg gtc cac ccg aag ctc aac cag atg 2211
Tyr Ile Ile Tyr Ala Lys Glu Arg Val His Pro Lys Leu Asn Gln Met
715 720 725

gac cag gac aag gtg gcc aag atg tac agt gac ctg agg aaa gaa tct 2259
Asp Gln Asp Lys Val Ala Lys Met Tyr Ser Asp Leu Arg Lys Glu Ser
730 735 740 745

atg gcg aca ggc agc atc ccc att acg gtg cgg cac atc gag tcc atg 2307
Met Ala Thr Gly Ser Ile Pro Ile Thr Val Arg His Ile Glu Ser Met
750 755 760

atc cgc atg gcg gag gcc cac gcg cgc atc cat ctg cgg gac tat gtg 2355
Ile Arg Met Ala Glu Ala His Ala Arg Ile His Leu Arg Asp Tyr Val
765 770 775

atc gaa gac gac gtc aac atg gcc atc cgc gtg atg ctg gag agc ttc 2403
Ile Glu Asp Asp Val Asn Met Ala Ile Arg Val Met Leu Glu Ser Phe
780 785 790

ata gac aca cag aag ttc agc gtc atg cgc agc atg cgc aag act ttt 2451
Ile Asp Thr Gln Lys Phe Ser Val Met Arg Ser Met Arg Lys Thr Phe
795 800 805

gcc cgc tac ctt tca ttc cgg cgt gac aac aat gag ctg ttg ctc ttc 2499
Ala Arg Tyr Leu Ser Phe Arg Arg Asp Asn Asn Glu Leu Leu Leu Phe
810 815 820 825

ata ctg aag cag tta gtg gca gag cag gtg aca tat cag cgc aac cgc 2547
Ile Leu Lys Gln Leu Val Ala Glu Gln Val Thr Tyr Gln Arg Asn Arg
830 835 840

ttt ggg gcc cag cag gac act att gag gtc cct gag aag gac ttg gtg 2595
Phe Gly Ala Gln Gln Asp Thr Ile Glu Val Pro Glu Lys Asp Leu Val
845 850 855

gat aag gct cgt cag atc aac atc cac aac ctc tct gca ttt tat gac 2643
Asp Lys Ala Arg Gln Ile Asn Ile His Asn Leu Ser Ala Phe Tyr Asp
860 865 870

agt gag ctc ttc agg atg aac aag ttc agc cac gac ctg aaa agg aaa 2691
Ser Glu Leu Phe Arg Met Asn Lys Phe Ser His Asp Leu Lys Arg Lys
875 880 885

```
atg atc ctg cag cag ttc tga ggccctatgc catccataag gattccttgg      2742
Met Ile Leu Gln Gln Phe  *
890                 895

gattctggtt tggggtggtc agtgccctct gtgctttatg gacacaaaac cagagcactt 2802
gatgaactcg gggtactagg gtcagggctt atagcaggat gtctggctgc acctggcatg 2862
actgtttgtt tctccaagcc tgctttgtgc ttctcacctt tgggtgggat gccttgccag 2922
tgtgtcttac ttggttgctg aacatcttgc cacctccgag tgctttgtct ccactcagta 2982
ccttggatca gagctgctga gttcaggatg cctgcgtgtg gtttaggtgt tagccttctt 3042
acatggatgt caggagagct gctgccctct tggcgtgagt tgcgtattca ggctgctttt 3102
gctgcctttg gccagagagc tggttgaaga tgtttgtaat cgttttcagt ctcctgcagg 3162
tttctgtgcc cctgtggtgg aagaggcacg acagtgccag cgcagcgttc tgggctcctc 3222
agtcgcaggg gtgggatgtg agtcatgcgg attatccact cgccacagtt atcagctgcc 3282
attgctccct gtctgtttcc ccactctctt atttgtgcat tcggtttggt ttctgtagtt 3342
ttaattttta ataaagttga ataaaatat                                   3371
```

<210> 18
<211> 895
<212> PRT
<213> Homo sapiens

<400> 18

```
Met Ala Ser Ser Pro Ala Gln Arg Arg Arg Gly Asn Asp Pro Leu Thr
 1               5                  10                  15
Ser Ser Pro Gly Arg Ser Ser Arg Arg Thr Asp Ala Leu Thr Ser Ser
            20                  25                  30
Pro Gly Arg Asp Leu Pro Pro Phe Glu Asp Glu Ser Glu Gly Leu Leu
        35                  40                  45
Gly Thr Glu Gly Pro Leu Glu Glu Glu Glu Asp Gly Glu Glu Leu Ile
    50                  55                  60
Gly Asp Gly Met Glu Arg Asp Tyr Arg Ala Ile Pro Glu Leu Asp Ala
65                  70                  75                  80
Tyr Glu Ala Glu Gly Leu Ala Leu Asp Asp Glu Asp Val Glu Glu Leu
                85                  90                  95
Thr Ala Ser Gln Arg Glu Ala Ala Glu Arg Ala Met Arg Gln Arg Asp
            100                 105                 110
Arg Glu Ala Gly Arg Gly Leu Gly Arg Met Arg Arg Gly Leu Leu Tyr
        115                 120                 125
Asp Ser Asp Glu Glu Asp Glu Glu Arg Pro Ala Arg Lys Arg Arg Gln
    130                 135                 140
Val Glu Arg Ala Thr Glu Asp Gly Glu Glu Asp Glu Glu Met Ile Glu
145                 150                 155                 160
Ser Ile Glu Asn Leu Glu Asp Leu Lys Gly His Ser Val Arg Glu Trp
                165                 170                 175
Val Ser Met Ala Gly Pro Arg Leu Glu Ile His His Arg Phe Lys Asn
            180                 185                 190
Phe Leu Arg Thr His Val Asp Ser His Gly His Asn Val Phe Lys Glu
        195                 200                 205
Arg Ile Ser Asp Met Cys Lys Glu Asn Arg Glu Ser Leu Val Val Asn
    210                 215                 220
Tyr Glu Asp Leu Ala Ala Arg Glu His Val Leu Ala Tyr Phe Leu Pro
225                 230                 235                 240
Glu Ala Pro Ala Glu Leu Leu Gln Ile Phe Asp Glu Ala Ala Leu Glu
                245                 250                 255
Val Val Leu Ala Met Tyr Pro Lys Tyr Asp Arg Ile Thr Asn His Ile
            260                 265                 270
His Val Arg Ile Ser His Leu Pro Leu Val Glu Glu Leu Arg Ser Leu
        275                 280                 285
Arg Gln Leu His Leu Asn Gln Leu Ile Arg Thr Ser Gly Val Val Thr
    290                 295                 300
Ser Cys Thr Gly Val Leu Pro Gln Leu Ser Met Val Lys Tyr Asn Cys
305                 310                 315                 320
Asn Lys Cys Asn Phe Val Leu Gly Pro Phe Cys Gln Ser Gln Asn Gln
                325                 330                 335
Glu Val Lys Pro Gly Ser Cys Pro Glu Cys Gln Ser Ala Gly Pro Phe
            340                 345                 350
```

Glu Val Asn Met Glu Glu Thr Ile Tyr Gln Asn Tyr Gln Arg Ile Arg
355 360 365
Ile Gln Glu Ser Pro Gly Lys Val Ala Ala Gly Arg Leu Pro Arg Ser
370 375 380
Lys Asp Ala Ile Leu Leu Ala Asp Leu Val Asp Ser Cys Asn Ala Gly
385 390 395 400
Asp Glu Ile Glu Leu Thr Gly Ile Tyr His Asn Asn Tyr Asp Gly Ser
405 410 415
Leu Asn Thr Ala Asn Gly Phe Pro Val Phe Ala Thr Val Ile Leu Ala
420 425 430
Asn His Val Ala Lys Lys Asp Asn Lys Val Ala Val Gly Glu Leu Thr
435 440 445
Asp Glu Asp Val Lys Met Ile Thr Ser Leu Ser Lys Asp Gln Gln Ile
450 455 460
Gly Glu Lys Ile Phe Ala Ser Ile Ala Pro Ser Ile Tyr Gly His Glu
465 470 475 480
Asp Ile Lys Arg Gly Leu Ala Leu Ala Leu Phe Gly Gly Glu Pro Lys
485 490 495
Asn Pro Gly Gly Lys His Lys Val Arg Gly Asp Ile Asn Val Leu Leu
500 505 510
Cys Gly Asp Pro Gly Thr Ala Lys Ser Gln Phe Leu Lys Tyr Ile Glu
515 520 525
Lys Val Ser Ser Arg Ala Ile Phe Thr Thr Gly Gln Gly Ala Ser Ala
530 535 540
Val Gly Leu Thr Ala Tyr Val Gln Arg His Pro Val Ser Arg Glu Trp
545 550 555 560
Thr Leu Glu Ala Gly Ala Leu Val Leu Ala Asp Arg Gly Val Cys Leu
565 570 575
Ile Asp Glu Phe Asp Lys Met Asn Asp Gln Asp Arg Thr Ser Ile His
580 585 590
Glu Ala Met Glu Gln Gln Ser Ile Ser Ile Ser Lys Ala Gly Ile Val
595 600 605
Thr Ser Leu Gln Ala Arg Cys Thr Val Ile Ala Ala Ala Asn Pro Ile
610 615 620
Gly Gly Arg Tyr Asp Pro Ser Leu Thr Phe Ser Glu Asn Val Asp Leu
625 630 635 640
Thr Glu Pro Ile Ile Ser Arg Phe Asp Ile Leu Cys Val Val Arg Asp
645 650 655
Thr Val Asp Pro Val Gln Asp Glu Met Leu Ala Arg Phe Val Val Gly
660 665 670
Ser His Val Arg His His Pro Ser Asn Lys Glu Glu Glu Gly Leu Ala
675 680 685
Asn Gly Ser Ala Ala Glu Pro Ala Met Pro Asn Thr Tyr Gly Val Glu
690 695 700
Pro Leu Pro Gln Glu Val Leu Lys Lys Tyr Ile Ile Tyr Ala Lys Glu
705 710 715 720
Arg Val His Pro Lys Leu Asn Gln Met Asp Gln Asp Lys Val Ala Lys
725 730 735
Met Tyr Ser Asp Leu Arg Lys Glu Ser Met Ala Thr Gly Ser Ile Pro
740 745 750
Ile Thr Val Arg His Ile Glu Ser Met Ile Arg Met Ala Glu Ala His
755 760 765
Ala Arg Ile His Leu Arg Asp Tyr Val Ile Glu Asp Asp Val Asn Met
770 775 780
Ala Ile Arg Val Met Leu Glu Ser Phe Ile Asp Thr Gln Lys Phe Ser
785 790 795 800
Val Met Arg Ser Met Arg Lys Thr Phe Ala Arg Tyr Leu Ser Phe Arg
805 810 815
Arg Asp Asn Asn Glu Leu Leu Leu Phe Ile Leu Lys Gln Leu Val Ala
820 825 830
Glu Gln Val Thr Tyr Gln Arg Asn Arg Phe Gly Ala Gln Gln Asp Thr
835 840 845
Ile Glu Val Pro Glu Lys Asp Leu Val Asp Lys Ala Arg Gln Ile Asn
850 855 860
Ile His Asn Leu Ser Ala Phe Tyr Asp Ser Glu Leu Phe Arg Met Asn
865 870 875 880

Lys Phe Ser His Asp Leu Lys Arg Lys Met Ile Leu Gln Gln Phe
885 890 895

<210> 19
<211> 1721
<212> DNA
<213> Homo sapiens

<220>
<221> CDS
<222> (58)...(1605)

<400> 19

```
gaattccctg gctgcttgaa tctgttctgc cccctcccca cccatttcac caccacc atg 60
                                                               Met
                                                                1

aca ccg ggc acc cag tct cct ttc ttc ctg ctg ctg ctc ctc aca gtg   108
Thr Pro Gly Thr Gln Ser Pro Phe Phe Leu Leu Leu Leu Leu Thr Val
            5                   10                  15

ctt aca gtt gtt aca ggt tct ggt cat gca agc tct acc cca ggt gga   156
Leu Thr Val Val Thr Gly Ser Gly His Ala Ser Ser Thr Pro Gly Gly
        20                  25                  30

gaa aag gag act tcg gct acc cag aga agt tca gtg ccc agc tct act   204
Glu Lys Glu Thr Ser Ala Thr Gln Arg Ser Ser Val Pro Ser Ser Thr
    35                  40                  45

gag aag aat gct gtg agt atg acc agc agc gta ctc tcc agc cac agc   252
Glu Lys Asn Ala Val Ser Met Thr Ser Ser Val Leu Ser Ser His Ser
50                  55                  60                  65

ccc ggt tca ggc tcc tcc acc act cag gga cag gat gtc act ctg gcc   300
Pro Gly Ser Gly Ser Ser Thr Thr Gln Gly Gln Asp Val Thr Leu Ala
                70                  75                  80

ccg gcc acg gaa cca gct tca ggt tca gct gcc acc tgg gga cag gat   348
Pro Ala Thr Glu Pro Ala Ser Gly Ser Ala Ala Thr Trp Gly Gln Asp
            85                  90                  95

gtc acc tcg gtc cca gtc acc agg cca gcc ctg ggc tcc acc acc ccg   396
Val Thr Ser Val Pro Val Thr Arg Pro Ala Leu Gly Ser Thr Thr Pro
        100                 105                 110

cca gcc cac gat gtc acc tca gcc ccg gac aac aag cca gcc ccg ggc   444
Pro Ala His Asp Val Thr Ser Ala Pro Asp Asn Lys Pro Ala Pro Gly
    115                 120                 125

tcc acc gcc ccc cca gcc cac ggt gtc acc tcg gcc ccg gac acc agg   492
Ser Thr Ala Pro Pro Ala His Gly Val Thr Ser Ala Pro Asp Thr Arg
130                 135                 140                 145

ccg ccc ccg ggc tcc acc gcc ccc cca gcc cac ggt gtc acc tcg gcc   540
Pro Pro Pro Gly Ser Thr Ala Pro Pro Ala His Gly Val Thr Ser Ala
                150                 155                 160

ccg gac acc agg ccg ccc ccg ggc tcc acc gcg ccc gca gcc cac ggt   588
Pro Asp Thr Arg Pro Pro Pro Gly Ser Thr Ala Pro Ala Ala His Gly
            165                 170                 175

gtc acc tcg gcc ccg gac acc agg ccg gcc ccg ggc tcc acc gcc ccc   636
Val Thr Ser Ala Pro Asp Thr Arg Pro Ala Pro Gly Ser Thr Ala Pro
        180                 185                 190
```

```
cca gcc cat ggt gtc acc tcg gcc ccg gac aac agg ccc gcc ttg gcg      684
Pro Ala His Gly Val Thr Ser Ala Pro Asp Asn Arg Pro Ala Leu Ala
    195                 200                 205

tcc acc gcc cct cca gtc cac aat gtc acc tcg gcc tca ggc tct gca      732
Ser Thr Ala Pro Pro Val His Asn Val Thr Ser Ala Ser Gly Ser Ala
210                 215                 220                 225

tca ggc tca gct tct act ctg gtg cac aac ggc acc tct gcc agg gct      780
Ser Gly Ser Ala Ser Thr Leu Val His Asn Gly Thr Ser Ala Arg Ala
                230                 235                 240

acc aca acc cca gcc agc aag agc act cca ttc tca att ccc agc cac      828
Thr Thr Thr Pro Ala Ser Lys Ser Thr Pro Phe Ser Ile Pro Ser His
            245                 250                 255

cac tct gat act cct acc acc ctt gcc agc cat agc acc aag act gat      876
His Ser Asp Thr Pro Thr Thr Leu Ala Ser His Ser Thr Lys Thr Asp
        260                 265                 270

gcc agt agc act cac cat agc acg gta cct cct ctc acc tcc tcc aat      924
Ala Ser Ser Thr His His Ser Thr Val Pro Pro Leu Thr Ser Ser Asn
    275                 280                 285

cac agc act tct ccc cag ttg tct act ggg gtc tct ttc ttt ttc ctg      972
His Ser Thr Ser Pro Gln Leu Ser Thr Gly Val Ser Phe Phe Phe Leu
290                 295                 300                 305

tct ttt cac att tca aac ctc cag ttt aat tcc tct ctg gaa gat ccc     1020
Ser Phe His Ile Ser Asn Leu Gln Phe Asn Ser Ser Leu Glu Asp Pro
                310                 315                 320

agc acc gac tac tac caa gag ctg cag aga gac att tct gaa atg ttt     1068
Ser Thr Asp Tyr Tyr Gln Glu Leu Gln Arg Asp Ile Ser Glu Met Phe
            325                 330                 335

ttg cag att tat aaa caa ggg ggt ttt ctg ggc ctc tcc aat att aag     1116
Leu Gln Ile Tyr Lys Gln Gly Gly Phe Leu Gly Leu Ser Asn Ile Lys
        340                 345                 350

ttc agg cca gga tct gtg gtg gta caa ttg act ctg gcc ttc cga gaa     1164
Phe Arg Pro Gly Ser Val Val Val Gln Leu Thr Leu Ala Phe Arg Glu
    355                 360                 365

ggt acc atc aat gtc cac gac gtg gag aca cag ttc aat cag tat aaa     1212
Gly Thr Ile Asn Val His Asp Val Glu Thr Gln Phe Asn Gln Tyr Lys
370                 375                 380                 385

acg gaa gca gcc tct cga tat aac ctg acg atc tca gac gtc agc gtg     1260
Thr Glu Ala Ala Ser Arg Tyr Asn Leu Thr Ile Ser Asp Val Ser Val
                390                 395                 400

agt gat gtg cca ttt cct ttc tct gcc cag tct ggg gct ggg gtg cca     1308
Ser Asp Val Pro Phe Pro Phe Ser Ala Gln Ser Gly Ala Gly Val Pro
            405                 410                 415

ggc tgg ggc atc gcg ctg ctg gtg ctg gtc tgt gtt ctg gtt gcg ctg     1356
Gly Trp Gly Ile Ala Leu Leu Val Leu Val Cys Val Leu Val Ala Leu
        420                 425                 430

gcc att gtc tat ctc att gcc ttg gct gtc tgt cag tgc cgc cga aag     1404
Ala Ile Val Tyr Leu Ile Ala Leu Ala Val Cys Gln Cys Arg Arg Lys
    435                 440                 445

aac tac ggg cag ctg gac atc ttt cca gcc cgg gat acc tac cat cct     1452
Asn Tyr Gly Gln Leu Asp Ile Phe Pro Ala Arg Asp Thr Tyr His Pro
```

```
450                 455                 460                 465

atg agc gag tac ccc acc tac cac acc cat ggg cgc tat gtg ccc cct    1500
Met Ser Glu Tyr Pro Thr Tyr His Thr His Gly Arg Tyr Val Pro Pro
                470                 475                 480

agc agt acc gat cgt agc ccc tat gag aag gtt tct gca ggt aat ggt    1548
Ser Ser Thr Asp Arg Ser Pro Tyr Glu Lys Val Ser Ala Gly Asn Gly
            485                 490                 495

ggc agc agc ctc tct tac aca aac cca gca gtg gca gcc act tct gcc    1596
Gly Ser Ser Leu Ser Tyr Thr Asn Pro Ala Val Ala Ala Thr Ser Ala
        500                 505                 510

aac ttg tag gggcacgtcg ccctctgagc tgagtggcca gccagtgcca           1645
Asn Leu  *
    515

ttccactcca ctcagggctc tctgggccag tcctcctggg agcccccacc acaacacttc 1705
ccaggcatgg aattcc                                                 1721
```

<210> 20
<211> 515
<212> PRT
<213> Homo sapiens

<400> 20

```
Met Thr Pro Gly Thr Gln Ser Pro Phe Phe Leu Leu Leu Leu Leu Thr
 1               5                  10                  15
Val Leu Thr Val Val Thr Gly Ser Gly His Ala Ser Ser Thr Pro Gly
            20                  25                  30
Gly Glu Lys Glu Thr Ser Ala Thr Gln Arg Ser Ser Val Pro Ser Ser
        35                  40                  45
Thr Glu Lys Asn Ala Val Ser Met Thr Ser Ser Val Leu Ser Ser His
    50                  55                  60
Ser Pro Gly Ser Gly Ser Ser Thr Thr Gln Gly Gln Asp Val Thr Leu
65                  70                  75                  80
Ala Pro Ala Thr Glu Pro Ala Ser Gly Ser Ala Ala Thr Trp Gly Gln
                85                  90                  95
Asp Val Thr Ser Val Pro Val Thr Arg Pro Ala Leu Gly Ser Thr Thr
            100                 105                 110
Pro Pro Ala His Asp Val Thr Ser Ala Pro Asp Asn Lys Pro Ala Pro
        115                 120                 125
Gly Ser Thr Ala Pro Pro Ala His Gly Val Thr Ser Ala Pro Asp Thr
    130                 135                 140
Arg Pro Pro Pro Gly Ser Thr Ala Pro Pro Ala His Gly Val Thr Ser
145                 150                 155                 160
Ala Pro Asp Thr Arg Pro Pro Pro Gly Ser Thr Ala Pro Ala Ala His
                165                 170                 175
Gly Val Thr Ser Ala Pro Asp Thr Arg Pro Ala Pro Gly Ser Thr Ala
            180                 185                 190
Pro Pro Ala His Gly Val Thr Ser Ala Pro Asp Asn Arg Pro Ala Leu
        195                 200                 205
Ala Ser Thr Ala Pro Pro Val His Asn Val Thr Ser Ala Ser Gly Ser
    210                 215                 220
Ala Ser Gly Ser Ala Ser Thr Leu Val His Asn Gly Thr Ser Ala Arg
225                 230                 235                 240
Ala Thr Thr Thr Pro Ala Ser Lys Ser Thr Pro Phe Ser Ile Pro Ser
                245                 250                 255
His His Ser Asp Thr Pro Thr Thr Leu Ala Ser His Ser Thr Lys Thr
            260                 265                 270
Asp Ala Ser Ser Thr His His Ser Thr Val Pro Pro Leu Thr Ser Ser
        275                 280                 285
Asn His Ser Thr Ser Pro Gln Leu Ser Thr Gly Val Ser Phe Phe Phe
    290                 295                 300
Leu Ser Phe His Ile Ser Asn Leu Gln Phe Asn Ser Ser Leu Glu Asp
```

```
305                 310                 315                 320
Pro Ser Thr Asp Tyr Tyr Gln Glu Leu Gln Arg Asp Ile Ser Glu Met
                325                 330                 335
Phe Leu Gln Ile Tyr Lys Gln Gly Gly Phe Leu Gly Leu Ser Asn Ile
            340                 345                 350
Lys Phe Arg Pro Gly Ser Val Val Val Gln Leu Thr Leu Ala Phe Arg
        355                 360                 365
Glu Gly Thr Ile Asn Val His Asp Val Glu Thr Gln Phe Asn Gln Tyr
    370                 375                 380
Lys Thr Glu Ala Ala Ser Arg Tyr Asn Leu Thr Ile Ser Asp Val Ser
385                 390                 395                 400
Val Ser Asp Val Pro Phe Pro Phe Ser Ala Gln Ser Gly Ala Gly Val
                405                 410                 415
Pro Gly Trp Gly Ile Ala Leu Leu Val Leu Val Cys Val Leu Val Ala
            420                 425                 430
Leu Ala Ile Val Tyr Leu Ile Ala Leu Ala Val Cys Gln Cys Arg Arg
        435                 440                 445
Lys Asn Tyr Gly Gln Leu Asp Ile Phe Pro Ala Arg Asp Thr Tyr His
    450                 455                 460
Pro Met Ser Glu Tyr Pro Thr Tyr His Thr His Gly Arg Tyr Val Pro
465                 470                 475                 480
Pro Ser Ser Thr Asp Arg Ser Pro Tyr Glu Lys Val Ser Ala Gly Asn
                485                 490                 495
Gly Gly Ser Ser Leu Ser Tyr Thr Asn Pro Ala Val Ala Ala Thr Ser
            500                 505                 510
Ala Asn Leu
        515
```

<210> 21
<211> 1061
<212> DNA
<213> Homo sapiens

<220>
<221> CDS
<222> (189)...(758)

<400> 21

```
tgccctgcgc ccgcaacccg agccgcaccc gccgcggacg gagcccatgc gcggggcgaa 60
ccgcgcgccc ccgcccccgc cccgccccgg cctcggcccc ggccctggcc ccgggggcag 120
tcgcgcctgt gaacggtggg gcaggagacc ctgtaggagg accccgggcc gcaggcccct 180
gaggagcg atg acg gaa tat aag ctg gtg gtg gtg ggc gcc ggc ggt gtg  230
         Met Thr Glu Tyr Lys Leu Val Val Val Gly Ala Gly Gly Val
         1               5                   10

ggc aag agt gcg ctg acc atc cag ctg atc cag aac cat ttt gtg gac   278
Gly Lys Ser Ala Leu Thr Ile Gln Leu Ile Gln Asn His Phe Val Asp
15                  20                  25                  30

gaa tac gac ccc act ata gag gat tcc tac cgg aag cag gtg gtc att   326
Glu Tyr Asp Pro Thr Ile Glu Asp Ser Tyr Arg Lys Gln Val Val Ile
                35                  40                  45

gat ggg gag acg tgc ctg ttg gac atc ctg gat acc gcc ggc cag gag   374
Asp Gly Glu Thr Cys Leu Leu Asp Ile Leu Asp Thr Ala Gly Gln Glu
            50                  55                  60

gag tac agc gcc atg cgg gac cag tac atg cgc acc ggg gag ggc ttc   422
Glu Tyr Ser Ala Met Arg Asp Gln Tyr Met Arg Thr Gly Glu Gly Phe
        65                  70                  75

ctg tgt gtg ttt gcc atc aac aac acc aag tct ttt gag gac atc cac   470
Leu Cys Val Phe Ala Ile Asn Asn Thr Lys Ser Phe Glu Asp Ile His
    80                  85                  90

cag tac agg gag cag atc aaa cgg gtg aag gac tcg gat gac gtg ccc   518
Gln Tyr Arg Glu Gln Ile Lys Arg Val Lys Asp Ser Asp Asp Val Pro
95                  100                 105                 110

atg gtg ctg gtg ggg aac aag tgt gac ctg gct gca cgc act gtg gaa   566
Met Val Leu Val Gly Asn Lys Cys Asp Leu Ala Ala Arg Thr Val Glu
                115                 120                 125

tct cgg cag gct cag gac ctc gcc cga agc tac ggc atc ccc tac atc   614
Ser Arg Gln Ala Gln Asp Leu Ala Arg Ser Tyr Gly Ile Pro Tyr Ile
            130                 135                 140

gag acc tcg gcc aag acc cgg cag gga gtg gag gat gcc ttc tac acg   662
Glu Thr Ser Ala Lys Thr Arg Gln Gly Val Glu Asp Ala Phe Tyr Thr
        145                 150                 155

ttg gtg cgt gag atc cgg cag cac aag ctg cgg aag ctg aac cct cct   710
Leu Val Arg Glu Ile Arg Gln His Lys Leu Arg Lys Leu Asn Pro Pro
    160                 165                 170

gat gag agt ggc ccc ggc tgc atg agc tgc aag tgt gtg ctc tcc tga   758
Asp Glu Ser Gly Pro Gly Cys Met Ser Cys Lys Cys Val Leu Ser  *
175                 180                 185

cgcagcacaa gctcaggaca tggaggtgcc ggatgcagga aggaggtgca gacggaagga 818
ggaggaagga aggacggaag caaggaagga aggaagggct gctggagccc agtcaccccg 878
ggaccgtggg ccgaggtgac tgcagaccct cccagggagg ctgtgcacag actgtcttga 938
acatcccaaa tgccaccgga accccagccc ttagctcccc tcccaggcct ctgtgggccc 998
ttgtcgggca cagatgggat cacagtaaat tattggatgg tcttgaaaaa aaaaaaaaaa 1058
aaa                                                               1061
```

<210> 22
<211> 189
<212> PRT
<213> Homo sapiens

<400> 22

```
Met Thr Glu Tyr Lys Leu Val Val Val Gly Ala Gly Gly Val Gly Lys
 1               5                  10                  15
Ser Ala Leu Thr Ile Gln Leu Ile Gln Asn His Phe Val Asp Glu Tyr
            20                  25                  30
Asp Pro Thr Ile Glu Asp Ser Tyr Arg Lys Gln Val Val Ile Asp Gly
        35                  40                  45
Glu Thr Cys Leu Leu Asp Ile Leu Asp Thr Ala Gly Gln Glu Glu Tyr
    50                  55                  60
Ser Ala Met Arg Asp Gln Tyr Met Arg Thr Gly Glu Gly Phe Leu Cys
65                  70                  75                  80
Val Phe Ala Ile Asn Asn Thr Lys Ser Phe Glu Asp Ile His Gln Tyr
                85                  90                  95
Arg Glu Gln Ile Lys Arg Val Lys Asp Ser Asp Asp Val Pro Met Val
            100                 105                 110
Leu Val Gly Asn Lys Cys Asp Leu Ala Ala Arg Thr Val Glu Ser Arg
        115                 120                 125
Gln Ala Gln Asp Leu Ala Arg Ser Tyr Gly Ile Pro Tyr Ile Glu Thr
    130                 135                 140
Ser Ala Lys Thr Arg Gln Gly Val Glu Asp Ala Phe Tyr Thr Leu Val
145                 150                 155                 160
Arg Glu Ile Arg Gln His Lys Leu Arg Lys Leu Asn Pro Pro Asp Glu
                165                 170                 175
Ser Gly Pro Gly Cys Met Ser Cys Lys Cys Val Leu Ser
            180                 185
```

<210> 23
<211> 4145
<212> DNA
<213> Homo sapiens

<220>
<221> CDS
<222> (450)...(2060)

<400> 23

```
caaacaagtg cggccatttc accagcccag gctggcttct gctgttgact ggctgtggca 60
cctcaagcag cccctttccc ctctagcctc agtttatcac cgcaagagct accattcatc 120
tagcacaacc tgaccatcct cacactggtc agttccaacc ttcccaggaa tcttctgtgg 180
ccatgttcac tccggtttta cagaacagag aacagaagct cagagaagtg aagcaacttg 240
cccagctatg agagacagag ccaggatttg aaaccagatg aggacgctga ggcccagaga 300
gggaaagcca cttgcctagg gacacacagc ggggagaggt ggagcagggc ctctatttcg 360
agacccctga ctccacacct ggtgtttgtg ccaagacccc aggctgcctc ccaggtcctc 420
tgggacagcc cctgccttct accaggacc atg ggt agc aac aag agc aag ccc   473
                                Met Gly Ser Asn Lys Ser Lys Pro
                                1               5

aag gat gcc agc cag cgg cgc cgc agc ctg gag ccc gcc gag aac gtg   521
Lys Asp Ala Ser Gln Arg Arg Arg Ser Leu Glu Pro Ala Glu Asn Val
    10                  15                  20

cac ggc gct ggc ggg ggc gct ttc ccc gcc tcg cag acc ccc agc aag   569
His Gly Ala Gly Gly Gly Ala Phe Pro Ala Ser Gln Thr Pro Ser Lys
25                  30                  35                  40

cca gcc tcg gcc gac ggc cac cgc ggc ccc agc gcg gcc ttc gcc ccc   617
Pro Ala Ser Ala Asp Gly His Arg Gly Pro Ser Ala Ala Phe Ala Pro
                45                  50                  55

gcg gcc gcc gag ccc aag ctg ttc gga ggc ttc aac tcc tcg gac acc   665
Ala Ala Ala Glu Pro Lys Leu Phe Gly Gly Phe Asn Ser Ser Asp Thr
            60                  65                  70

gtc acc tcc ccg cag agg gcg ggc ccg ctg gcc ggt gga gtg acc acc   713
Val Thr Ser Pro Gln Arg Ala Gly Pro Leu Ala Gly Gly Val Thr Thr
        75                  80                  85

ttt gtg gcc ctc tat gac tat gag tct agg acg gag aca gac ctg tcc   761
Phe Val Ala Leu Tyr Asp Tyr Glu Ser Arg Thr Glu Thr Asp Leu Ser
    90                  95                  100

ttc aag aaa ggc gag cgg ctc cag att gtc aac aac aca gag gga gac   809
Phe Lys Lys Gly Glu Arg Leu Gln Ile Val Asn Asn Thr Glu Gly Asp
105                 110                 115                 120

tgg tgg ctg gcc cac tcg ctc agc aca gga cag aca ggc tac atc ccc   857
Trp Trp Leu Ala His Ser Leu Ser Thr Gly Gln Thr Gly Tyr Ile Pro
                125                 130                 135

agc aac tac gtg gcg ccc tcc gac tcc atc cag gct gag gag tgg tat   905
Ser Asn Tyr Val Ala Pro Ser Asp Ser Ile Gln Ala Glu Glu Trp Tyr
            140                 145                 150

ttt ggc aag atc acc aga cgg gag tca gag cgg tta ctg ctc aat gca   953
Phe Gly Lys Ile Thr Arg Arg Glu Ser Glu Arg Leu Leu Leu Asn Ala
        155                 160                 165

gag aac ccg aga ggg acc ttc ctc gtg cga gaa agt gag acc acg aaa   1001
Glu Asn Pro Arg Gly Thr Phe Leu Val Arg Glu Ser Glu Thr Thr Lys
    170                 175                 180

ggt gcc tac tgc ctc tca gtg tct gac ttc gac aac gcc aag ggc ctc   1049
Gly Ala Tyr Cys Leu Ser Val Ser Asp Phe Asp Asn Ala Lys Gly Leu
185                 190                 195                 200
```

```
aac gtg aag cac tac aag atc cgc aag ctg gac agc ggc ggc ttc tac      1097
Asn Val Lys His Tyr Lys Ile Arg Lys Leu Asp Ser Gly Gly Phe Tyr
                205                 210                 215

atc acc tcc cgc acc cag ttc aac agc ctg cag cag ctg gtg gcc tac      1145
Ile Thr Ser Arg Thr Gln Phe Asn Ser Leu Gln Gln Leu Val Ala Tyr
            220                 225                 230

tac tcc aaa cac gcc gat ggc ctg tgc cac cgc ctc acc acc gtg tgc      1193
Tyr Ser Lys His Ala Asp Gly Leu Cys His Arg Leu Thr Thr Val Cys
        235                 240                 245

ccc acg tcc aag ccg cag act cag ggc ctg gcc aag gat gcc tgg gag      1241
Pro Thr Ser Lys Pro Gln Thr Gln Gly Leu Ala Lys Asp Ala Trp Glu
    250                 255                 260

atc cct cgg gag tcg ctg cgg ctg gag gtc aag ctg ggc cag ggc tgc      1289
Ile Pro Arg Glu Ser Leu Arg Leu Glu Val Lys Leu Gly Gln Gly Cys
265                 270                 275                 280

ttt ggc gag gtg tgg atg ggg acc tgg aac ggt acc acc agg gtg gcc      1337
Phe Gly Glu Val Trp Met Gly Thr Trp Asn Gly Thr Thr Arg Val Ala
                285                 290                 295

atc aaa acc ctg aag cct ggc acg atg tct cca gag gcc ttc ctg cag      1385
Ile Lys Thr Leu Lys Pro Gly Thr Met Ser Pro Glu Ala Phe Leu Gln
            300                 305                 310

gag gcc cag gtc atg aag aag ctg agg cat gag aag ctg gtg cag ttg      1433
Glu Ala Gln Val Met Lys Lys Leu Arg His Glu Lys Leu Val Gln Leu
        315                 320                 325

tat gct gtg gtt tca gag gag ccc att tac atc gtc acg gag tac atg      1481
Tyr Ala Val Val Ser Glu Glu Pro Ile Tyr Ile Val Thr Glu Tyr Met
    330                 335                 340

agc aag ggg agt ttg ctg gac ttt ctc aag ggg gag aca ggc aag tac      1529
Ser Lys Gly Ser Leu Leu Asp Phe Leu Lys Gly Glu Thr Gly Lys Tyr
345                 350                 355                 360

ctg cgg ctg cct cag ctg gtg gac atg gct gct cag atc gcc tca ggc      1577
Leu Arg Leu Pro Gln Leu Val Asp Met Ala Ala Gln Ile Ala Ser Gly
                365                 370                 375

atg gcg tac gtg gag cgg atg aac tac gtc cac cgg gac ctt cgt gca      1625
Met Ala Tyr Val Glu Arg Met Asn Tyr Val His Arg Asp Leu Arg Ala
            380                 385                 390

gcc aac atc ctg gtg gga gag aac ctg gtg tgc aaa gtg gcc gac ttt      1673
Ala Asn Ile Leu Val Gly Glu Asn Leu Val Cys Lys Val Ala Asp Phe
        395                 400                 405

ggg ctg gct cgg ctc att gaa gac aat gag tac acg gcg cgg caa ggt      1721
Gly Leu Ala Arg Leu Ile Glu Asp Asn Glu Tyr Thr Ala Arg Gln Gly
    410                 415                 420

gcc aaa ttc ccc atc aag tgg acg gct cca gaa gct gcc ctc tat ggc      1769
Ala Lys Phe Pro Ile Lys Trp Thr Ala Pro Glu Ala Ala Leu Tyr Gly
425                 430                 435                 440

cgc ttc acc atc aag tcg gac gtg tgg tcc ttc ggg atc ctg ctg act      1817
Arg Phe Thr Ile Lys Ser Asp Val Trp Ser Phe Gly Ile Leu Leu Thr
                445                 450                 455

gag ctc acc aca aag gga cgg gtg ccc tac cct ggg atg gtg aac cgc      1865
Glu Leu Thr Thr Lys Gly Arg Val Pro Tyr Pro Gly Met Val Asn Arg
```

```
                      460                       465                       470

gag gtg ctg gac cag gtg gag cgg ggc tac cgg atg ccc tgc ccg ccg   1913
Glu Val Leu Asp Gln Val Glu Arg Gly Tyr Arg Met Pro Cys Pro Pro
        475                 480                 485

gag tgt ccc gag tcc ctg cac gac ctc atg tgc cag tgc tgg cgg aag   1961
Glu Cys Pro Glu Ser Leu His Asp Leu Met Cys Gln Cys Trp Arg Lys
    490                 495                 500

gag cct gag gag cgg ccc acc ttc gag tac ctg cag gcc ttc ctg gag   2009
Glu Pro Glu Glu Arg Pro Thr Phe Glu Tyr Leu Gln Ala Phe Leu Glu
505                 510                 515                 520

gac tac ttc acg tcc acc gag ccc cag tac cag ccc ggg gag aac ctc   2057
Asp Tyr Phe Thr Ser Thr Glu Pro Gln Tyr Gln Pro Gly Glu Asn Leu
                525                 530                 535

tag gcacaggcgg gcccagaccg gcttctcggc ttggatcctg ggctgggtgg        2110
 *

ccccctgtctc ggggcttgcc ccactctgcc tgcctgctgt tggtcctctc tctgtggggc 2170
tgaattgcca ggggcgaggc ccttcctctt tggtggcatg gaaggggctt ctggacctag 2230
ggtggcctga gagggcggtg ggtatgcgag accagcacgg tgactctgtc cagctcccgc 2290
tgtggccgca cgcctctccc tgcactccct cctggagctc tgtgggtctc tggaagagga 2350
accaggagaa gggctggggc cggggctgag ggtgcccttt tccagcctca gcctactccg 2410
ctcactgaac tccttcccca cttctgtgcc acccccggtc tatgtcgaga gctggccaaa 2470
gagcctttcc aaagaggagc gatgggcccc tggccccgcc tgcctgccac cctgcccctt 2530
gccatccatt ctggaaacac ctgtaggcag aggctgccga gacagaccct ctgccgctgc 2590
ttccaggctg ggcagcacaa ggccttgcct ggcctgatga tggtgggtgg gtgggatgag 2650
taccccctca aaccctgccc tccttagacc tgagggaccc ttcgagatca tcacttcctt 2710
gcccccattt cacccatggg gagacagttg agagcgggga tgtgacatgc ccaaggccac 2770
ggagcagttc agagtggagg cgggcttgga acccggtgct ccctctgtca tcctcaggaa 2830
ccaacaattc gtcggaggca tcatggaaag actgggacag cccaggaaac aaggggtctg 2890
aggatgcatt cgagatggca gattcccact gccgctgccc gctcagccca gctgttggga 2950
acagcatgga ggcagatgtg gggctgagct ggggaatcag ggtaaaaggt gcaggtgtgg 3010
agagagaggc ttcaatcggc ttgtgggtga tgtttgacct tcagagccag ccggctatga 3070
aagggagcga gcccctcggc tctggaggca atcaagcaga catagaagag ccaagagtcc 3130
aggaggccct ggtcctggcc tccttccccg tactttgtcc cgtggcattt caattcctgg 3190
ccctgttctc ctccccaagt cggcaccctt taactcatga ggagggaaaa gagtgcctaa 3250
gcgggggtga aagaggacgt gttacccact gccatgcacc aggactggct gtgtaacctt 3310
gggtggcccc tgctgtctct ctgggctgca gagtctgccc cacatgtggc catggcctct 3370
gcaactgctc agctctggtc caggccctgt ggcaggacac acatggtgag cctagccctg 3430
ggacatcagg agactgggct ctggctctgt tcggcctttg ggtgtgtggt ggattctccc 3490
tgggcctcag tgtgcccatc tgtaaagggg cagctgacag tttgtggcat cttgccaagg 3550
gtccctgtgt gtgtgtatgt gtgtgcatgt gtgcgtgtct ccatgtgcgt ccatatttaa 3610
catgtaaaaa tgtccccccc gctccgtccc ccaaacatgt tgtacatttc accatggccc 3670
cctcatcata gcaataacat tcccactgcc aggggttctt gagccagcca ggccctgcca 3730
gtggggaagg aggccaagca gtgcctgcct atgaaatttc aacttttcct ttcatacgtc 3790
tttattaccc aagtcttctc ccgtccattc cagtcaaatc tgggctcact caccccagcg 3850
agctctcaaa tccctctcca actgcctaag gccctttgtg taaggtgtct taatactgtc 3910
cttttttttt ttttaacagt gttttgtaga tttcagatga ctatgcagag gcctggggga 3970
cccctggctc tgggccgggc ctggggctcc gaaattccaa ggcccagact tgcgggggt  4030
ggggggtat ccagaattgg ttgtaaatac tttgcatatt gtctgattaa acacaaacag  4090
acctcagaaa aaaaaaaaaa aaaaaaaaaa aaaaaaaaaa aaaaaaaaaa aaaaa      4145
```

<210> 24
<211> 536
<212> PRT
<213> Homo sapiens

<400> 24

Met Gly Ser Asn Lys Ser Lys Pro Lys Asp Ala Ser Gln Arg Arg Arg
1 5 10 15
Ser Leu Glu Pro Ala Glu Asn Val His Gly Ala Gly Gly Gly Ala Phe

20 25 30
Pro Ala Ser Gln Thr Pro Ser Lys Pro Ala Ser Ala Asp Gly His Arg
35 40 45
Gly Pro Ser Ala Ala Phe Ala Pro Ala Ala Ala Glu Pro Lys Leu Phe
50 55 60
Gly Gly Phe Asn Ser Ser Asp Thr Val Thr Ser Pro Gln Arg Ala Gly
65 70 75 80
Pro Leu Ala Gly Gly Val Thr Thr Phe Val Ala Leu Tyr Asp Tyr Glu
85 90 95
Ser Arg Thr Glu Thr Asp Leu Ser Phe Lys Lys Gly Glu Arg Leu Gln
100 105 110
Ile Val Asn Asn Thr Glu Gly Asp Trp Trp Leu Ala His Ser Leu Ser
115 120 125
Thr Gly Gln Thr Gly Tyr Ile Pro Ser Asn Tyr Val Ala Pro Ser Asp
130 135 140
Ser Ile Gln Ala Glu Glu Trp Tyr Phe Gly Lys Ile Thr Arg Arg Glu
145 150 155 160
Ser Glu Arg Leu Leu Leu Asn Ala Glu Asn Pro Arg Gly Thr Phe Leu
165 170 175
Val Arg Glu Ser Glu Thr Thr Lys Gly Ala Tyr Cys Leu Ser Val Ser
180 185 190
Asp Phe Asp Asn Ala Lys Gly Leu Asn Val Lys His Tyr Lys Ile Arg
195 200 205
Lys Leu Asp Ser Gly Gly Phe Tyr Ile Thr Ser Arg Thr Gln Phe Asn
210 215 220
Ser Leu Gln Gln Leu Val Ala Tyr Tyr Ser Lys His Ala Asp Gly Leu
225 230 235 240
Cys His Arg Leu Thr Thr Val Cys Pro Thr Ser Lys Pro Gln Thr Gln
245 250 255
Gly Leu Ala Lys Asp Ala Trp Glu Ile Pro Arg Glu Ser Leu Arg Leu
260 265 270
Glu Val Lys Leu Gly Gln Gly Cys Phe Gly Glu Val Trp Met Gly Thr
275 280 285
Trp Asn Gly Thr Thr Arg Val Ala Ile Lys Thr Leu Lys Pro Gly Thr
290 295 300
Met Ser Pro Glu Ala Phe Leu Gln Glu Ala Gln Val Met Lys Lys Leu
305 310 315 320
Arg His Glu Lys Leu Val Gln Leu Tyr Ala Val Val Ser Glu Glu Pro
325 330 335
Ile Tyr Ile Val Thr Glu Tyr Met Ser Lys Gly Ser Leu Leu Asp Phe
340 345 350
Leu Lys Gly Glu Thr Gly Lys Tyr Leu Arg Leu Pro Gln Leu Val Asp
355 360 365
Met Ala Ala Gln Ile Ala Ser Gly Met Ala Tyr Val Glu Arg Met Asn
370 375 380
Tyr Val His Arg Asp Leu Arg Ala Ala Asn Ile Leu Val Gly Glu Asn
385 390 395 400
Leu Val Cys Lys Val Ala Asp Phe Gly Leu Ala Arg Leu Ile Glu Asp
405 410 415
Asn Glu Tyr Thr Ala Arg Gln Gly Ala Lys Phe Pro Ile Lys Trp Thr
420 425 430
Ala Pro Glu Ala Ala Leu Tyr Gly Arg Phe Thr Ile Lys Ser Asp Val
435 440 445
Trp Ser Phe Gly Ile Leu Leu Thr Glu Leu Thr Thr Lys Gly Arg Val
450 455 460
Pro Tyr Pro Gly Met Val Asn Arg Glu Val Leu Asp Gln Val Glu Arg
465 470 475 480
Gly Tyr Arg Met Pro Cys Pro Pro Glu Cys Pro Glu Ser Leu His Asp
485 490 495
Leu Met Cys Gln Cys Trp Arg Lys Glu Pro Glu Glu Arg Pro Thr Phe
500 505 510
Glu Tyr Leu Gln Ala Phe Leu Glu Asp Tyr Phe Thr Ser Thr Glu Pro
515 520 525
Gln Tyr Gln Pro Gly Glu Asn Leu
530 535

<210> 25
<211> 1333
<212> DNA
<213> Homo sapiens

<220>
<221> CDS
<222> (101)...(1030)

<400> 25

```
gggaggccg cctggttttc ctccctcctt ctgcacgtct gctggggtct cttcctctcc 60
aggccttgcc gtccccctgg cctctcttcc cagctcacac atg aag atg cac ttg   115
                                            Met Lys Met His Leu
                                            1               5

caa agg gct ctg gtg gtc ctg gcc ctg ctg aac ttt gcc acg gtc agc   163
Gln Arg Ala Leu Val Val Leu Ala Leu Leu Asn Phe Ala Thr Val Ser
                10                  15                  20

ctc tct ctg tcc act tgc acc acc ttg gac ttc ggc cac atc aag aag   211
Leu Ser Leu Ser Thr Cys Thr Thr Leu Asp Phe Gly His Ile Lys Lys
            25                  30                  35

aag agg gtg gaa gcc att agg gga cag atc ttg agc aag ctc agg ctc   259
Lys Arg Val Glu Ala Ile Arg Gly Gln Ile Leu Ser Lys Leu Arg Leu
        40                  45                  50

acc agc ccc cct gag cca acg gtg atg acc cac gtc ccc tat cag gtc   307
Thr Ser Pro Pro Glu Pro Thr Val Met Thr His Val Pro Tyr Gln Val
    55                  60                  65

ctg gcc ctt tac aac agc acc cgg gag ctg ctg gag gag atg cat ggg   355
Leu Ala Leu Tyr Asn Ser Thr Arg Glu Leu Leu Glu Glu Met His Gly
70                  75                  80                  85

gag agg gag gaa ggc tgc acc cag gaa aac acc gag tcg gaa tac tat   403
Glu Arg Glu Glu Gly Cys Thr Gln Glu Asn Thr Glu Ser Glu Tyr Tyr
                90                  95                  100

gcc aaa gaa atc cat aaa ttc gac atg atc cag ggg ctg gcg gag cac   451
Ala Lys Glu Ile His Lys Phe Asp Met Ile Gln Gly Leu Ala Glu His
            105                 110                 115

aac gaa ctg gct gtc tgc cct aaa gga att acc tcc aag gtt ttc cgc   499
Asn Glu Leu Ala Val Cys Pro Lys Gly Ile Thr Ser Lys Val Phe Arg
        120                 125                 130

ttc aat gtg tcc tca gtg gag aaa aat aga acc aac cta ttc cga gca   547
Phe Asn Val Ser Ser Val Glu Lys Asn Arg Thr Asn Leu Phe Arg Ala
    135                 140                 145

gaa ttc cgg gtc ttg cgg gtg ccc aac ccc agc tct aag cgg aat gag   595
Glu Phe Arg Val Leu Arg Val Pro Asn Pro Ser Ser Lys Arg Asn Glu
150                 155                 160                 165

cag agg atc gag ctc ttc cag atc ctt cgg cca gat gag cac att gcc   643
Gln Arg Ile Glu Leu Phe Gln Ile Leu Arg Pro Asp Glu His Ile Ala
                170                 175                 180

aaa cag cgc tat atc ggt ggc aag aat ctg ccc aca cgg ggc act gcc   691
Lys Gln Arg Tyr Ile Gly Gly Lys Asn Leu Pro Thr Arg Gly Thr Ala
            185                 190                 195

gag tgg ctg tcc ttt gat gtc act gac act gtg cgt gag tgg ctg ttg   739
Glu Trp Leu Ser Phe Asp Val Thr Asp Thr Val Arg Glu Trp Leu Leu
```

```
                    200                  205                 210

          aga aga gag tcc aac tta ggt cta gaa atc agc att cac tgt cca tgt     787
          Arg Arg Glu Ser Asn Leu Gly Leu Glu Ile Ser Ile His Cys Pro Cys
              215                 220                 225

          cac acc ttt cag ccc aat gga gat atc ctg gaa aac att cac gag gtg     835
          His Thr Phe Gln Pro Asn Gly Asp Ile Leu Glu Asn Ile His Glu Val
          230                 235                 240                 245

          atg gaa atc aaa ttc aaa ggc gtg gac aat gag gat gac cat ggc cgt     883
          Met Glu Ile Lys Phe Lys Gly Val Asp Asn Glu Asp Asp His Gly Arg
                          250                 255                 260

          gga gat ctg ggg cgc ctc aag aag cag aag gat cac cac aac cct cat     931
          Gly Asp Leu Gly Arg Leu Lys Lys Gln Lys Asp His His Asn Pro His
                      265                 270                 275

          cta atc ctc atg atg att ccc cca cac cgg ctc gac aac ccg ggc cag     979
          Leu Ile Leu Met Met Ile Pro Pro His Arg Leu Asp Asn Pro Gly Gln
                  280                 285                 290

          ggg ggt cag agg aag aag cgg gct ttg gac acc aat tac tgc ttc cgg     1027
          Gly Gly Gln Arg Lys Lys Arg Ala Leu Asp Thr Asn Tyr Cys Phe Arg
              295                 300                 305

          tga gactgggccc acatgggaac caacatctac tgcctgccta ctgcccaatg          1080
           *

          gctaggtcag gccccagagc caagccacac tcaacagagg gtccctgata ctattcacaa 1140
          acatctccag gaagaagact gaaaatctct cacagagatt ttctctgtga aatctctttc 1200
          tgttttcctg ggagtcccac tgtttttcca taggctaact ctggaaggag ctggctgaag 1260
          taaatgagga aaactctgtg aaaaaaaaaa aaaaaaaaaa aaaaaaaaaa aaaaaaaaaa 1320
          aaaaaaaaaa aaa                                                    1333
```

<210> 26
<211> 309
<212> PRT
<213> Homo sapiens

<400> 26

Met Lys Met His Leu Gln Arg Ala Leu Val Val Leu Ala Leu Leu Asn
1 5 10 15
Phe Ala Thr Val Ser Leu Ser Leu Ser Thr Cys Thr Thr Leu Asp Phe
20 25 30
Gly His Ile Lys Lys Lys Arg Val Glu Ala Ile Arg Gly Gln Ile Leu
35 40 45
Ser Lys Leu Arg Leu Thr Ser Pro Pro Glu Pro Thr Val Met Thr His
50 55 60
Val Pro Tyr Gln Val Leu Ala Leu Tyr Asn Ser Thr Arg Glu Leu Leu
65 70 75 80
Glu Glu Met His Gly Glu Arg Glu Glu Gly Cys Thr Gln Glu Asn Thr
85 90 95
Glu Ser Glu Tyr Tyr Ala Lys Glu Ile His Lys Phe Asp Met Ile Gln
100 105 110
Gly Leu Ala Glu His Asn Glu Leu Ala Val Cys Pro Lys Gly Ile Thr
115 120 125
Ser Lys Val Phe Arg Phe Asn Val Ser Ser Val Glu Lys Asn Arg Thr
130 135 140
Asn Leu Phe Arg Ala Glu Phe Arg Val Leu Arg Val Pro Asn Pro Ser
145 150 155 160
Ser Lys Arg Asn Glu Gln Arg Ile Glu Leu Phe Gln Ile Leu Arg Pro
165 170 175
Asp Glu His Ile Ala Lys Gln Arg Tyr Ile Gly Gly Lys Asn Leu Pro
180 185 190

Thr Arg Gly Thr Ala Glu Trp Leu Ser Phe Asp Val Thr Asp Thr Val
195 200 205
Arg Glu Trp Leu Leu Arg Arg Glu Ser Asn Leu Gly Leu Glu Ile Ser
210 215 220
Ile His Cys Pro Cys His Thr Phe Gln Pro Asn Gly Asp Ile Leu Glu
225 230 235 240
Asn Ile His Glu Val Met Glu Ile Lys Phe Lys Gly Val Asp Asn Glu
245 250 255
Asp Asp His Gly Arg Gly Asp Leu Gly Arg Leu Lys Lys Gln Lys Asp
260 265 270
His His Asn Pro His Leu Ile Leu Met Met Ile Pro Pro His Arg Leu
275 280 285
Asp Asn Pro Gly Gln Gly Gly Gln Arg Lys Lys Arg Ala Leu Asp Thr
290 295 300
Asn Tyr Cys Phe Arg
305

<210> 27
<211> 6378
<212> DNA
<213> Homo sapiens

<220>
<221> CDS
<222> (140)...(3409)

<400> 27

```
cccattactg ttggagctac agggagagaa acaggaggag actgcaagag atcatttggg 60
aaggccgtgg gcacgctctt tactccatgt gtgggacatt cattgcggaa taacatcgga 120
ggagaagttt cccagagct atg ggg act tcc cat ccg gcg ttc ctg gtc tta  172
                     Met Gly Thr Ser His Pro Ala Phe Leu Val Leu
                     1               5                   10

ggc tgt ctt ctc aca ggg ctg agc cta atc ctc tgc cag ctt tca tta   220
Gly Cys Leu Leu Thr Gly Leu Ser Leu Ile Leu Cys Gln Leu Ser Leu
            15                  20                  25

ccc tct atc ctt cca aat gaa aat gaa aag gtt gtg cag ctg aat tca   268
Pro Ser Ile Leu Pro Asn Glu Asn Glu Lys Val Val Gln Leu Asn Ser
        30                  35                  40

tcc ttt tct ctg aga tgc ttt ggg gag agt gaa gtg agc tgg cag tac   316
Ser Phe Ser Leu Arg Cys Phe Gly Glu Ser Glu Val Ser Trp Gln Tyr
    45                  50                  55

ccc atg tct gaa gaa gag agc tcc gat gtg gaa atc aga aat gaa gaa   364
Pro Met Ser Glu Glu Glu Ser Ser Asp Val Glu Ile Arg Asn Glu Glu
60                  65                  70                  75

aac aac agc ggc ctt ttt gtg acg gtc ttg gaa gtg agc agt gcc tcg   412
Asn Asn Ser Gly Leu Phe Val Thr Val Leu Glu Val Ser Ser Ala Ser
                80                  85                  90

gcg gcc cac aca ggg ttg tac act tgc tat tac aac cac act cag aca   460
Ala Ala His Thr Gly Leu Tyr Thr Cys Tyr Tyr Asn His Thr Gln Thr
            95                  100                 105

gaa gag aat gag ctt gaa ggc agg cac att tac atc tat gtg cca gac   508
Glu Glu Asn Glu Leu Glu Gly Arg His Ile Tyr Ile Tyr Val Pro Asp
        110                 115                 120

cca gat gta gcc ttt gta cct cta gga atg acg gat tat tta gtc atc   556
Pro Asp Val Ala Phe Val Pro Leu Gly Met Thr Asp Tyr Leu Val Ile
    125                 130                 135
```

```
gtg gag gat gat gat tct gcc att ata cct tgt cgc aca act gat ccc    604
Val Glu Asp Asp Asp Ser Ala Ile Ile Pro Cys Arg Thr Thr Asp Pro
140                 145                 150                 155

gag act cct gta acc tta cac aac agt gag ggg gtg gta cct gcc tcc    652
Glu Thr Pro Val Thr Leu His Asn Ser Glu Gly Val Val Pro Ala Ser
                160                 165                 170

tac gac agc aga cag ggc ttt aat ggg acc ttc act gta ggg ccc tat    700
Tyr Asp Ser Arg Gln Gly Phe Asn Gly Thr Phe Thr Val Gly Pro Tyr
            175                 180                 185

atc tgt gag gcc acc gtc aaa gga aag aag ttc cag acc atc cca ttt    748
Ile Cys Glu Ala Thr Val Lys Gly Lys Lys Phe Gln Thr Ile Pro Phe
        190                 195                 200

aat gtt tat gct tta aaa gca aca tca gag ctg gat cta gaa atg gaa    796
Asn Val Tyr Ala Leu Lys Ala Thr Ser Glu Leu Asp Leu Glu Met Glu
    205                 210                 215

gct ctt aaa acc gtg tat aag tca ggg gaa acg att gtg gtc acc tgt    844
Ala Leu Lys Thr Val Tyr Lys Ser Gly Glu Thr Ile Val Val Thr Cys
220                 225                 230                 235

gct gtt ttt aac aat gag gtg gtt gac ctt caa tgg act tac cct gga    892
Ala Val Phe Asn Asn Glu Val Val Asp Leu Gln Trp Thr Tyr Pro Gly
                240                 245                 250

gaa gtg aaa ggc aaa ggc atc aca atg ctg gaa gaa atc aaa gtc cca    940
Glu Val Lys Gly Lys Gly Ile Thr Met Leu Glu Glu Ile Lys Val Pro
            255                 260                 265

tcc atc aaa ttg gtg tac act ttg acg gtc ccc gag gcc acg gtg aaa    988
Ser Ile Lys Leu Val Tyr Thr Leu Thr Val Pro Glu Ala Thr Val Lys
        270                 275                 280

gac agt gga gat tac gaa tgt gct gcc cgc cag gct acc agg gag gtc    1036
Asp Ser Gly Asp Tyr Glu Cys Ala Ala Arg Gln Ala Thr Arg Glu Val
    285                 290                 295

aaa gaa atg aag aaa gtc act att tct gtc cat gag aaa ggt ttc att    1084
Lys Glu Met Lys Lys Val Thr Ile Ser Val His Glu Lys Gly Phe Ile
300                 305                 310                 315

gaa atc aaa ccc acc ttc agc cag ttg gaa gct gtc aac ctg cat gaa    1132
Glu Ile Lys Pro Thr Phe Ser Gln Leu Glu Ala Val Asn Leu His Glu
                320                 325                 330

gtc aaa cat ttt gtt gta gag gtg cgg gcc tac cca cct ccc agg ata    1180
Val Lys His Phe Val Val Glu Val Arg Ala Tyr Pro Pro Pro Arg Ile
            335                 340                 345

tcc tgg ctg aaa aac aat ctg act ctg att gaa aat ctc act gag atc    1228
Ser Trp Leu Lys Asn Asn Leu Thr Leu Ile Glu Asn Leu Thr Glu Ile
        350                 355                 360

acc act gat gtg gaa aag att cag gaa ata agg tat cga agc aaa tta    1276
Thr Thr Asp Val Glu Lys Ile Gln Glu Ile Arg Tyr Arg Ser Lys Leu
    365                 370                 375

aag ctg atc cgt gct aag gaa gaa gac agt ggc cat tat act att gta    1324
Lys Leu Ile Arg Ala Lys Glu Glu Asp Ser Gly His Tyr Thr Ile Val
380                 385                 390                 395

gct caa aat gaa gat gct gtg aag agc tat act ttt gaa ctg tta act    1372
Ala Gln Asn Glu Asp Ala Val Lys Ser Tyr Thr Phe Glu Leu Leu Thr
```

```
                400                 405                 410

caa gtt cct tca tcc att ctg gac ttg gtc gat gat cac cat ggc tca      1420
Gln Val Pro Ser Ser Ile Leu Asp Leu Val Asp Asp His His Gly Ser
            415                 420                 425

act ggg gga cag acg gtg agg tgc aca gct gaa ggc acg ccg ctt cct      1468
Thr Gly Gly Gln Thr Val Arg Cys Thr Ala Glu Gly Thr Pro Leu Pro
        430                 435                 440

gat att gag tgg atg ata tgc aaa gat att aag aaa tgt aat aat gaa      1516
Asp Ile Glu Trp Met Ile Cys Lys Asp Ile Lys Lys Cys Asn Asn Glu
    445                 450                 455

act tcc tgg act att ttg gcc aac aat gtc tca aac atc atc acg gag      1564
Thr Ser Trp Thr Ile Leu Ala Asn Asn Val Ser Asn Ile Ile Thr Glu
460                 465                 470                 475

atc cac tcc cga gac agg agt acc gtg gag ggc cgt gtg act ttc gcc      1612
Ile His Ser Arg Asp Arg Ser Thr Val Glu Gly Arg Val Thr Phe Ala
                480                 485                 490

aaa gtg gag gag acc atc gcc gtg cga tgc ctg gct aag aat ctc ctt      1660
Lys Val Glu Glu Thr Ile Ala Val Arg Cys Leu Ala Lys Asn Leu Leu
            495                 500                 505

gga gct gag aac cga gag ctg aag ctg gtg gct ccc acc ctg cgt tct      1708
Gly Ala Glu Asn Arg Glu Leu Lys Leu Val Ala Pro Thr Leu Arg Ser
        510                 515                 520

gaa ctc acg gtg gct gct gca gtc ctg gtg ctg ttg gtg att gtg atc      1756
Glu Leu Thr Val Ala Ala Ala Val Leu Val Leu Leu Val Ile Val Ile
    525                 530                 535

atc tca ctt att gtc ctg gtt gtc att tgg aaa cag aaa ccg agg tat      1804
Ile Ser Leu Ile Val Leu Val Val Ile Trp Lys Gln Lys Pro Arg Tyr
540                 545                 550                 555

gaa att cgc tgg agg gtc att gaa tca atc agc ccg gat gga cat gaa      1852
Glu Ile Arg Trp Arg Val Ile Glu Ser Ile Ser Pro Asp Gly His Glu
                560                 565                 570

tat att tat gtg gac ccg atg cag ctg cct tat gac tca aga tgg gag      1900
Tyr Ile Tyr Val Asp Pro Met Gln Leu Pro Tyr Asp Ser Arg Trp Glu
            575                 580                 585

ttt cca aga gat gga cta gtg ctt ggt cgg gtc ttg ggg tct gga gcg      1948
Phe Pro Arg Asp Gly Leu Val Leu Gly Arg Val Leu Gly Ser Gly Ala
        590                 595                 600

ttt ggg aag gtg gtt gaa gga aca gcc tat gga tta agc cgg tcc caa      1996
Phe Gly Lys Val Val Glu Gly Thr Ala Tyr Gly Leu Ser Arg Ser Gln
    605                 610                 615

cct gtc atg aaa gtt gca gtg aag atg cta aaa ccc acg gcc aga tcc      2044
Pro Val Met Lys Val Ala Val Lys Met Leu Lys Pro Thr Ala Arg Ser
620                 625                 630                 635

agt gaa aaa caa gct ctc atg tct gaa ctg aag ata atg act cac ctg      2092
Ser Glu Lys Gln Ala Leu Met Ser Glu Leu Lys Ile Met Thr His Leu
                640                 645                 650

ggg cca cat ttg aac att gta aac ttg ctg gga gcc tgc acc aag tca      2140
Gly Pro His Leu Asn Ile Val Asn Leu Leu Gly Ala Cys Thr Lys Ser
            655                 660                 665
```

```
ggc ccc att tac atc atc aca gag tac tgc ttc tat gga gat ttg gtc    2188
Gly Pro Ile Tyr Ile Ile Thr Glu Tyr Cys Phe Tyr Gly Asp Leu Val
        670                 675                 680

aac tat ttg cat aag aat agg gat agc ttc ctg agc cac cac cca gag    2236
Asn Tyr Leu His Lys Asn Arg Asp Ser Phe Leu Ser His His Pro Glu
    685                 690                 695

aag cca aag aaa gag ctg gat atc ttt gga ttg aac cct gct gat gaa    2284
Lys Pro Lys Lys Glu Leu Asp Ile Phe Gly Leu Asn Pro Ala Asp Glu
700                 705                 710                 715

agc aca cgg agc tat gtt att tta tct ttt gaa aac aat ggt gac tac    2332
Ser Thr Arg Ser Tyr Val Ile Leu Ser Phe Glu Asn Asn Gly Asp Tyr
                720                 725                 730

atg gac atg aag cag gct gat act aca cag tat gtc ccc atg cta gaa    2380
Met Asp Met Lys Gln Ala Asp Thr Thr Gln Tyr Val Pro Met Leu Glu
            735                 740                 745

agg aaa gag gtt tct aaa tat tcc gac atc cag aga tca ctc tat gat    2428
Arg Lys Glu Val Ser Lys Tyr Ser Asp Ile Gln Arg Ser Leu Tyr Asp
        750                 755                 760

cgt cca gcc tca tat aag aag aaa tct atg tta gac tca gaa gtc aaa    2476
Arg Pro Ala Ser Tyr Lys Lys Lys Ser Met Leu Asp Ser Glu Val Lys
    765                 770                 775

aac ctc ctt tca gat gat aac tca gaa ggc ctt act tta ttg gat ttg    2524
Asn Leu Leu Ser Asp Asp Asn Ser Glu Gly Leu Thr Leu Leu Asp Leu
780                 785                 790                 795

ttg agc ttc acc tat caa gtt gcc cga gga atg gag ttt ttg gct tca    2572
Leu Ser Phe Thr Tyr Gln Val Ala Arg Gly Met Glu Phe Leu Ala Ser
                800                 805                 810

aaa aat tgt gtc cac cgt gat ctg gct gct cgc aac gtc ctc ctg gca    2620
Lys Asn Cys Val His Arg Asp Leu Ala Ala Arg Asn Val Leu Leu Ala
            815                 820                 825

caa gga aaa att gtg aag atc tgt gac ttt ggc ctg gcc aga gac atc    2668
Gln Gly Lys Ile Val Lys Ile Cys Asp Phe Gly Leu Ala Arg Asp Ile
        830                 835                 840

atg cat gat tcg aac tat gtg tcg aaa ggc agt acc ttt ctg ccc gtg    2716
Met His Asp Ser Asn Tyr Val Ser Lys Gly Ser Thr Phe Leu Pro Val
    845                 850                 855

aag tgg atg gct cct gag agc atc ttt gac aac ctc tac acc aca ctg    2764
Lys Trp Met Ala Pro Glu Ser Ile Phe Asp Asn Leu Tyr Thr Thr Leu
860                 865                 870                 875

agt gat gtc tgg tct tat ggc att ctg ctc tgg gag atc ttt tcc ctt    2812
Ser Asp Val Trp Ser Tyr Gly Ile Leu Leu Trp Glu Ile Phe Ser Leu
                880                 885                 890

ggt ggc acc cct tac ccc ggc atg atg gtg gat tct act ttc tac aat    2860
Gly Gly Thr Pro Tyr Pro Gly Met Met Val Asp Ser Thr Phe Tyr Asn
            895                 900                 905

aag atc aag agt ggg tac cgg atg gcc aag cct gac cac gct acc agt    2908
Lys Ile Lys Ser Gly Tyr Arg Met Ala Lys Pro Asp His Ala Thr Ser
        910                 915                 920

gaa gtc tac gag atc atg gtg aaa tgc tgg aac agt gag ccg gag aag    2956
Glu Val Tyr Glu Ile Met Val Lys Cys Trp Asn Ser Glu Pro Glu Lys
```

925 930 935

aga ccc tcc ttt tac cac ctg agt gag att gtg gag aat ctg ctg cct 3004
Arg Pro Ser Phe Tyr His Leu Ser Glu Ile Val Glu Asn Leu Leu Pro
940 945 950 955

gga caa tat aaa aag agt tat gaa aaa att cac ctg gac ttc ctg aag 3052
Gly Gln Tyr Lys Lys Ser Tyr Glu Lys Ile His Leu Asp Phe Leu Lys
960 965 970

agt gac cat cct gct gtg gca cgc atg cgt gtg gac tca gac aat gca 3100
Ser Asp His Pro Ala Val Ala Arg Met Arg Val Asp Ser Asp Asn Ala
975 980 985

tac att ggt gtc acc tac aaa aac gag gaa gac aag ctg aag gac tgg 3148
Tyr Ile Gly Val Thr Tyr Lys Asn Glu Glu Asp Lys Leu Lys Asp Trp
990 995 1000

gag ggt ggt ctg gat gag cag aga ctg agc gct gac agt ggc tac atc 3196
Glu Gly Gly Leu Asp Glu Gln Arg Leu Ser Ala Asp Ser Gly Tyr Ile
1005 1010 1015

att cct ctg cct gac att gac cct gtc cct gag gag gag gac ctg ggc 3244
Ile Pro Leu Pro Asp Ile Asp Pro Val Pro Glu Glu Glu Asp Leu Gly
1020 1025 1030 1035

aag agg aac aga cac agc tcg cag acc tct gaa gag agt gcc att gag 3292
Lys Arg Asn Arg His Ser Ser Gln Thr Ser Glu Glu Ser Ala Ile Glu
1040 1045 1050

acg ggt tcc agc agt tcc acc ttc atc aag aga gag gac gag acc att 3340
Thr Gly Ser Ser Ser Ser Thr Phe Ile Lys Arg Glu Asp Glu Thr Ile
1055 1060 1065

gaa gac atc gac atg atg gac gac atc ggc ata gac tct tca gac ctg 3388
Glu Asp Ile Asp Met Met Asp Asp Ile Gly Ile Asp Ser Ser Asp Leu
1070 1075 1080

gtg gaa gac agc ttc ctg taa ctggcggatt cgaggggttc cttccacttc 3439
Val Glu Asp Ser Phe Leu *
1085

tggggccacc tctggatccc gttcagaaaa ccactttatt gcaatgcgga ggttgagagg 3499
aggacttggt tgatgtttaa agagaagttc ccagccaagg gcctcgggga gcgttctaaa 3559
tatgaatgaa tgggatattt tgaaatgaac tttgtcagtg ttgcctctcg caatgcctca 3619
gtagcatctc agtggtgtgt gaagtttgga gatagatgga taagggaata ataggccaca 3679
gaaggtgaac tttgtgcttc aaggacattg gtgagagtcc aacagacaca atttatactg 3739
cgacagaact tcagcattgt aattatgtaa ataactctaa ccaaggctgt gtttagattg 3799
tattaactat cttctttgga cttctgaaga gaccactcaa tccatccatg tacttccctc 3859
ttgaaacctg atgtcagctg ctgttgaact tttaaagaa gtgcatgaaa aaccattttt 3919
gaaccttaaa aggtactggt actatagcat tttgctatct tttttagtgt taagagataa 3979
agaataataa ttaaccaacc ttgtttaata gatttgggtc atttagaagc ctgacaactc 4039
attttcatat tgtaatctat gtttataata ctactactgt tatcagtaat gctaaatgtg 4099
taataatgta acatgatttc cctccagaga aagcacaatt taaaacaatc cttactaagt 4159
aggtgatgag tttgacagtt tttgacattt atattaaata acatgtttct ctataaagta 4219
tggtaatagc tttagtgaat taaatttagt tgagcataga gaacaaagta aaagtagtgt 4279
tgtccaggaa gtcagaattt ttaactgtac tgaataggtt ccccaatcca tcgtattaaa 4339
aaacaattaa ctgccctctg aaataatggg attagaaaca aacaaaactc ttaagtccta 4399
aaagttctca atgtagaggc ataaacctgt gctgaacata acttctcatg tatattaccc 4459
aatggaaaat ataatgatca gcaaaaagac tggatttgca gaagtttttt ttttttttct 4519
tcatgcctga tgaaagcttt ggcaacccca atatatgtat tttttgaatc tatgaacctg 4579
aaaagggtca gaaggatgcc cagacatcag cctccttctt tcacccctta ccccaaagag 4639
aaagagtttg aaactcgaga ccataaagat attctttagt ggaggctgga tgtgcattag 4699
cctggatcct cagttctcaa atgtgtgtgg cagccaggat gactagatcc tgggtttcca 4759
tccttgagat tctgaagtat gaagtctgag ggaaaccaga gtctgtattt ttctaaactc 4819
cctggctgtt ctgatcggcc agtttcgga aacactgact taggtttcag gaagttgcca 4879

```
tgggaaacaa ataatttgaa ctttggaaca gggttggaat tcaaccacgc aggaagccta 4939
ctatttaaat ccttggcttc aggttagtga catttaatgc catctagcta gcaattgcga 4999
ccttaattta actttccagt cttagctgag gctgagaaag ctaaagtttg gttttgacag 5059
gttttccaaa agtaaagatg ctacttccca ctgtatgggg gagattgaac tttccccgtc 5119
tcccgtcttc tgcctcccac tccatacccc gccaaggaaa ggcatgtaca aaaattatgc 5179
aattcagtgt tccaagtctc tgtgtaacca gctcagtgtt ttggtggaaa aaacatttta 5239
agttttactg ataatttgag gttagatggg aggatgaatt gtcacatcta tccacactgt 5299
caaacaggtt ggtgtgggtt cattggcatt ctttgcaata ctgcttaatt gctgatacca 5359
tatgaatgaa acatgggctg tgattactgc aatcactgtg ctatcggcag atgatgcttt 5419
ggaagatgca gaagcaataa taaagtactt gactacctac tggtgtaatc tcaatgcaag 5479
ccccaacttt cttatccaac tttttcatag taagtgcgaa gactgagcca gattggccaa 5539
ttaaaaacga aaacctgact aggttctgta gagccaatta gacttgaaat acgtttgtgt 5599
ttctagaatc acagctcaag cattctgttt atcgctcact ctcccttgta cagccttatt 5659
ttgttggtgc tttgcatttt gatattgctg tgagccttgc atgacatcat gaggccggat 5719
gaaacttctc agtccagcag tttccagtcc taacaaatgc tcccacctga atttgtatat 5779
gactgcattt gtgggtgtgt gtgtgttttc agcaaattcc agatttgttt ccttttggcc 5839
tcctgcaaag tctccagaag aaaatttgcc aatctttcct actttctatt tttatgatga 5899
caatcaaagc cggcctgaga aacactattt gtgacttttt aaacgattag tgatgtcctt 5959
aaaatgtggt ctgccaatct gtacaaaatg gtcctatttt tgtgaagagg gacataagat 6019
aaaatgatgt tatacatcaa tatgtatata tgtatttcta tatagacttg gagaatactg 6079
ccaaaacatt tatgacaagc tgtatcactg ccttcgttta tattttttta actgtgataa 6139
tccccacagg cacattaact gttgcacttt tgaatgtcca aaatttatat tttagaaata 6199
ataaaaagaa agatacttac atgttcccaa aacaatggtg tggtgaatgt gtgagaaaaa 6259
ctaacttgat agggtctacc aatacaaaat gtattacgaa tgcccctgtt catgtttttg 6319
ttttaaaacg tgtaaatgaa gatctttata tttcaataaa tgatatataa tttaaagtt  6378
```

<210> 28
<211> 1089
<212> PRT
<213> Homo sapiens

<400> 28

```
Met Gly Thr Ser His Pro Ala Phe Leu Val Leu Gly Cys Leu Leu Thr
 1               5                  10                  15
Gly Leu Ser Leu Ile Leu Cys Gln Leu Ser Leu Pro Ser Ile Leu Pro
            20                  25                  30
Asn Glu Asn Glu Lys Val Val Gln Leu Asn Ser Ser Phe Ser Leu Arg
        35                  40                  45
Cys Phe Gly Glu Ser Glu Val Ser Trp Gln Tyr Pro Met Ser Glu Glu
    50                  55                  60
Glu Ser Ser Asp Val Glu Ile Arg Asn Glu Glu Asn Asn Ser Gly Leu
65                  70                  75                  80
Phe Val Thr Val Leu Glu Val Ser Ser Ala Ser Ala Ala His Thr Gly
                85                  90                  95
Leu Tyr Thr Cys Tyr Tyr Asn His Thr Gln Thr Glu Glu Asn Glu Leu
            100                 105                 110
Glu Gly Arg His Ile Tyr Ile Tyr Val Pro Asp Pro Asp Val Ala Phe
        115                 120                 125
Val Pro Leu Gly Met Thr Asp Tyr Leu Val Ile Val Glu Asp Asp Asp
    130                 135                 140
Ser Ala Ile Ile Pro Cys Arg Thr Thr Asp Pro Glu Thr Pro Val Thr
145                 150                 155                 160
Leu His Asn Ser Glu Gly Val Val Pro Ala Ser Tyr Asp Ser Arg Gln
                165                 170                 175
Gly Phe Asn Gly Thr Phe Thr Val Gly Pro Tyr Ile Cys Glu Ala Thr
            180                 185                 190
Val Lys Gly Lys Lys Phe Gln Thr Ile Pro Phe Asn Val Tyr Ala Leu
        195                 200                 205
Lys Ala Thr Ser Glu Leu Asp Leu Glu Met Glu Ala Leu Lys Thr Val
    210                 215                 220
Tyr Lys Ser Gly Glu Thr Ile Val Val Thr Cys Ala Val Phe Asn Asn
225                 230                 235                 240
Glu Val Val Asp Leu Gln Trp Thr Tyr Pro Gly Glu Val Lys Gly Lys
                245                 250                 255
Gly Ile Thr Met Leu Glu Glu Ile Lys Val Pro Ser Ile Lys Leu Val
            260                 265                 270
```

```
Tyr Thr Leu Thr Val Pro Glu Ala Thr Val Lys Asp Ser Gly Asp Tyr
        275                 280                 285
Glu Cys Ala Ala Arg Gln Ala Thr Arg Glu Val Lys Glu Met Lys Lys
    290                 295                 300
Val Thr Ile Ser Val His Glu Lys Gly Phe Ile Glu Ile Lys Pro Thr
305                 310                 315                 320
Phe Ser Gln Leu Glu Ala Val Asn Leu His Glu Val Lys His Phe Val
                325                 330                 335
Val Glu Val Arg Ala Tyr Pro Pro Pro Arg Ile Ser Trp Leu Lys Asn
            340                 345                 350
Asn Leu Thr Leu Ile Glu Asn Leu Thr Glu Ile Thr Thr Asp Val Glu
        355                 360                 365
Lys Ile Gln Glu Ile Arg Tyr Arg Ser Lys Leu Lys Leu Ile Arg Ala
    370                 375                 380
Lys Glu Glu Asp Ser Gly His Tyr Thr Ile Val Ala Gln Asn Glu Asp
385                 390                 395                 400
Ala Val Lys Ser Tyr Thr Phe Glu Leu Leu Thr Gln Val Pro Ser Ser
                405                 410                 415
Ile Leu Asp Leu Val Asp Asp His His Gly Ser Thr Gly Gly Gln Thr
            420                 425                 430
Val Arg Cys Thr Ala Glu Gly Thr Pro Leu Pro Asp Ile Glu Trp Met
        435                 440                 445
Ile Cys Lys Asp Ile Lys Lys Cys Asn Asn Glu Thr Ser Trp Thr Ile
    450                 455                 460
Leu Ala Asn Asn Val Ser Asn Ile Ile Thr Glu Ile His Ser Arg Asp
465                 470                 475                 480
Arg Ser Thr Val Glu Gly Arg Val Thr Phe Ala Lys Val Glu Glu Thr
                485                 490                 495
Ile Ala Val Arg Cys Leu Ala Lys Asn Leu Leu Gly Ala Glu Asn Arg
            500                 505                 510
Glu Leu Lys Leu Val Ala Pro Thr Leu Arg Ser Glu Leu Thr Val Ala
        515                 520                 525
Ala Ala Val Leu Val Leu Leu Val Ile Val Ile Ile Ser Leu Ile Val
    530                 535                 540
Leu Val Val Ile Trp Lys Gln Lys Pro Arg Tyr Glu Ile Arg Trp Arg
545                 550                 555                 560
Val Ile Glu Ser Ile Ser Pro Asp Gly His Glu Tyr Ile Tyr Val Asp
                565                 570                 575
Pro Met Gln Leu Pro Tyr Asp Ser Arg Trp Glu Phe Pro Arg Asp Gly
            580                 585                 590
Leu Val Leu Gly Arg Val Leu Gly Ser Gly Ala Phe Gly Lys Val Val
        595                 600                 605
Glu Gly Thr Ala Tyr Gly Leu Ser Arg Ser Gln Pro Val Met Lys Val
    610                 615                 620
Ala Val Lys Met Leu Lys Pro Thr Ala Arg Ser Ser Glu Lys Gln Ala
625                 630                 635                 640
Leu Met Ser Glu Leu Lys Ile Met Thr His Leu Gly Pro His Leu Asn
                645                 650                 655
Ile Val Asn Leu Leu Gly Ala Cys Thr Lys Ser Gly Pro Ile Tyr Ile
            660                 665                 670
Ile Thr Glu Tyr Cys Phe Tyr Gly Asp Leu Val Asn Tyr Leu His Lys
        675                 680                 685
Asn Arg Asp Ser Phe Leu Ser His His Pro Glu Lys Pro Lys Lys Glu
    690                 695                 700
Leu Asp Ile Phe Gly Leu Asn Pro Ala Asp Glu Ser Thr Arg Ser Tyr
705                 710                 715                 720
Val Ile Leu Ser Phe Glu Asn Asn Gly Asp Tyr Met Asp Met Lys Gln
                725                 730                 735
Ala Asp Thr Thr Gln Tyr Val Pro Met Leu Glu Arg Lys Glu Val Ser
            740                 745                 750
Lys Tyr Ser Asp Ile Gln Arg Ser Leu Tyr Asp Arg Pro Ala Ser Tyr
        755                 760                 765
Lys Lys Lys Ser Met Leu Asp Ser Glu Val Lys Asn Leu Leu Ser Asp
    770                 775                 780
Asp Asn Ser Glu Gly Leu Thr Leu Leu Asp Leu Leu Ser Phe Thr Tyr
785                 790                 795                 800
```

```
Gln Val Ala Arg Gly Met Glu Phe Leu Ala Ser Lys Asn Cys Val His
                805                 810                 815
Arg Asp Leu Ala Ala Arg Asn Val Leu Leu Ala Gln Gly Lys Ile Val
            820                 825                 830
Lys Ile Cys Asp Phe Gly Leu Ala Arg Asp Ile Met His Asp Ser Asn
        835                 840                 845
Tyr Val Ser Lys Gly Ser Thr Phe Leu Pro Val Lys Trp Met Ala Pro
    850                 855                 860
Glu Ser Ile Phe Asp Asn Leu Tyr Thr Thr Leu Ser Asp Val Trp Ser
865                 870                 875                 880
Tyr Gly Ile Leu Leu Trp Glu Ile Phe Ser Leu Gly Gly Thr Pro Tyr
                885                 890                 895
Pro Gly Met Met Val Asp Ser Thr Phe Tyr Asn Lys Ile Lys Ser Gly
            900                 905                 910
Tyr Arg Met Ala Lys Pro Asp His Ala Thr Ser Glu Val Tyr Glu Ile
        915                 920                 925
Met Val Lys Cys Trp Asn Ser Glu Pro Glu Lys Arg Pro Ser Phe Tyr
    930                 935                 940
His Leu Ser Glu Ile Val Glu Asn Leu Leu Pro Gly Gln Tyr Lys Lys
945                 950                 955                 960
Ser Tyr Glu Lys Ile His Leu Asp Phe Leu Lys Ser Asp His Pro Ala
                965                 970                 975
Val Ala Arg Met Arg Val Asp Ser Asp Asn Ala Tyr Ile Gly Val Thr
            980                 985                 990
Tyr Lys Asn Glu Glu Asp Lys Leu Lys Asp Trp Glu Gly Gly Leu Asp
        995                 1000                1005
Glu Gln Arg Leu Ser Ala Asp Ser Gly Tyr Ile Ile Pro Leu Pro Asp
    1010                1015                1020
Ile Asp Pro Val Pro Glu Glu Glu Asp Leu Gly Lys Arg Asn Arg His
1025                1030                1035                1040
Ser Ser Gln Thr Ser Glu Glu Ser Ala Ile Glu Thr Gly Ser Ser Ser
                1045                1050                1055
Ser Thr Phe Ile Lys Arg Glu Asp Glu Thr Ile Glu Asp Ile Asp Met
            1060                1065                1070
Met Asp Asp Ile Gly Ile Asp Ser Ser Asp Leu Val Glu Asp Ser Phe
        1075                1080                1085
Leu
```

<210> 29
<211> 2121
<212> DNA
<213> Homo sapiens

<220>
<221> CDS
<222> (559)...(1878)

<400> 29

```
ctgctcgcgg ccgccaccgc cgggccccgg ccgtccctgg ctcccctcct gcctcgagaa 60
gggcagggct tctcagaggc ttggcgggaa aaaagaacgg agggagggat cgcgctgagt 120
ataaaagccg gttttcgggg ctttatctaa ctcgctgtag taattccagc gagaggcaga 180
gggagcgagc gggcggccgg ctagggtgga agagccgggc gagcagagct gcgctgcggg 240
cgtcctggga agggagatcc ggagcgaata gggggcttcg cctctggccc agccctcccg 300
cttgatcccc caggccagcg gtccgcaacc cttgccgcat ccacgaaact ttgcccatag 360
cagcgggcgg gcactttgca ctggaactta caacacccga gcaaggacgc gactctcccg 420
acgcggggag gctattctgc ccatttgggg acacttcccc gccgctgcca ggacccgctt 480
ctctgaaagg ctctccttgc agctgcttag acgctggatt tttttcgggt agtggaaaac 540
cagcagcctc ccgcgacg atg ccc ctc aac gtt agc ttc acc aac agg aac   591
                    Met Pro Leu Asn Val Ser Phe Thr Asn Arg Asn
                     1               5                  10

tat gac ctc gac tac gac tcg gtg cag ccg tat ttc tac tgc gac gag   639
Tyr Asp Leu Asp Tyr Asp Ser Val Gln Pro Tyr Phe Tyr Cys Asp Glu
            15                  20                  25
```

```
gag gag aac ttc tac cag cag cag cag cag agc gag ctg cag ccc ccg    687
Glu Glu Asn Phe Tyr Gln Gln Gln Gln Gln Ser Glu Leu Gln Pro Pro
        30                  35                  40

gcg ccc agc gag gat atc tgg aag aaa ttc gag ctg ctg ccc acc ccg    735
Ala Pro Ser Glu Asp Ile Trp Lys Lys Phe Glu Leu Leu Pro Thr Pro
    45                  50                  55

ccc ctg tcc cct agc cgc cgc tcc ggg ctc tgc tcg ccc tcc tac gtt    783
Pro Leu Ser Pro Ser Arg Arg Ser Gly Leu Cys Ser Pro Ser Tyr Val
60                  65                  70                  75

gcg gtc aca ccc ttc tcc ctt cgg gga gac aac gac ggc ggt ggc ggg    831
Ala Val Thr Pro Phe Ser Leu Arg Gly Asp Asn Asp Gly Gly Gly Gly
                80                  85                  90

agc ttc tcc acg gcc gac cag ctg gag atg gtg acc gag ctg ctg gga    879
Ser Phe Ser Thr Ala Asp Gln Leu Glu Met Val Thr Glu Leu Leu Gly
            95                  100                 105

gga gac atg gtg aac cag agt ttc atc tgc gac ccg gac gac gag acc    927
Gly Asp Met Val Asn Gln Ser Phe Ile Cys Asp Pro Asp Asp Glu Thr
        110                 115                 120

ttc atc aaa aac atc atc atc cag gac tgt atg tgg agc ggc ttc tcg    975
Phe Ile Lys Asn Ile Ile Ile Gln Asp Cys Met Trp Ser Gly Phe Ser
    125                 130                 135

gcc gcc gcc aag ctc gtc tca gag aag ctg gcc tcc tac cag gct gcg    1023
Ala Ala Ala Lys Leu Val Ser Glu Lys Leu Ala Ser Tyr Gln Ala Ala
140                 145                 150                 155

cgc aaa gac agc ggc agc ccg aac ccc gcc cgc ggc cac agc gtc tgc    1071
Arg Lys Asp Ser Gly Ser Pro Asn Pro Ala Arg Gly His Ser Val Cys
                160                 165                 170

tcc acc tcc agc ttg tac ctg cag gat ctg agc gcc gcc gcc tca gag    1119
Ser Thr Ser Ser Leu Tyr Leu Gln Asp Leu Ser Ala Ala Ala Ser Glu
            175                 180                 185

tgc atc gac ccc tcg gtg gtc ttc ccc tac cct ctc aac gac agc agc    1167
Cys Ile Asp Pro Ser Val Val Phe Pro Tyr Pro Leu Asn Asp Ser Ser
        190                 195                 200

tcg ccc aag tcc tgc gcc tcg caa gac tcc agc gcc ttc tct ccg tcc    1215
Ser Pro Lys Ser Cys Ala Ser Gln Asp Ser Ser Ala Phe Ser Pro Ser
    205                 210                 215

tcg gat tct ctg ctc tcc tcg acg gag tcc tcc ccg cag ggc agc ccc    1263
Ser Asp Ser Leu Leu Ser Ser Thr Glu Ser Ser Pro Gln Gly Ser Pro
220                 225                 230                 235

gag ccc ctg gtg ctc cat gag gag aca ccg ccc acc acc agc agc gac    1311
Glu Pro Leu Val Leu His Glu Glu Thr Pro Pro Thr Thr Ser Ser Asp
                240                 245                 250

tct gag gag gaa caa gaa gat gag gaa gaa atc gat gtt gtt tct gtg    1359
Ser Glu Glu Glu Gln Glu Asp Glu Glu Glu Ile Asp Val Val Ser Val
            255                 260                 265

gaa aag agg cag gct cct ggc aaa agg tca gag tct gga tca cct tct    1407
Glu Lys Arg Gln Ala Pro Gly Lys Arg Ser Glu Ser Gly Ser Pro Ser
        270                 275                 280

gct gga ggc cac agc aaa cct cct cac agc cca ctg gtc ctc aag agg    1455
```

```
Ala Gly Gly His Ser Lys Pro Pro His Ser Pro Leu Val Leu Lys Arg
    285                 290                 295

tgc cac gtc tcc aca cat cag cac aac tac gca gcg cct ccc tcc act     1503
Cys His Val Ser Thr His Gln His Asn Tyr Ala Ala Pro Pro Ser Thr
300                 305                 310                 315

cgg aag gac tat cct gct gcc aag agg gtc aag ttg gac agt gtc aga     1551
Arg Lys Asp Tyr Pro Ala Ala Lys Arg Val Lys Leu Asp Ser Val Arg
                320                 325                 330

gtc ctg aga cag atc agc aac aac cga aaa tgc acc agc ccc agg tcc     1599
Val Leu Arg Gln Ile Ser Asn Asn Arg Lys Cys Thr Ser Pro Arg Ser
            335                 340                 345

tcg gac acc gag gag aat gtc aag agg cga aca cac aac gtc ttg gag     1647
Ser Asp Thr Glu Glu Asn Val Lys Arg Arg Thr His Asn Val Leu Glu
        350                 355                 360

cgc cag agg agg aac gag cta aaa cgg agc ttt ttt gcc ctg cgt gac     1695
Arg Gln Arg Arg Asn Glu Leu Lys Arg Ser Phe Phe Ala Leu Arg Asp
    365                 370                 375

cag atc ccg gag ttg gaa aac aat gaa aag gcc ccc aag gta gtt atc     1743
Gln Ile Pro Glu Leu Glu Asn Asn Glu Lys Ala Pro Lys Val Val Ile
380                 385                 390                 395

ctt aaa aaa gcc aca gca tac atc ctg tcc gtc caa gca gag gag caa     1791
Leu Lys Lys Ala Thr Ala Tyr Ile Leu Ser Val Gln Ala Glu Glu Gln
                400                 405                 410

aag ctc att tct gaa gag gac ttg ttg cgg aaa cga cga gaa cag ttg     1839
Lys Leu Ile Ser Glu Glu Asp Leu Leu Arg Lys Arg Arg Glu Gln Leu
            415                 420                 425

aaa cac aaa ctt gaa cag cta cgg aac tct tgt gcg taa ggaaaagtaa      1888
Lys His Lys Leu Glu Gln Leu Arg Asn Ser Cys Ala  *
        430                 435

ggaaaacgat tccttctaac agaaatgtcc tgagcaatca cctatgaact tgtttcaaat 1948
gcatgatcaa atgcaacctc acaaccttgg ctgagtcttg agactgaaag atttagccat 2008
aatgtaaact gcctcaaatt ggactttggg cataaaagaa ctttttatg cttaccatct 2068
ttttttttc tttaacagat ttgtatttaa gaattgtttt taaaaaattt taa        2121
```

<210> 30
<211> 439
<212> PRT
<213> Homo sapiens

<400> 30

```
Met Pro Leu Asn Val Ser Phe Thr Asn Arg Asn Tyr Asp Leu Asp Tyr
 1               5                  10                  15
Asp Ser Val Gln Pro Tyr Phe Tyr Cys Asp Glu Glu Glu Asn Phe Tyr
            20                  25                  30
Gln Gln Gln Gln Gln Ser Glu Leu Gln Pro Pro Ala Pro Ser Glu Asp
        35                  40                  45
Ile Trp Lys Lys Phe Glu Leu Leu Pro Thr Pro Pro Leu Ser Pro Ser
    50                  55                  60
Arg Arg Ser Gly Leu Cys Ser Pro Ser Tyr Val Ala Val Thr Pro Phe
65                  70                  75                  80
Ser Leu Arg Gly Asp Asn Asp Gly Gly Gly Gly Ser Phe Ser Thr Ala
                85                  90                  95
Asp Gln Leu Glu Met Val Thr Glu Leu Leu Gly Gly Asp Met Val Asn
            100                 105                 110
Gln Ser Phe Ile Cys Asp Pro Asp Asp Glu Thr Phe Ile Lys Asn Ile
        115                 120                 125

Ile Ile Gln Asp Cys Met Trp Ser Gly Phe Ser Ala Ala Ala Lys Leu
    130                 135                 140
Val Ser Glu Lys Leu Ala Ser Tyr Gln Ala Ala Arg Lys Asp Ser Gly
145                 150                 155                 160
Ser Pro Asn Pro Ala Arg Gly His Ser Val Cys Ser Thr Ser Ser Leu
                165                 170                 175
Tyr Leu Gln Asp Leu Ser Ala Ala Ala Ser Glu Cys Ile Asp Pro Ser
            180                 185                 190
Val Val Phe Pro Tyr Pro Leu Asn Asp Ser Ser Ser Pro Lys Ser Cys
        195                 200                 205
Ala Ser Gln Asp Ser Ser Ala Phe Ser Pro Ser Ser Asp Ser Leu Leu
    210                 215                 220
Ser Ser Thr Glu Ser Ser Pro Gln Gly Ser Pro Glu Pro Leu Val Leu
225                 230                 235                 240
His Glu Glu Thr Pro Pro Thr Thr Ser Ser Asp Ser Glu Glu Glu Gln
                245                 250                 255
Glu Asp Glu Glu Glu Ile Asp Val Val Ser Val Glu Lys Arg Gln Ala
            260                 265                 270
Pro Gly Lys Arg Ser Glu Ser Gly Ser Pro Ser Ala Gly Gly His Ser
        275                 280                 285
Lys Pro Pro His Ser Pro Leu Val Leu Lys Arg Cys His Val Ser Thr
    290                 295                 300
His Gln His Asn Tyr Ala Ala Pro Pro Ser Thr Arg Lys Asp Tyr Pro
305                 310                 315                 320
Ala Ala Lys Arg Val Lys Leu Asp Ser Val Arg Val Leu Arg Gln Ile
                325                 330                 335
Ser Asn Asn Arg Lys Cys Thr Ser Pro Arg Ser Ser Asp Thr Glu Glu
            340                 345                 350
Asn Val Lys Arg Arg Thr His Asn Val Leu Glu Arg Gln Arg Arg Asn
        355                 360                 365
Glu Leu Lys Arg Ser Phe Phe Ala Leu Arg Asp Gln Ile Pro Glu Leu
    370                 375                 380
Glu Asn Asn Glu Lys Ala Pro Lys Val Val Ile Leu Lys Lys Ala Thr
385                 390                 395                 400
Ala Tyr Ile Leu Ser Val Gln Ala Glu Glu Gln Lys Leu Ile Ser Glu
                405                 410                 415
Glu Asp Leu Leu Arg Lys Arg Arg Glu Gln Leu Lys His Lys Leu Glu
            420                 425                 430
Gln Leu Arg Asn Ser Cys Ala
        435
```

<210> 31
<211> 1374
<212> DNA
<213> Homo sapiens

<220>
<221> CDS
<222> (103)...(1047)

<400> 31

```
ggacagcttg gagatagggc ccggaattgc gggcgtcact ctgctcctgc gacctagcca 60
ggcgtgaggg agtgacagca gcgcattcgc gggacgagag cg atg agt gag aac    114
                                               Met Ser Glu Asn
                                                1

gcc gca cca ggt ctg atc tca gag ctg aag ctg gct gtg ccc tgg ggc   162
Ala Ala Pro Gly Leu Ile Ser Glu Leu Lys Leu Ala Val Pro Trp Gly
 5                  10                  15                  20

cac atc gca gcc aaa gcc tgg ggc tcc ctg cag ggc cct cca gtt ctc   210
His Ile Ala Ala Lys Ala Trp Gly Ser Leu Gln Gly Pro Pro Val Leu
                25                  30                  35

tgc ctg cac ggc tgg ctg gac aat gcc agc tcc ttc gac aga ctc atc   258
```

```
Cys Leu His Gly Trp Leu Asp Asn Ala Ser Ser Phe Asp Arg Leu Ile
                40                  45                  50

cct ctt ctc ccg caa gac ttt tat tac gtt gcc atg gat ttc gga ggt      306
Pro Leu Leu Pro Gln Asp Phe Tyr Tyr Val Ala Met Asp Phe Gly Gly
            55                  60                  65

cat ggg ctc tcg tcc cat tac agc cca ggt gtc cca tat tac ctc cag      354
His Gly Leu Ser Ser His Tyr Ser Pro Gly Val Pro Tyr Tyr Leu Gln
        70                  75                  80

act ttt gtg agt gag atc cga aga gtt gtg gca gcc ttg aaa tgg aat      402
Thr Phe Val Ser Glu Ile Arg Arg Val Val Ala Ala Leu Lys Trp Asn
85                  90                  95                  100

cga ttc tcc att ctg ggc cac agc ttc ggt ggc gtc gtg ggc gga atg      450
Arg Phe Ser Ile Leu Gly His Ser Phe Gly Gly Val Val Gly Gly Met
                105                 110                 115

ttt ttc tgt acc ttc ccc gag atg gtg gat aaa ctt atc ttg ctg gac      498
Phe Phe Cys Thr Phe Pro Glu Met Val Asp Lys Leu Ile Leu Leu Asp
            120                 125                 130

acg ccg ctc ttt ctc ctg gaa tca gat gaa atg gag aac ttg ctg acc      546
Thr Pro Leu Phe Leu Leu Glu Ser Asp Glu Met Glu Asn Leu Leu Thr
        135                 140                 145

tac aag cgg aga gcc ata gag cac gtg ctg cag gta gag gcc tcc cag      594
Tyr Lys Arg Arg Ala Ile Glu His Val Leu Gln Val Glu Ala Ser Gln
    150                 155                 160

gag ccc tcg cac gtg ttc agc ctg aag cag ctg ctg cag agg tta ctg      642
Glu Pro Ser His Val Phe Ser Leu Lys Gln Leu Leu Gln Arg Leu Leu
165                 170                 175                 180

aag agc aat agc cac ttg agt gag gag tgc ggg gag ctt ctc ctg caa      690
Lys Ser Asn Ser His Leu Ser Glu Glu Cys Gly Glu Leu Leu Leu Gln
                185                 190                 195

aga gga acc acg aag gtg gcc aca ggt ctg gtt ctg aac aga gac cag      738
Arg Gly Thr Thr Lys Val Ala Thr Gly Leu Val Leu Asn Arg Asp Gln
            200                 205                 210

agg ctc gcc tgg gca gag aac agc att gac ttc atc agc agg gag ctg      786
Arg Leu Ala Trp Ala Glu Asn Ser Ile Asp Phe Ile Ser Arg Glu Leu
        215                 220                 225

tgt gcg cat tcc atc agg aag ctg cag gcc cat gtc ctg ttg atc aaa      834
Cys Ala His Ser Ile Arg Lys Leu Gln Ala His Val Leu Leu Ile Lys
    230                 235                 240

gca gtc cac gga tat ttt gat tca aga cag aat tac tct gag aag gag      882
Ala Val His Gly Tyr Phe Asp Ser Arg Gln Asn Tyr Ser Glu Lys Glu
245                 250                 255                 260

tcc ctg tcg ttc atg ata gac acg atg aaa tcc acc ctc aaa gag cag      930
Ser Leu Ser Phe Met Ile Asp Thr Met Lys Ser Thr Leu Lys Glu Gln
                265                 270                 275

ttc cag ttt gtg gaa gtc cca ggc aat cac tgt gtc cac atg agc gaa      978
Phe Gln Phe Val Glu Val Pro Gly Asn His Cys Val His Met Ser Glu
            280                 285                 290

ccc cag cac gtg gcc agt atc atc agc tcc ttc tta cag tgc aca cac     1026
Pro Gln His Val Ala Ser Ile Ile Ser Ser Phe Leu Gln Cys Thr His
        295                 300                 305
```

```
atg ctc cca gcc cag ctg tag ctctgggcct ggaactatga agacctagtg      1077
Met Leu Pro Ala Gln Leu  *
    310

ctcccagact caacactggg actctgagtt cctgagcccc acaacaaggc cagggatggt 1137
ggggacaggc ctcactagtc ttgaggccca gcctaggatg gtagtcaggg gaaggagcga 1197
gattccaact tcaacatctg tgacctcaag gggagacag agtctgggtt ccagggctgc 1257
tttctcctgg ctaataataa atatccagcc agctggagga aggaagggca ggctgggccc 1317
acctagcctt tccctgctgc ccaactggat ggaaaataaa aggttcttgt attctca    1374
```

<210> 32
<211> 314
<212> PRT
<213> Homo sapiens

<400> 32

```
Met Ser Glu Asn Ala Ala Pro Gly Leu Ile Ser Glu Leu Lys Leu Ala
1               5                   10                  15
Val Pro Trp Gly His Ile Ala Ala Lys Ala Trp Gly Ser Leu Gln Gly
            20                  25                  30
Pro Pro Val Leu Cys Leu His Gly Trp Leu Asp Asn Ala Ser Ser Phe
        35                  40                  45
Asp Arg Leu Ile Pro Leu Leu Pro Gln Asp Phe Tyr Tyr Val Ala Met
    50                  55                  60
Asp Phe Gly Gly His Gly Leu Ser Ser His Tyr Ser Pro Gly Val Pro
65                  70                  75                  80
Tyr Tyr Leu Gln Thr Phe Val Ser Glu Ile Arg Arg Val Val Ala Ala
                85                  90                  95
Leu Lys Trp Asn Arg Phe Ser Ile Leu Gly His Ser Phe Gly Gly Val
            100                 105                 110
Val Gly Gly Met Phe Phe Cys Thr Phe Pro Glu Met Val Asp Lys Leu
        115                 120                 125
Ile Leu Leu Asp Thr Pro Leu Phe Leu Leu Glu Ser Asp Glu Met Glu
    130                 135                 140
Asn Leu Leu Thr Tyr Lys Arg Arg Ala Ile Glu His Val Leu Gln Val
145                 150                 155                 160
Glu Ala Ser Gln Glu Pro Ser His Val Phe Ser Leu Lys Gln Leu Leu
                165                 170                 175
Gln Arg Leu Leu Lys Ser Asn Ser His Leu Ser Glu Glu Cys Gly Glu
            180                 185                 190
Leu Leu Leu Gln Arg Gly Thr Thr Lys Val Ala Thr Gly Leu Val Leu
        195                 200                 205
Asn Arg Asp Gln Arg Leu Ala Trp Ala Glu Asn Ser Ile Asp Phe Ile
    210                 215                 220
Ser Arg Glu Leu Cys Ala His Ser Ile Arg Lys Leu Gln Ala His Val
225                 230                 235                 240
Leu Leu Ile Lys Ala Val His Gly Tyr Phe Asp Ser Arg Gln Asn Tyr
                245                 250                 255
Ser Glu Lys Glu Ser Leu Ser Phe Met Ile Asp Thr Met Lys Ser Thr
            260                 265                 270
Leu Lys Glu Gln Phe Gln Phe Val Glu Val Pro Gly Asn His Cys Val
        275                 280                 285
His Met Ser Glu Pro Gln His Val Ala Ser Ile Ile Ser Ser Phe Leu
    290                 295                 300
Gln Cys Thr His Met Leu Pro Ala Gln Leu
305                 310
```

<210> 33
<211> 22
<212> DNA
<213> Artificial Sequence

<220>
<223> TaqMan primer

<400> 33
tacacaccac cttcgctgaa ag 22

<210> 34
<211> 22
<212> DNA
<213> Artificial Sequence

<220>
<223> TaqMan primer

<400> 34
ggcctggttc tcattcaaat tg 22

<210> 35
<211> 26
<212> DNA
<213> Artificial Sequence

<220>
<223> TaqMan primer

<400> 35
cgcattgctg agtctcacct gcagtc 26

<210> 36
<211> 25
<212> DNA
<213> Artificial Sequence

<220>
<223> TaqMan primer

<400> 36
cagccttaca gagactggaa aagaa 25

<210> 37
<211> 19
<212> DNA
<213> Artificial Sequence

<220>
<223> TaqMan primer

<400> 37
gaggctcagg gacccaaag 19

<210> 38
<211> 24
<212> DNA
<213> Artificial Sequence

<220>
<223> TaqMan primer

<400> 38
ccaaaccaag gcccccagag aggt 24

<210> 39
<211> 19
<212> DNA
<213> Artificial Sequence

<220>
<223> TaqMan primer

<400> 39
cctaccgcca gcacattgt 19

<210> 40
<211> 23
<212> DNA
<213> Artificial Sequence

<220>
<223> TaqMan primer

<400> 40
gctgttgtag gcattgatga aca 23

<210> 41
<211> 24
<212> DNA
<213> Artificial Sequence

<220>
<223> TaqMan primer

<400> 41
aatgacatga accccggcaa cctg 24